# EUROPEAN PATENT APPLICATION

(11) **EP 4 847 045 A1**
(43) Date of publication of application: **02.12.2026**
(21) Application number: 24920031.2
(22) Date of filing: 31.12.2024
(51) Int. Cl.: A61K 47/64, A61K 31/4745, A61P 35/00

(54) **POLYPEPTIDE CONJUGATE AND USE THEREOF**

(30) Priority: 23.01.2024 CN 202410097180; 19.12.2024 CN 202411884584
(71) Applicant: Shanghai Chixi Biomedical Technology Co., Ltd, Shanghai 201210 (CN)
(72) Inventor: SHEN, Zan, Shanghai 201210 (CN); ZHAO, Yingxi, Shanghai 201210 (CN)
(74) Representative: Metida
(86) International application number: PCT/CN2024/144651
(87) International publication number: WO 2025/156953

(57) **Abstract**

The present disclosure provides a polypeptide conjugate and a use thereof, and particularly relates to a Belotecan drug and derivative polypeptide conjugate drugs thereof. The polypeptide conjugate drugs provided by the present disclosure are selectively released in a tumor microenvironment, which can solve the problem of limited clinical use due to the non-targeting property and significant side effects of the Belotecan drug in the prior art. Clinically, greater doses can be used to treat cancers and improve the overall therapeutic effect.

## Description

### TECHNICAL FIELD

The present disclosure relates to a use of a polypeptide conjugate drug, and in particular to a tumor-targeting activated Belotecan as well as a derivative polypeptide conjugate and a use thereof.

### BACKGROUND

Colorectal cancer, also known as carcinoma of large intestine, refers to a cancer of epithelial origin of the large intestine, including colon cancer and rectal cancer, and adenocarcinoma is a more common pathological pattern. In China, colon cancer is a kind of common malignant tumor in the gastrointestinal tract without obvious early symptoms, which is manifested by symptoms such as change in bowel habits, hematochezia, diarrhea, alternation of diarrhea and constipation, local abdominal pain and the like as the cancer grows. Colon cancer is manifested by systemic symptoms such as anemia and weight loss in a late stage of cancer. In view of an increasing trend in the morbidity and mortality of colorectal cancer in China, new therapeutic drugs are needed to improve the therapeutic effect, and early diagnosis and early surgical removal treatment can improve a postoperative disease-free survival rate and prolong overall survival.

Osteosarcoma is one of the rare tumors, with a morbidity of 4-5 cases per million people, about 5,000 new cases are reported per year in China, and all medications in the current clinical diagnosis and treatment protocols are poor in therapeutic effect.

Breast cancer is a cancer with the highest morbidity, and there were more than 2.3 million new patients worldwide in 2022. Currently, these two cancers are mainly treated with targeted drugs supplemented by chemotherapeutic drugs, and the targeted drugs are usually specific to a single type of gene mutation and hardly achieve a broad-spectrum effect, while the chemotherapeutic drugs have excessive side effects and mediocre curative effects. In view of such situations, there is an urgent need to develop new therapeutic drugs that are effective for treating breast cancer.

Belotecan is a synthetic water-soluble camptothecin analogue. Belotecan exhibits extensive antitumor activity against various human tumor cells, with a potency equal to or higher than that of camptothecin and Topotecan, as well as good therapeutic effects for lung cancer, ovarian cancer and colorectal cancer. However, Belotecan is limited in clinical applications due to its significant side effects such as neutropenia and thrombocytopenia, anaemia, diarrhea and anorexia caused by gastrointestinal toxicity.

Prior art document 1: CN113274507A

At present, prior arts for preparing other anticancer compounds (drugs) into conjugated complexes to enhance drug targeting and reduce toxicity are already available. For example, disclosed in the patent CN113274507A is an "immunostimulatory conjugated complex delivered and activated in a targeted manner", including an MI group, a selective group S, a tripeptide group C cleaved by asparaginyl endopeptidase, an auxiliary linker arm A and a conjugated drug, in which the tripeptide group C is preferably AAN (alanine-alanine-asparagine), and the auxiliary linker arm A is preferably L (leucine) or PAB (p-aminobenzyl alcohol). Compared with an anticancer compound (Payload), the conjugated complex in the above prior art has been optimized to a certain extent in terms of reducing toxicity and improving efficacy, but the effect is still unsatisfactory and cannot meet the practical needs for clinical applications in cancer therapy. In fact, improvements in drug targeting, enhancement of drug efficacy, reduction of toxic and side effects and the like are very important for clinical treatment of cancer patients, reduced drug toxicity can significantly relieve patients' symptoms of side effects (e.g. diarrhea, anorexia, anemia and the like) even in cases where there is no much difference in efficacy, and the reduced toxicity means that the treatment with larger doses can be performed in order to improve the overall therapeutic effect.

Regarding Belotecan, there is currently no research in the prior art on improving the targeting and reducing the side effects of the antitumor drug Belotecan. In view of the foregoing, there is an urgent need to develop a compound that can significantly enhance the targeting and efficacy of Belotecan, and significantly reduce the side effects so as to achieve a better effect in cancer treatment.

### SUMMARY

The present disclosure provides a tumor-targeting activated polypeptide conjugate as well as a preparation method and a use thereof, which can solve the problem of limited clinical use in the prior art due to non-targeting and significant side effects of the drug Belotecan. The polypeptide conjugate of the present disclosure can be selectively released in a tumor microenvironment, exhibiting good stability and targeting in normal cells where it is hardly released, the toxic and side effects of Belotecan are also greatly reduced, and a larger dose can be used clinically to treat the cancer, so that the overall therapeutic effect is improved.

The present disclosure provides a polypeptide conjugate, which has a structural formula as follows:

E-nPEG-L1-L2-L3-L4-D

where,
E is a group with maleimide;
nPEG is n-polyethylene glycol, and n is an integer greater than or equal to 2 and less than or equal to 40;
L1 and L2 are glycine, alanine, phenylalanine, threonine, serine or none;
L3 is glycine, alanine, lysine, citrulline or asparagine;
L4 is glycine, proline, p-aminobenzyl alcohol or none; and
D is Belotecan or a derivative thereof.

The present disclosure further provides a polypeptide conjugate, which has a structural formula as follows:

E-nPEG-L1-L2-L3-L4-D

where,
E is a group with maleimide;
nPEG is n-polyethylene glycol, and n is an integer greater than or equal to 2 and less than or equal to 40;
L1 and L2 are glycine, alanine, phenylalanine, threonine, serine or none;
L3 is glycine, alanine, lysine, citrulline or asparagine;
L4 is leucine, isoleucine or none; and
D is Belotecan or a derivative thereof.

The present disclosure further provides a polypeptide conjugate, which has a structural formula as follows:

E-nPEG-L1-L2-L3-L4-D

where,
E is a group with maleimide;
nPEG is n-polyethylene glycol, and n is an integer greater than or equal to 2 and less than or equal to 40;
L1 and L2 are glycine, alanine, phenylalanine, threonine, serine or none;
L3 is glycine, alanine, lysine, citrulline or asparagine;
L4 is glycine, proline, p-aminobenzyl alcohol, leucine, isoleucine or none; and
D is Belotecan or a derivative thereof.

The present disclosure provides a polypeptide conjugate, which has a structural formula as follows:

E-nPEG-L1-L2-L3-L4-D

where,
E is a group with maleimide;
nPEG is n-polyethylene glycol, and n is an integer greater than or equal to 2 and less than or equal to 40;
L1 and L2 are glycine, alanine, phenylalanine, threonine or serine;
L3 is glycine, alanine, lysine, citrulline or asparagine;
L4 is glycine, proline or p-aminobenzyl alcohol; and
D is Belotecan or a derivative thereof.

The present disclosure provides a polypeptide conjugate, which has a structural formula as follows:

E-nPEG-L1-L2-L3-L4-D

where,
E is a group with maleimide;
nPEG is n-polyethylene glycol, and n is an integer greater than or equal to 2 and less than or equal to 40;
L1 and L2 are glycine, alanine, phenylalanine, threonine or serine;
L3 is glycine, alanine, lysine, citrulline or asparagine;
L4 is glycine; and
D is Belotecan or a derivative thereof.

The present disclosure further provides a polypeptide conjugate, which has a structural formula as follows:

E-nPEG-L1-L2-L3-L4-D

where,
E is a group with maleimide;
nPEG is n-polyethylene glycol, and n is an integer greater than or equal to 2 and less than or equal to 40;
L1 and L2 are glycine, alanine, phenylalanine, threonine or serine;
L3 is glycine, alanine, lysine, citrulline or asparagine;
L4 is leucine or isoleucine; and
D is Belotecan or a derivative thereof.

The present disclosure further provides a polypeptide conjugate, which has a structural formula as follows:

E-nPEG-L1-L2-L3-L4-D

where,
E is a group with maleimide;
nPEG is n-polyethylene glycol, and n is an integer greater than or equal to 2 and less than or equal to 40;
L1 and L2 are glycine, alanine, phenylalanine, threonine or serine;
L3 is glycine, alanine, lysine, citrulline or asparagine;
L4 is glycine, proline, p-aminobenzyl alcohol, leucine or isoleucine; and
D is Belotecan or a derivative thereof. The polypeptide conjugate described in the present disclosure has a D group structure of: 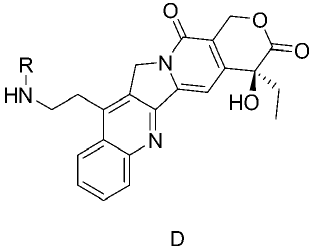
where, R is methyl, ethyl, n-propyl, isopropyl, n-butyl, isobutyl, cyclopropyl, cyclobutyl or cyclopentyl.

The polypeptide conjugate described in the present disclosure has an E group structure of: 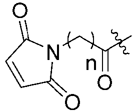 where, n=1-18.

The polypeptide conjugate described in the present disclosure can be of any of the following structures: 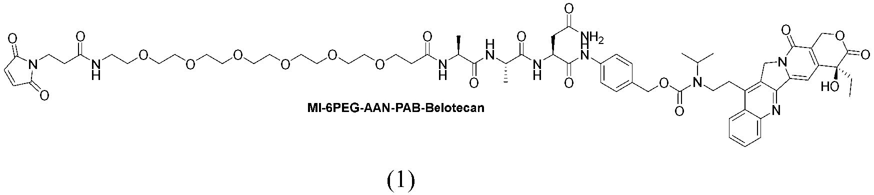 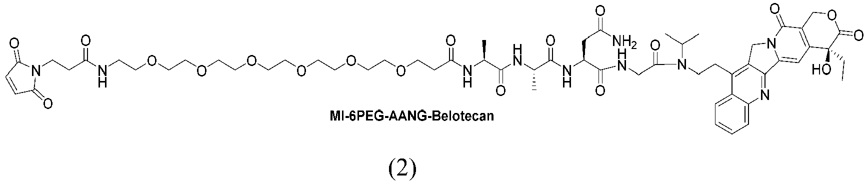 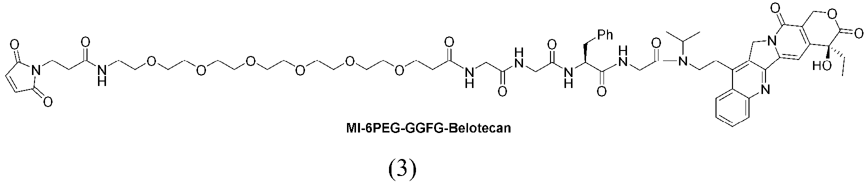 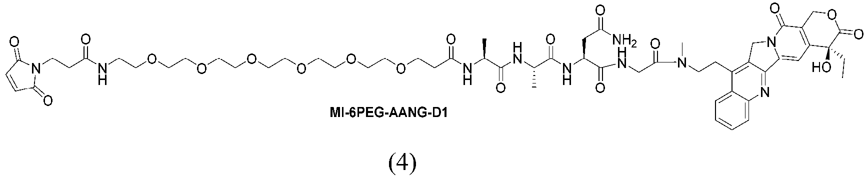 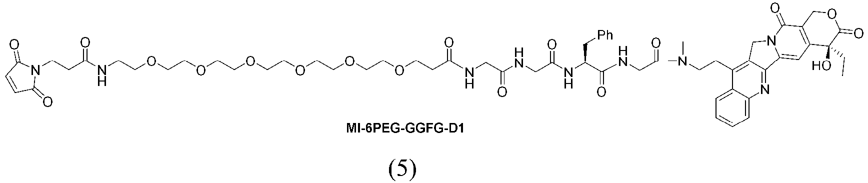 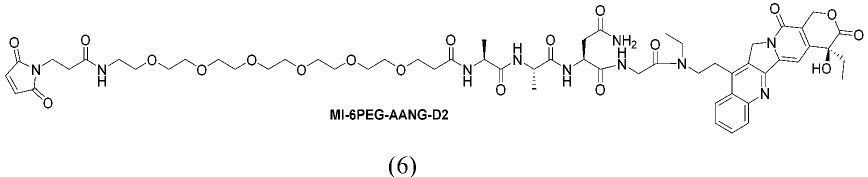 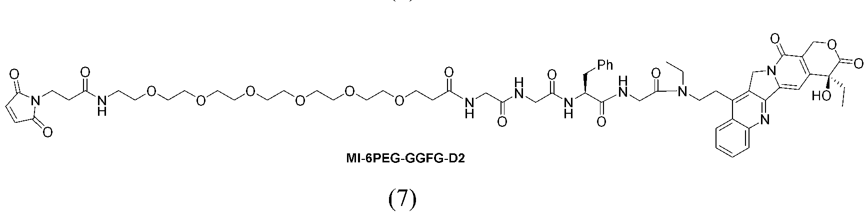 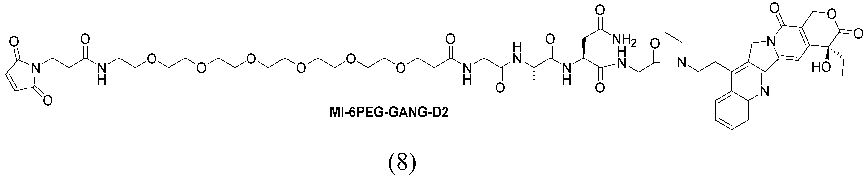 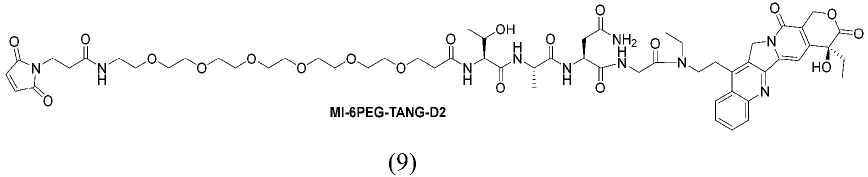 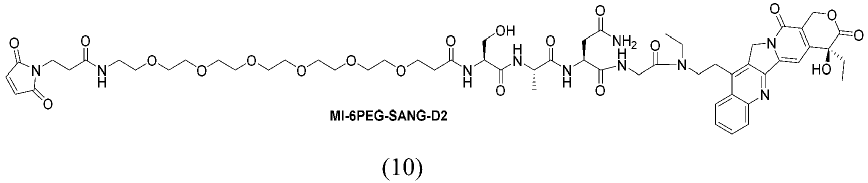 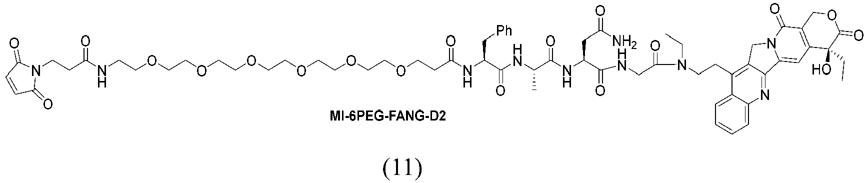 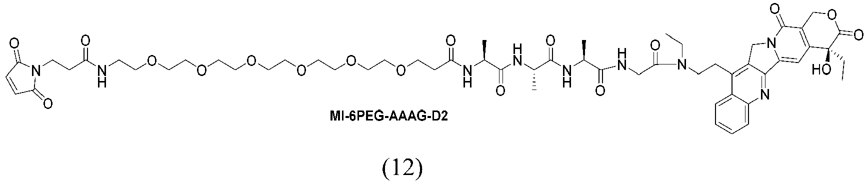 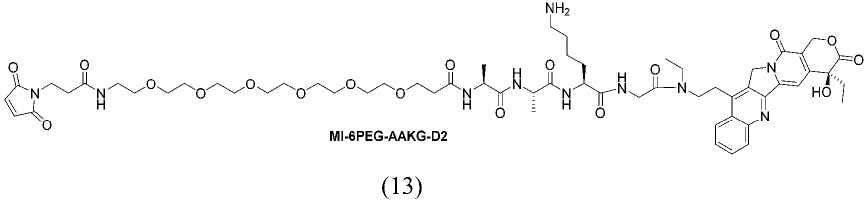 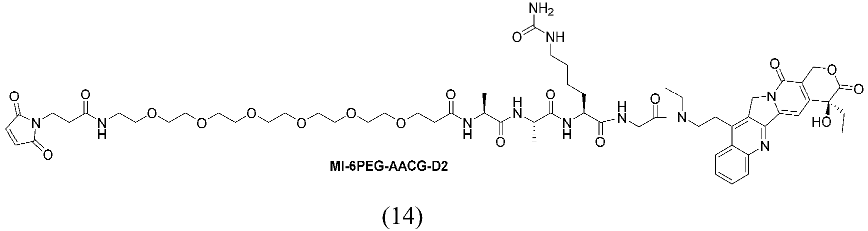 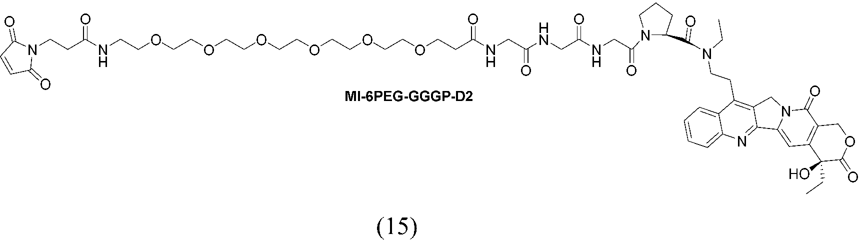 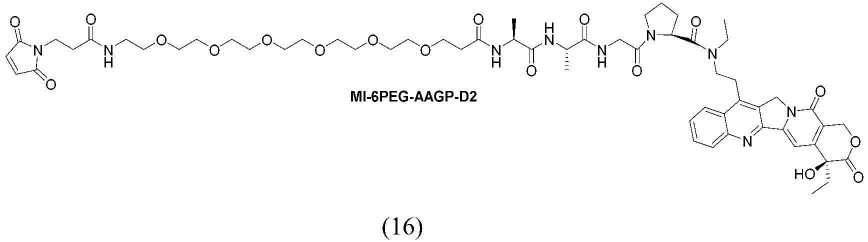 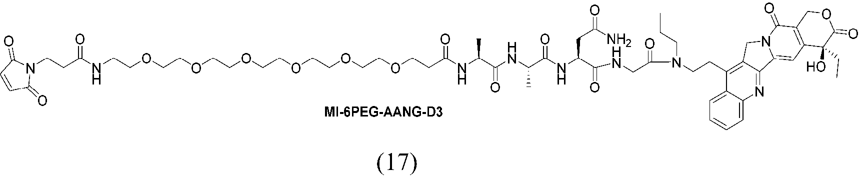 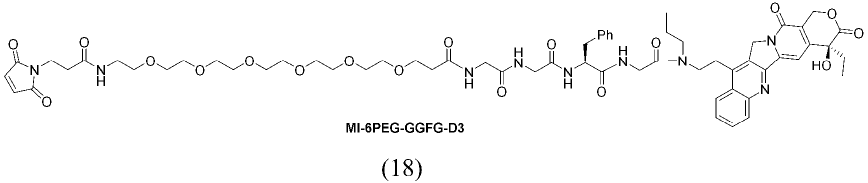 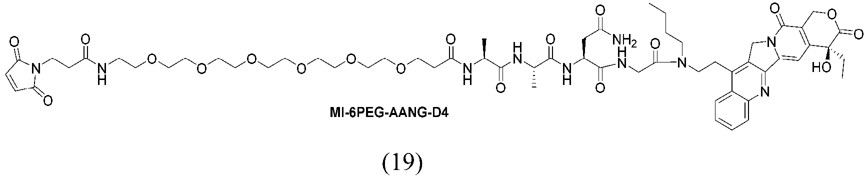 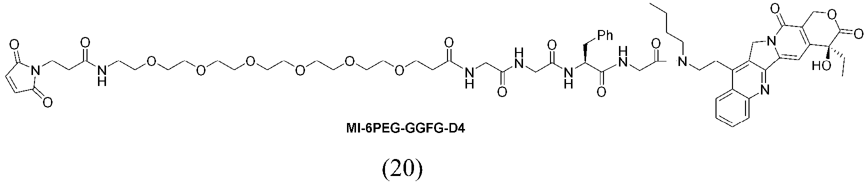 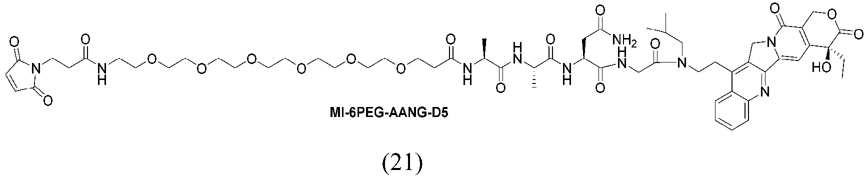 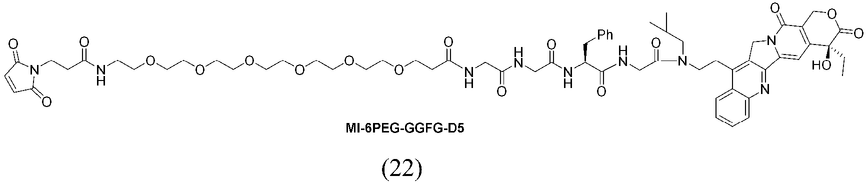 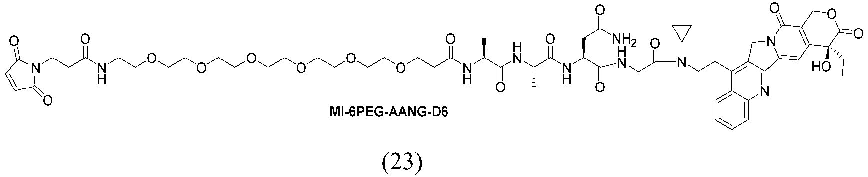 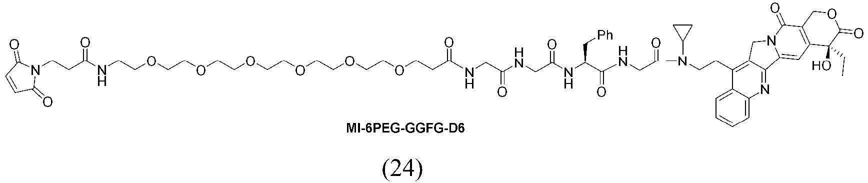 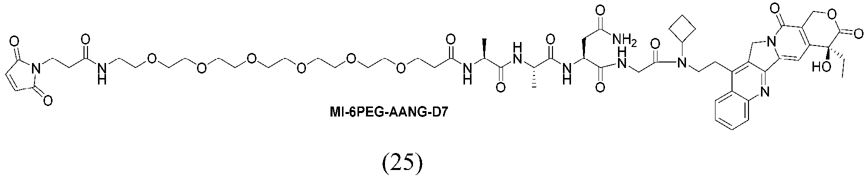 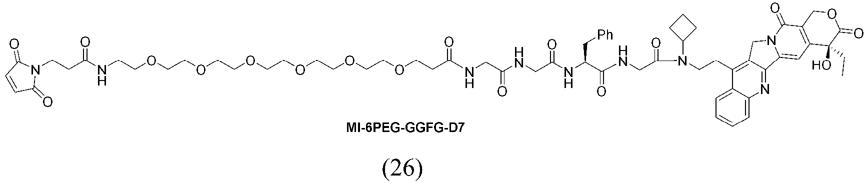 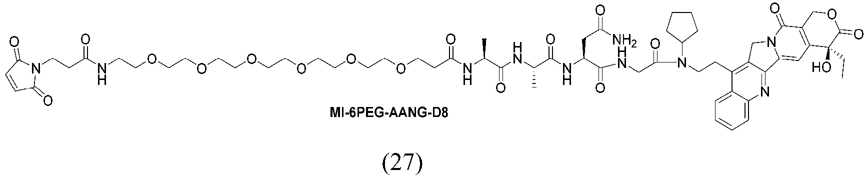 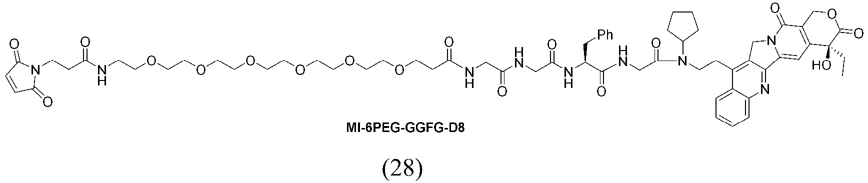 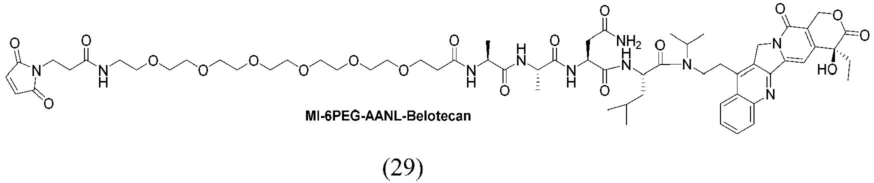 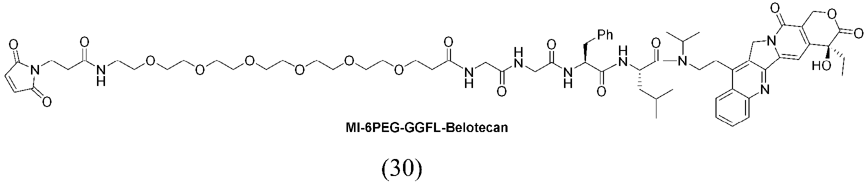 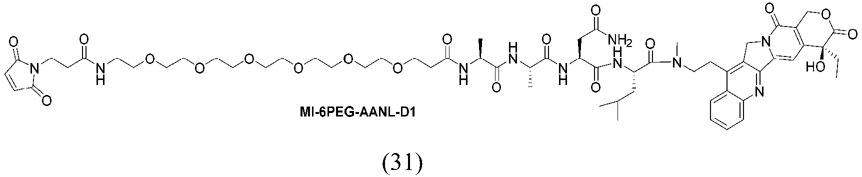 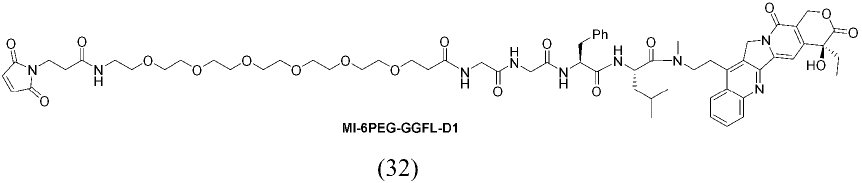 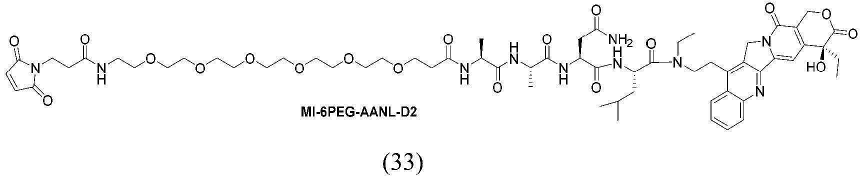 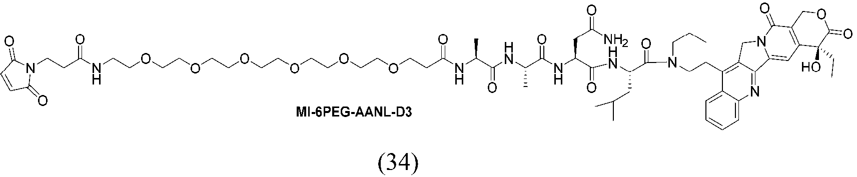 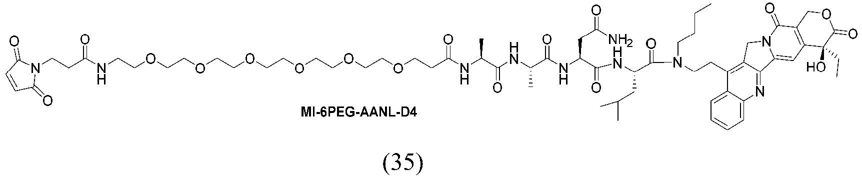 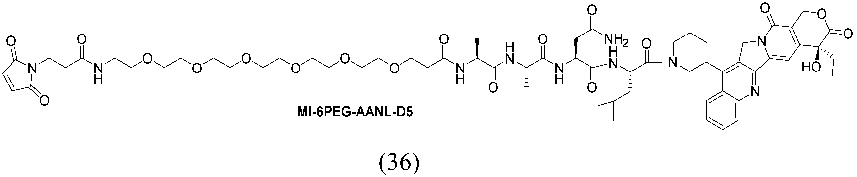 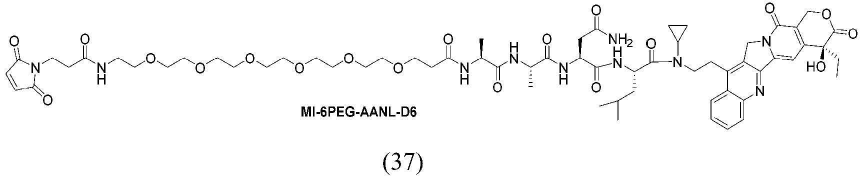 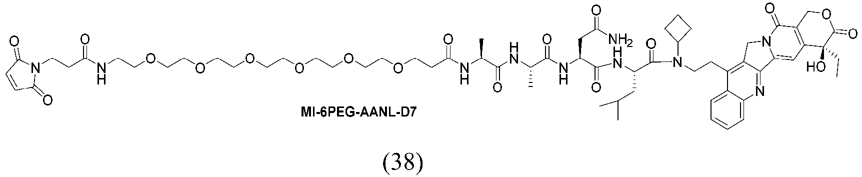 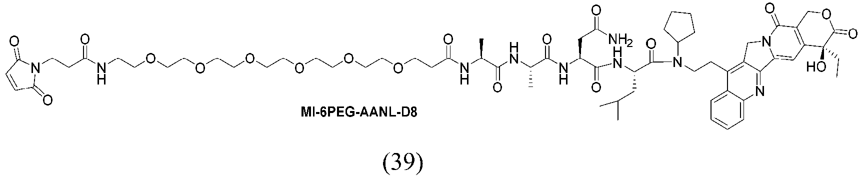 where, G: glycine, A: alanine, T: threonine, S: serine, K: lysine, N: asparagine, F: phenylalanine, PAB: aminobenzyl alcohol, L: leucine, I: isoleucine, and C: citrulline.

The present disclosure further provides a pharmaceutical composition, and the pharmaceutical composition includes a therapeutically effective amount of the polypeptide conjugate described in the present disclosure and a pharmaceutically acceptable carrier.

The present disclosure further provides a use of the polypeptide conjugate or the pharmaceutical composition in preparing an antitumor drug.

Compared with the Belotecan in the prior art, the polypeptide conjugate provided by the present disclosure greatly reduces toxicity and side effects, significantly enhances an inhibitory effect on tumor cells, and significantly improves the safety, which can be well released in tumor tissues rather than normal tissues, thereby achieving targeted release. Clinically, larger doses can be used to treat cancers and improve the overall therapeutic effect.

### BRIEF DESCRIPTION OF THE DRAWINGS

FIGs. 1-1, 1-2 and 1-3 are statistical plots of experimental data from an efficacy study of a Belotecan polypeptide conjugate provided by the present disclosure in treatment of an animal model of sarcoma SJSA-1 (Example 40);
FIGs. 2-1, 2-2 and 2-3 are statistical plots of experimental data from an efficacy study of a Belotecan polypeptide conjugate provided by the present disclosure in treatment of an animal model of A2780 ovarian cancer (Example 41);
FIG. 3 is a statistical plot of experimental data from an efficacy study of a Belotecan polypeptide conjugate provided by the present disclosure in treatment of an animal model of colo320 colorectal cancer (Example 42);
FIG. 4 is a statistical plot of experimental data from an efficacy study of a Belotecan polypeptide conjugate provided by the present disclosure in treatment of an animal model of colo320 colorectal cancer (Example 43);
FIG. 5 is a statistical plot of experimental results of a maximum tolerated dose (MTD) determination of a Belotecan polypeptide conjugate provided by the present disclosure (Example 44).

### DETAILED DESCRIPTIONS OF THE EMBODIMENTS

### [Example 1] Synthesis of MI-6PEG-AAN-PAB-Belotecan

A synthesis method for a polypeptide conjugate MI-6PEG-AAN-PAB-Belotecan is shown as follows:

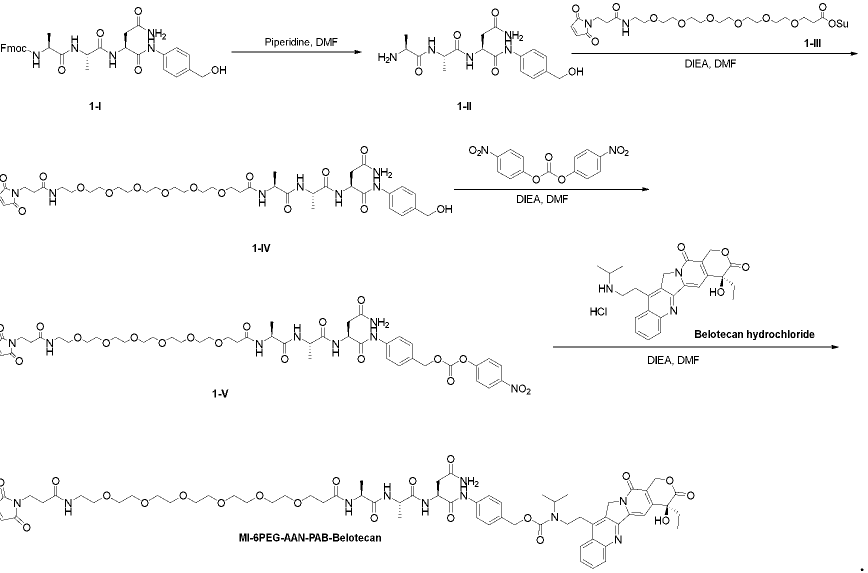

Specific synthesis steps are as follows:

### 1. Synthesis of a compound 1-II

A compound 1-I (500 mg, 0.83 mmol) was dissolved in N,N-dimethylformamide (20 mL), and piperidine (1 mL) was added thereto. A reaction solution reacted at room temperature of 25°C for 1 h. A thin-layer chromatography (TLC) was adopted to detect and ensure that the reaction was complete. The reaction solution was then evaporated to dryness under a reduced pressure. Residues therein were purified through a silica gel column (dichloromethane (DCM): methanol (MeOH) = 10:1) to obtain a light yellow solid compound 1-II (280 mg, a yield of 88.9%).

### 2. Synthesis of a compound 1-IV

The compound 1-II (280 mg, 0.74 mmol) was dissolved in N, N-dimethylformamide (15 mL), and a compound 1-III (450 mg, 0.75 mmol) and diisopropylethylamine (250 mg, 1.94 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a silica gel column (DCM:MeOH = 100:1 to 10:1) to obtain a yellow solid compound 1-IV (310 mg, a yield of 48.4%).

### 3. Synthesis of a compound 1-V

The compound 1-IV (310 mg, 0.36 mmol) was dissolved in N, N-dimethylformamide (15 mL), and bis(4-nitrophenyl) carbonate (180 mg, 0.59 mmol) was added thereto. A reaction solution reacted at room temperature of 25°C for 3 h. A TLC was adopted to detect and ensure that the reaction was complete. The reaction solution was then evaporated to dryness under a reduced pressure. Residues therein were purified through a silica gel column (DCM:MeOH = 100:1 to 12:1) to obtain a yellow solid compound 1-V (190 mg, a yield of 51.2%).

### 4. Synthesis of a compound MI-6PEG-AAN-PAB-Belotecan

Belotecan hydrochloride (80 mg, 0.17 mmol) was dissolved in N, N-dimethylformamide (15 mL), and the compound 1-V (190 mg, 0.18 mmol) and diisopropylethylamine (250 mg, 1.94 mmol) were added thereto. A reaction solution reacted at room temperature of 25°C for 4 h. The reaction solution was then evaporated to dryness under a reduced pressure. Residues therein were purified by high-pressure reversed-phase preparation to obtain yellow solid MI-6PEG-AAN-PAB-Belotecan (18 mg, a yield of 8.0%).

### [Example 2] Synthesis of MI-6PEG-GGFG-Belotecan

A synthesis method for a polypeptide conjugate MI-6PEG-GGFG-Belotecan is shown as follows:

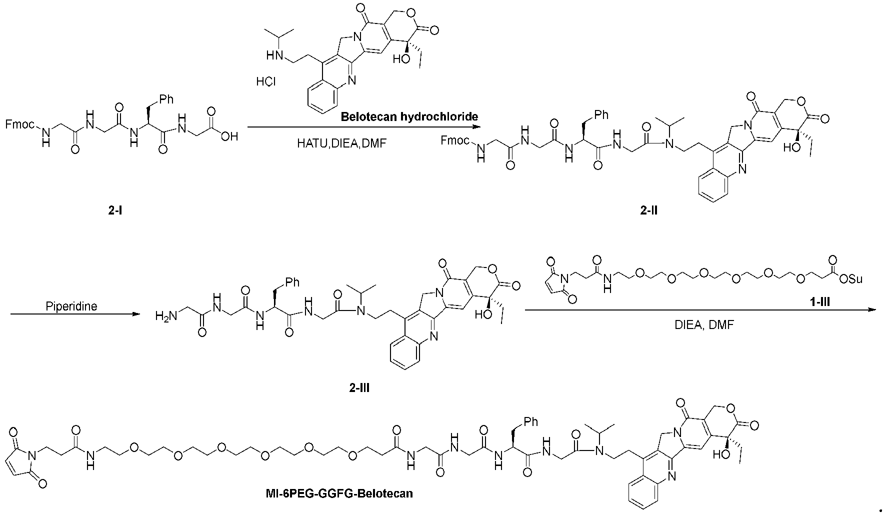

Specific synthesis steps are as follows:

### 1. Synthesis of a compound 2-II

A compound Belotecan hydrochloride (705 mg, 1.50 mmol) and a compound 2-I (850 mg, 1.52 mmol) were dissolved in dichloromethane (30 mL), and O-(7-azabenzotriazol-1-yl)-N,N,N',N'-tetramethyluronium hexafluorophosphate (HATU) (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 2-II (813 mg, a yield of 55.6%).

### 2. Synthesis of a compound 2-III

The compound 2-II (813 mg, 0.83 mmol) was dissolved in N,N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 2-III (560 mg, a yield of 89.7%).

### 3. Synthesis of a compound MI-6PEG-GGFG-Belotecan

The compound 2-III (560 mg, 0.74 mmol) was dissolved in N,N-dimethylformamide (15 mL), and a compound 1-III (580 mg, 0.96 mmol) and diisopropylethylamine (400 mg, 3.10 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFG-Belotecan (284 mg, a yield of 3 1.0%).

### [Example 3] Synthesis of MI-6PEG-AANG-Belotecan

A synthesis method for a polypeptide conjugate MI-6PEG-AANG-Belotecan is shown as follows:

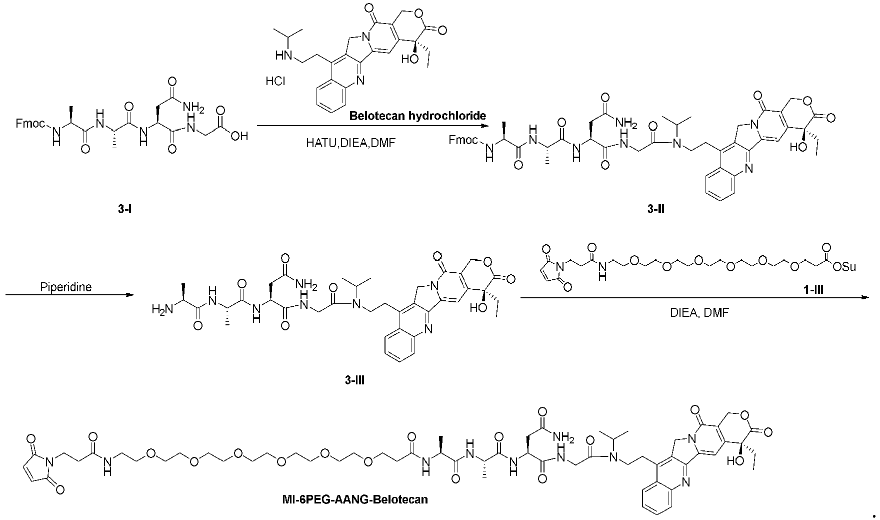

### Specific synthesis steps are as follows:

### 1. Synthesis of a compound 3-II

A compound Belotecan hydrochloride (705 mg, 1.50 mmol) and a compound 3-I (800 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 3-II (860 mg, a yield of 59.2%).

### 2. Synthesis of a compound 3-III

The compound 3-II (860 mg, 0.89 mmol) was dissolved in N,N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 3-III (580 mg, a yield of 87.6%).

### 3. Synthesis of a compound MI-6PEG-AANG-Belotecan

A compound 3-III (580 mg, 0.78 mmol) was dissolved in N,N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain a yellow solid compound MI-6PEG-AANG-Belotecan (220 mg, a yield of 22.9%).

### [Example 4] Synthesis of MI-6PEG-AANG-D1

A synthesis method for a polypeptide conjugate MI-6PEG-AANG-D1 is shown as follows:

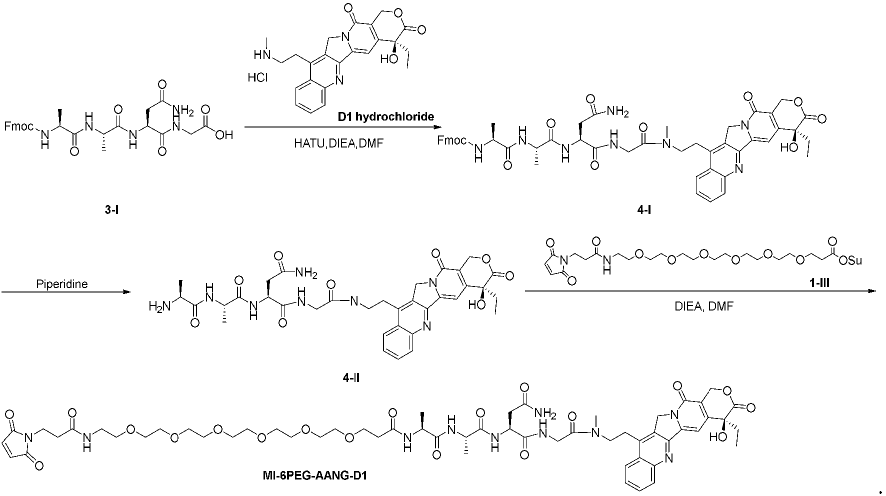

Specific synthesis steps are as follows:

### 1. Synthesis of a compound 4-I

A compound D1 hydrochloride (663 mg, 1.50 mmol) and a compound 3-I (800 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 4-I (855 mg, a yield of 60.6%).

### 2. Synthesis of a compound 4-II

The compound 4-I (855 mg, 0.91 mmol) was dissolved in N,N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 4-II (615 mg, a yield of 94.0%).

### 3. Synthesis of a compound MI-6PEG-AANG-D1

The compound 4-II (615 mg, 0.86 mmol) was dissolved in N,N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AANG-D1 (190 mg, a yield of 18.3%).

### [Example 5] Synthesis of MI-6PEG-GGFG-D1

A synthesis method for a polypeptide conjugate MI-6PEG-GGFG-D1 is shown as follows:

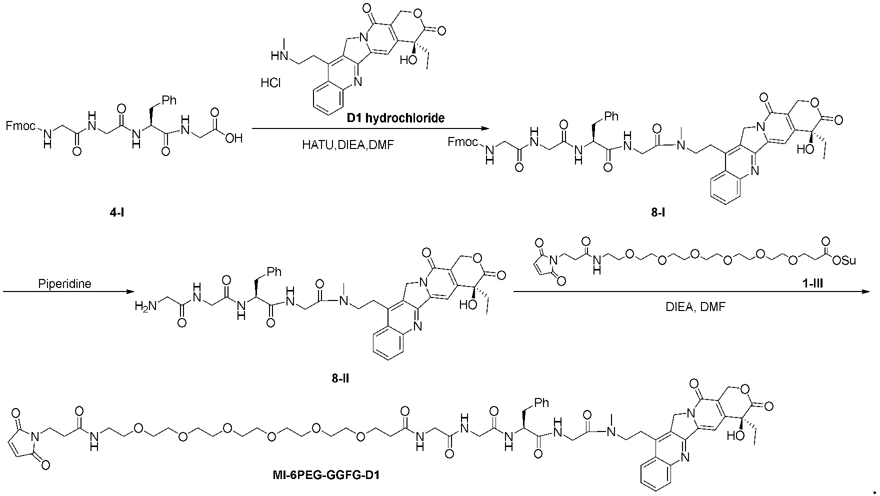

Specific synthesis steps are as follows:

### 1. Synthesis of a compound 8-I

A compound D1 hydrochloride (663 mg, 1.50 mmol) and a compound 4-I (850 mg, 1.52 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 8-I (694 mg, a yield of 48.9%).

### 2. Synthesis of a compound 8-II

The compound 8-I (694 mg, 0.73 mmol) was dissolved in N,N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 8-II (498 mg, a yield of 94.3%).

### 3. Synthesis of a compound MI-6PEG-GGFG-D1

The compound 8-II (498 mg, 0.69 mmol) was dissolved in N,N-dimethylformamide (15 mL), and a compound 1-III (580 mg, 0.96 mmol) and diisopropylethylamine (400 mg, 3.10 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFG-D1 (191 mg, a yield of 22.9%).

### [Example 6] Synthesis of MI-6PEG-GGFL-D1

A synthesis method for a polypeptide conjugate MI-6PEG-GGFL-D1 is shown as follows:

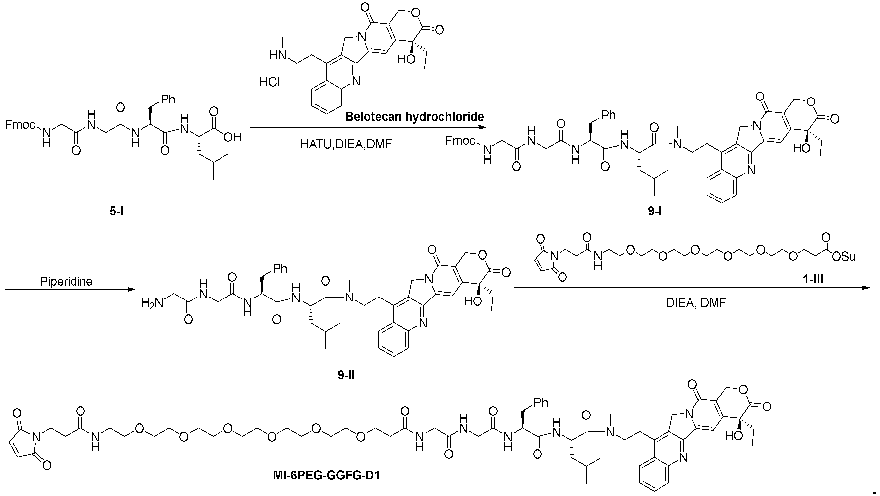

Specific synthesis steps are as follows:

### 1. Synthesis of a compound 9-I

A compound D1 hydrochloride (663 mg, 1.50 mmol) and a compound 5-I (935 mg, 1.52 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 9-I (742 mg, a yield of 49.4%).

### 2. Synthesis of a compound 9-II

The compound 9-I (742 mg, 0.74 mmol) was dissolved in N,N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 9-II (454 mg, a yield of 78.7%).

### 3. Synthesis of a compound MI-6PEG-GGFL-D1

The compound 9-II (454 mg, 0.58 mmol) was dissolved in N,N-dimethylformamide (15 mL), and a compound 1-III (520 mg, 0.86 mmol) and diisopropylethylamine (350 mg, 2.71 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFL-D1 (159 mg, a yield of 21.6%).

### [Example 7] Synthesis of MI-6PEG-AANG-D2

A synthesis method for a polypeptide conjugate MI-6PEG-AANG-D2 is shown as follows:

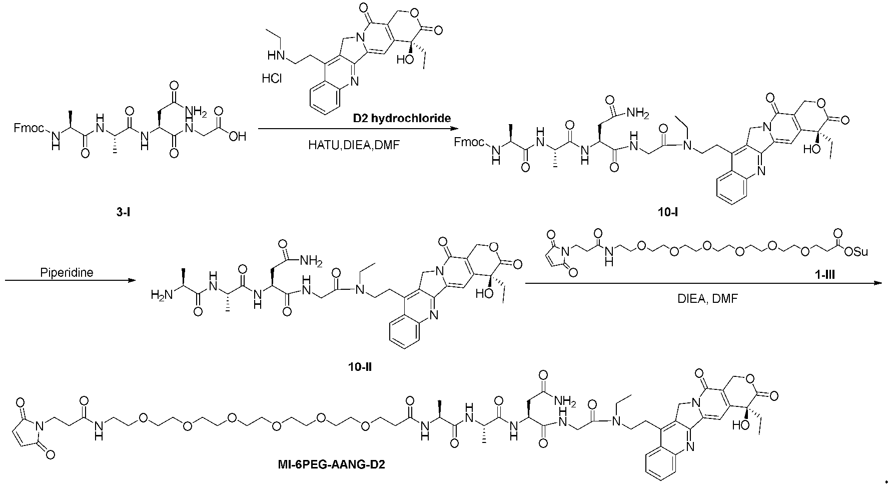

Specific synthesis steps are as follows:

### 1. Synthesis of a compound 10-I

A compound D2 hydrochloride (685 mg, 1.50 mmol) and a compound 3-I (800 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 10-I (810 mg, a yield of 56.5%).

### 2. Synthesis of a compound 10-II

The compound 10-I (810 mg, 0.85 mmol) was dissolved in N,N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 10-II (545 mg, a yield of 86.5%).

### 3. Synthesis of a compound MI-6PEG-AANG-D2

The compound 10-II (545 mg, 0.74 mmol) was dissolved in N,N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AANG-D2 (138 mg, a yield of 15.3%).

### [Example 8] Synthesis of MI-6PEG-GGFG-D2

A synthesis method for a polypeptide conjugate MI-6PEG-GGFG-D2 is shown as follows:

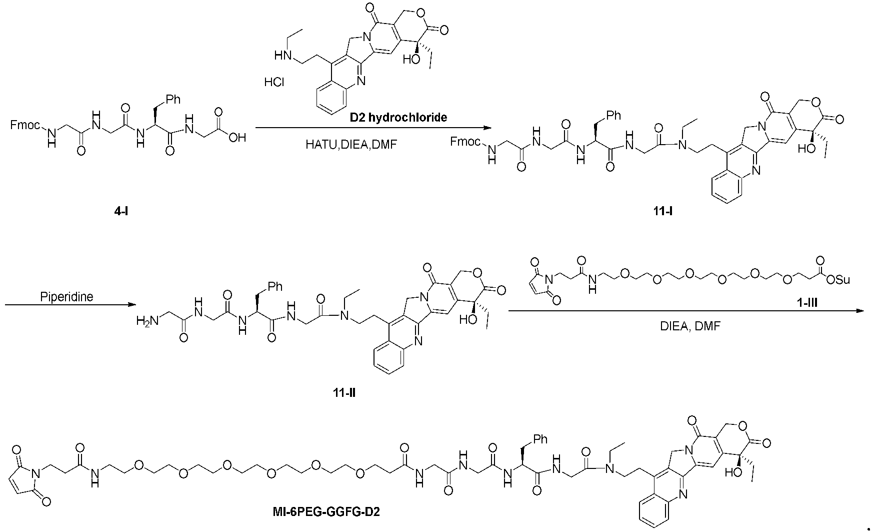

Specific synthesis steps are as follows:

### 1. Synthesis of a compound 11-I

A compound D2 hydrochloride (685 mg, 1.50 mmol) and a compound 4-I (850 mg, 1.52 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 11-I (615 mg, a yield of 42.7%).

### 2. Synthesis of a compound 11-II

The compound 11-I (615 mg, 0.64 mmol) was dissolved in N,N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 11-II (400 mg, a yield of 84.7%).

### 3. Synthesis of a compound MI-6PEG-GGFG-D2

The compound 11-II (400 mg, 0.54 mmol) was dissolved in N,N-dimethylformamide (15 mL), and a compound 1-III (580 mg, 0.96 mmol) and diisopropylethylamine (400 mg, 3.10 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFG-D2 (93 mg, a yield of 14.1%).

### [Example 9] Synthesis of MI-6PEG-GANG-D2

A synthesis method for a polypeptide conjugate MI-6PEG-GANG-D2 is shown as follows:

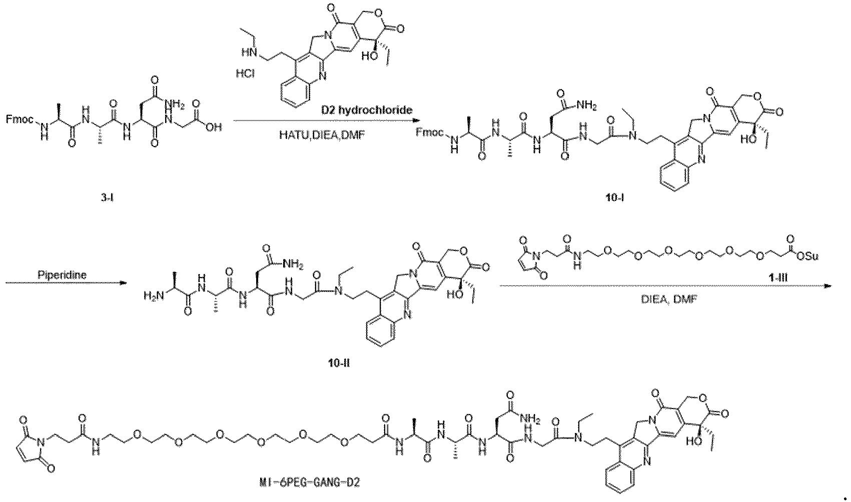

Specific synthesis steps are as follows:

### 1. Synthesis of a compound 12-II

A compound D2 hydrochloride (685 mg, 1.50 mmol) and a compound 12-I (810 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 12-II (765 mg, a yield of 54.2%).

### 2. Synthesis of a compound 12-III

The compound 12-II (765 mg, 0.81 mmol) was dissolved in N,N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 12-III (498 mg, a yield of 85.5%).

### 3. Synthesis of a compound MI-6PEG-GANG-D2

The compound 12-III (498 mg, 0.69 mmol) was dissolved in N,N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-GANG-D2 (115 mg, a yield of 13.8%).

### [Example 10] Synthesis of MI-6PEG-TANG-D2

A synthesis method for a polypeptide conjugate MI-6PEG-TANG-D2 is shown as follows:

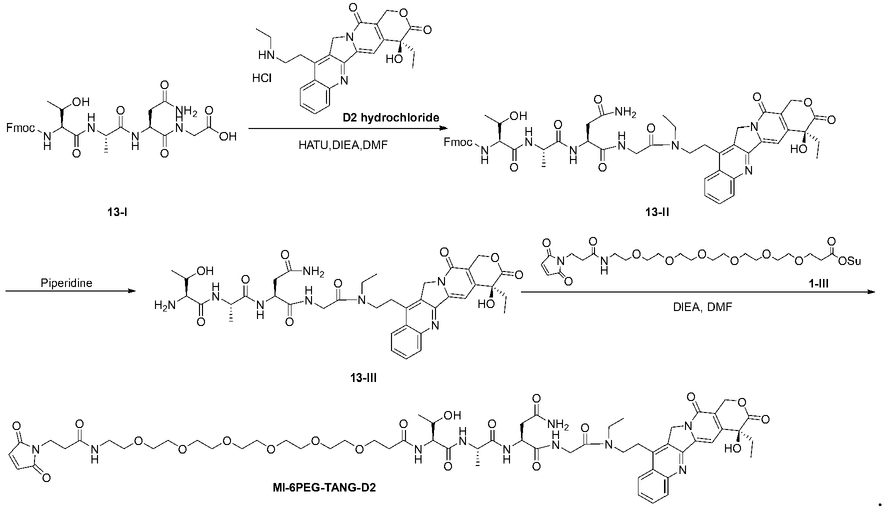

Specific synthesis steps are as follows:

### 1. Synthesis of a compound 13-II

A compound D2 hydrochloride (685 mg, 1.50 mmol) and a compound 13-1 (875 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 13-II (846 mg, a yield of 57.3%).

### 2. Synthesis of a compound 13-III

The compound 13-II (846 mg, 0.86 mmol) was dissolved in N,N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 13-III (552 mg, a yield of 84.1%).

### 3. Synthesis of a compound MI-6PEG-TANG-D2

The compound 13-III (552 mg, 0.72 mmol) was dissolved in N,N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-TANG-D2 (165 mg, a yield of 18.3%).

### [Example 11] Synthesis of MI-6PEG-SANG-D2

A synthesis method for a polypeptide conjugate MI-6PEG-SANG-D2 is shown as follows:

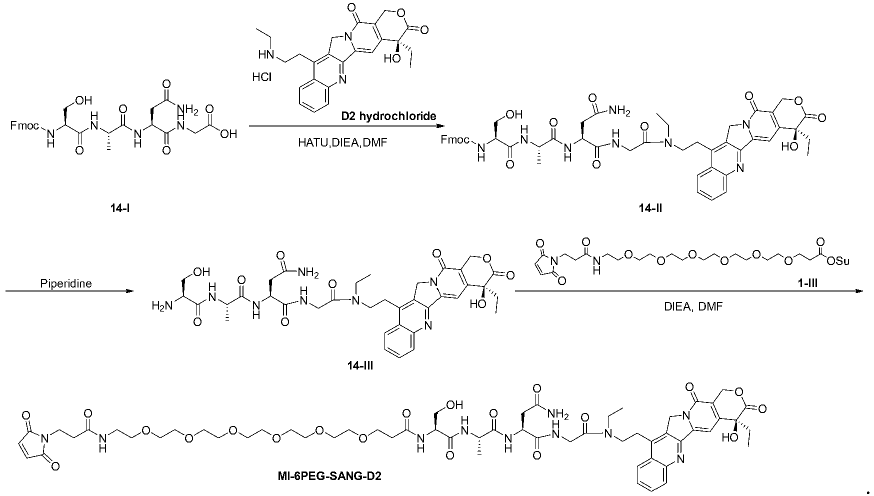

Specific synthesis steps are as follows:

### 1. Synthesis of a compound 14-II

A compound D2 hydrochloride (685 mg, 1.50 mmol) and a compound 14-I (855 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 14-II (893 mg, a yield of 61.3%).

### 2. Synthesis of a compound 14-III

The compound 14-II (893 mg, 0.92 mmol) was dissolved in N,N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 14-III (601 mg, a yield of 87.2%).

### 3. Synthesis of a compound MI-6PEG-SANG-D2

The compound 14-III (601 mg, 0.80 mmol) was dissolved in N,N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-SANG-D2 (225 mg, a yield of 22.8%).

### [Example 12] Synthesis of MI-6PEG-FANG-D2

A synthesis method for a polypeptide conjugate MI-6PEG-FANG-D2 is shown as follows:

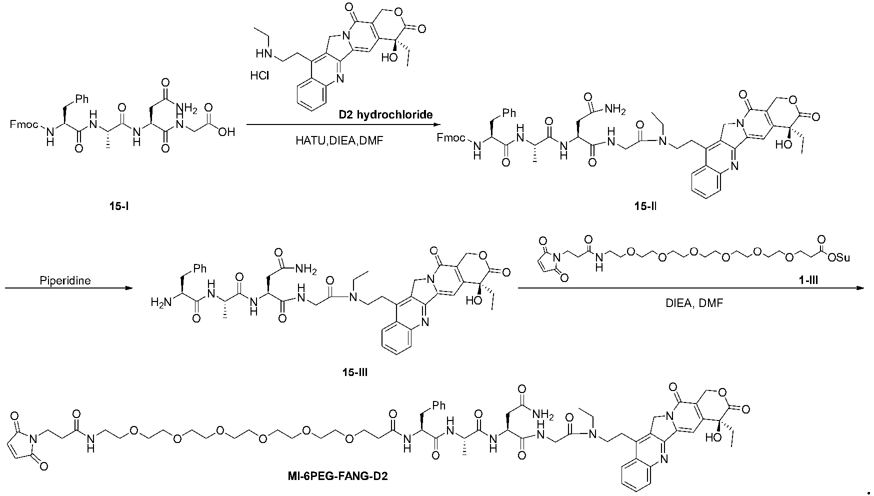

Specific synthesis steps are as follows:

### 1. Synthesis of a compound 15-II

A compound D2 hydrochloride (685 mg, 1.50 mmol) and a compound 15-I (945 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 15-II (922 mg, a yield of 59.6%).

### 2. Synthesis of a compound 15-III

The compound 15-II (922 mg, 0.89 mmol) was dissolved in N,N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 15-III (624 mg, a yield of 86.7%).

### 3. Synthesis of a compound MI-6PEG-FANG-D2

The compound 15-III (624 mg, 0.77 mmol) was dissolved in N,N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-FANG-D2 (200 mg, a yield of 20.1%).

### [Example 13] Synthesis of MI-6PEG-AAAG-D2

A synthesis method for a polypeptide conjugate MI-6PEG-AAAG-D2 is shown as follows:

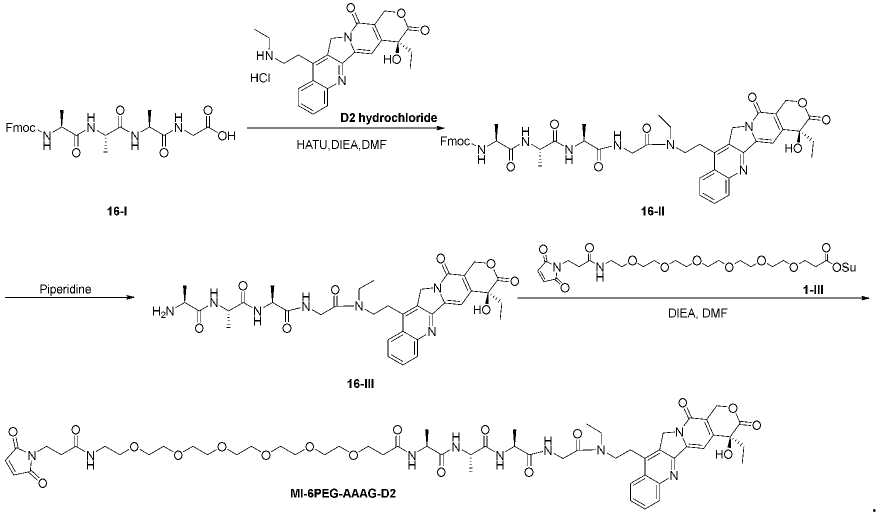

Specific synthesis steps are as follows:

### 1. Synthesis of a compound 16-II

A compound D2 hydrochloride (685 mg, 1.50 mmol) and a compound 16-I (765 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 16-II (695 mg, a yield of 50.8%).

### 2. Synthesis of a compound 16-III

The compound 16-II (695 mg, 0.76 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM: MeOH = 100:1 to 5:1) to obtain a yellow solid compound 16-III (488 mg, a yield of 93.1%).

### 3. Synthesis of a compound MI-6PEG-AAAG-D2

The compound 16-III (488 mg, 0.71 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AAAG-D2 (145 mg, a yield of 17.4%).

### [Example 14] Synthesis of MI-6PEG-AAKG-D2

A synthesis method for a polypeptide conjugate MI-6PEG-AAKG-D2 is shown as follows:

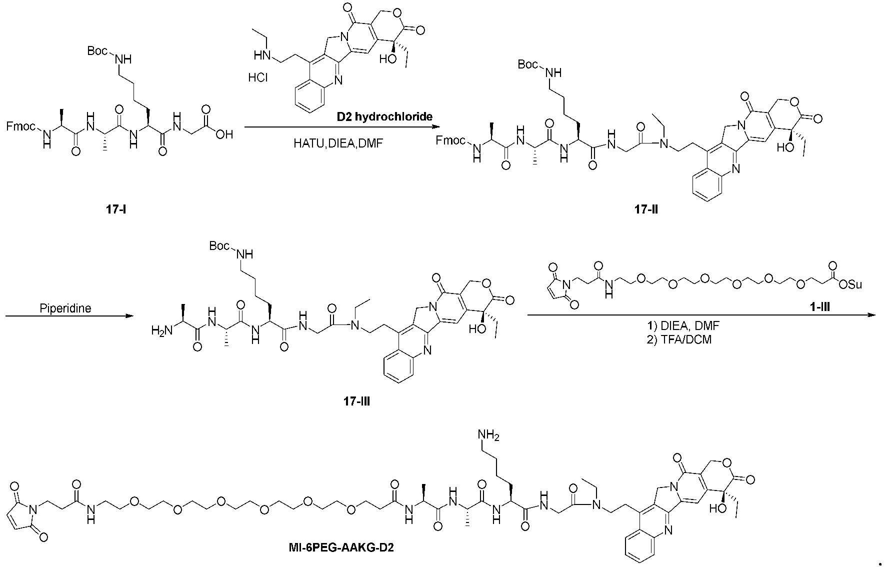

Specific synthesis steps are as follows:

### 1. Synthesis of a compound 17-II

A compound D2 hydrochloride (685 mg, 1.50 mmol) and a compound 17-I (1.0 g, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 17-II (900 mg, a yield of 56.1%).

### 2. Synthesis of a compound 17-III

The compound 17-II (900 mg, 0.84 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 17-III (680 mg, a yield of 95.6%).

### 3. Synthesis of a compound MI-6PEG-AAKG-D2

The compound 17-III (680 mg, 0.80 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was evaporated to dryness under a reduced pressure. Residues therein were dissolved in dichloromethane (10 mL), and 3 mL of trifluoroacetic acid was added thereto. A reaction solution reacted at room temperature of 25°C for 2 h, and LC-MS was adopted to detect and ensure that the reaction was complete. The reaction solution was evaporated to dryness under a reduced pressure, and residues therein were purified through a reversed-phase column to obtain an off-white solid compound MI-6PEG-AAKG-D2 (185 mg, a yield of 18.8%).

### [Example 15] Synthesis of MI-6PEG-AACG-D2

A synthesis method for a polypeptide conjugate MI-6PEG-AACG-D2 is shown as follows:

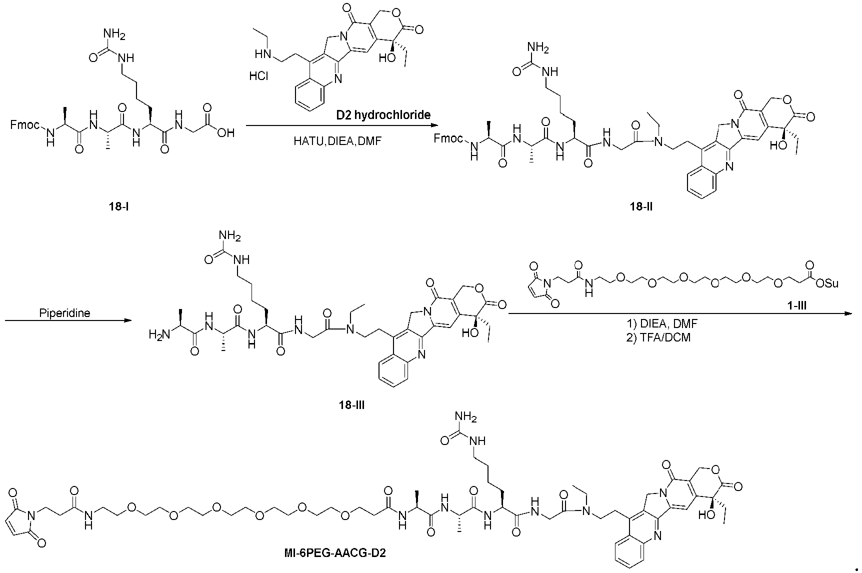

Specific synthesis steps are as follows:

### 1. Synthesis of a compound 18-II

A compound D2 hydrochloride (685 mg, 1.50 mmol) and a compound 18-I (915 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 18-II (860 mg, a yield of 56.7%).

### 2. Synthesis of a compound 18-III

The compound 18-II (860 mg, 0.85mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM: MeOH = 100:1 to 5:1) to obtain a yellow solid compound 18-III (596 mg, a yield of 88.8%).

### 3. Synthesis of a compound MI-6PEG-AACG-D2

The compound 18-III (596 mg, 0.75 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AACG-D2 (119 mg, a yield of 12.4%).

### [Example 16] Synthesis of MI-6PEG-GGGP-D2

A synthesis method for a polypeptide conjugate MI-6PEG-GGGP-D2 is shown as follows:

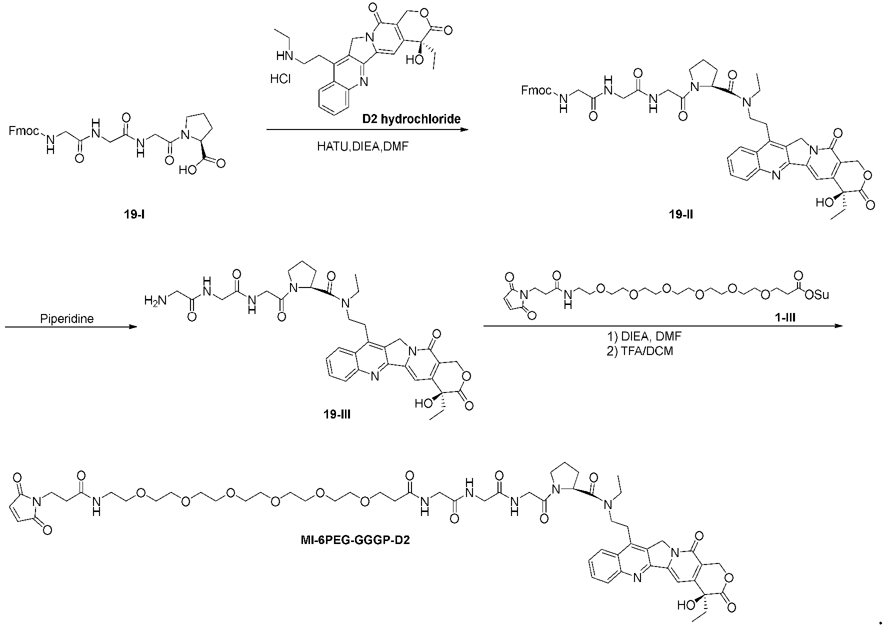

Specific synthesis steps are as follows:

### 1. Synthesis of a compound 19-II

A compound D2 hydrochloride (685 mg, 1.50 mmol) and a compound 19-I (765 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 19-II (585 mg, a yield of 42.9%).

### 2. Synthesis of a compound 19-III

The compound 19-II (585 mg, 0.64 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 19-III (410 mg, a yield of 93.1%).

### 3. Synthesis of a compound MI-6PEG-GGGP-D2

The compound 19-III (410 mg, 0.60 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-GGGP-D2 (90 mg, a yield of 12.8%).

### [Example 17] Synthesis of MI-6PEG-AAGP-D2

A synthesis method for a polypeptide conjugate MI-6PEG-AAGP-D2 is shown as follows:

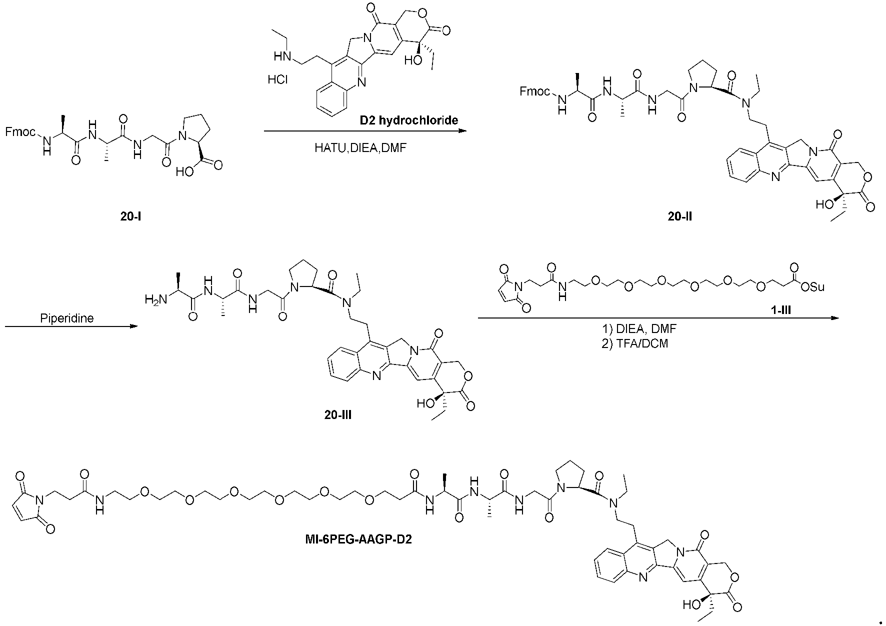

Specific synthesis steps are as follows:

### 1. Synthesis of a compound 20-II

A compound D2 hydrochloride (685 mg, 1.50 mmol) and a compound 20-I (805 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 20-II (615 mg, a yield of 43.7%).

### 2. Synthesis of a compound 20-III

The compound 20-II (615 mg, 0.66 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 20-III (422 mg, a yield of 89.3%).

### 3. Synthesis of a compound MI-6PEG-AAGP-D2

The compound 20-III (422 mg, 0.59 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AAGP-D2 (115 mg, a yield of 16.2%).

### [Example 18] Synthesis of MI-6PEG-AANG-D3

A synthesis method for a polypeptide conjugate MI-6PEG-AANG-D3 is shown as follows:

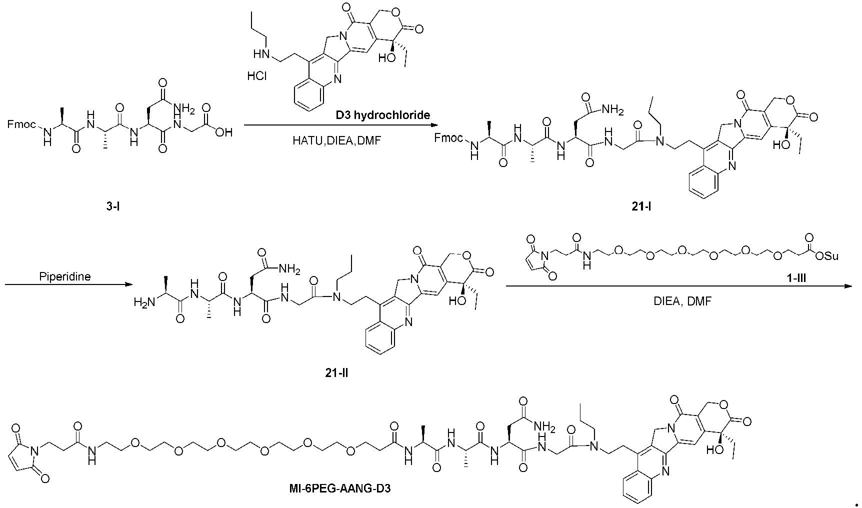

Specific synthesis steps are as follows:

### 1. Synthesis of a compound 21-I

A compound D3 hydrochloride (705 mg, 1.50 mmol) and a compound 3-I (800 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 21-I (760 mg, a yield of 52.3%).

### 2. Synthesis of a compound 21-II

The compound 21-I (760 mg, 0.78 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 21-II (420 mg, a yield of 72.1%).

### 3. Synthesis of a compound MI-6PEG-AANG-D3

The compound 21-II (420 mg, 0.56 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AANG-D3 (86 mg, a yield of 12.5%).

### [Example 19] Synthesis of MI-6PEG-GGFG-D3

A synthesis method for a polypeptide conjugate MI-6PEG-GGFG-D3 is shown as follows:

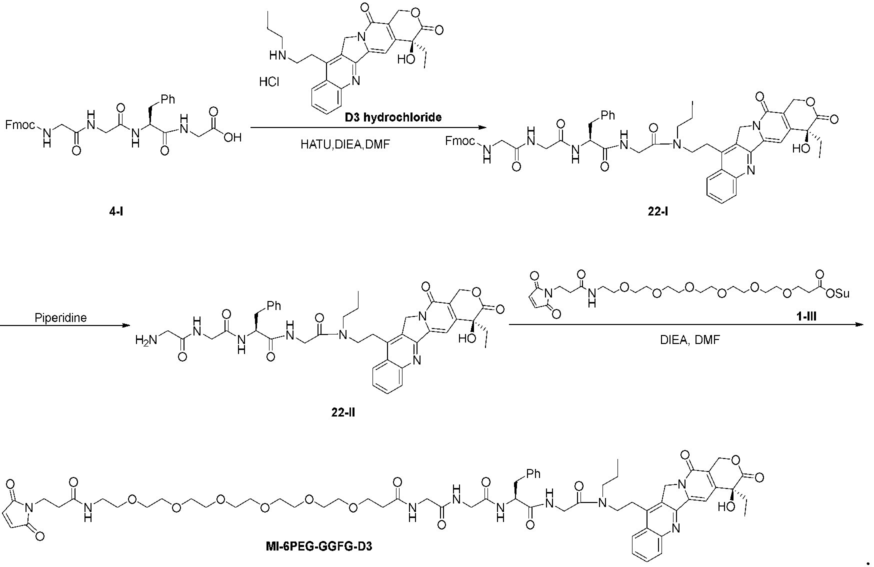

Specific synthesis steps are as follows:

### 1. Synthesis of a compound 22-I

A compound D3 hydrochloride (705 mg, 1.50 mmol) and a compound 4-I (850 mg, 1.52 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 22-I (545 mg, a yield of 37.3%).

### 2. Synthesis of a compound 22-II

The compound 22-I (545 mg, 0.56 mmol) was dissolved in N, N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 22-II (320 mg, a yield of 76.0%).

### 3. Synthesis of a compound MI-6PEG-GGFG-D3

The compound 22-II (320 mg, 0.42 mmol) was dissolved in N, N-dimethylformamide (15 mL), and a compound 1-III (580 mg, 0.96 mmol) and diisopropylethylamine (400 mg, 3.10 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFG-D3 (102 mg, a yield of 19.6%).

### [Example 20] Synthesis of MI-6PEG-AANG-D4

A synthesis method for a polypeptide conjugate MI-6PEG-AANG-D4 is shown as follows:

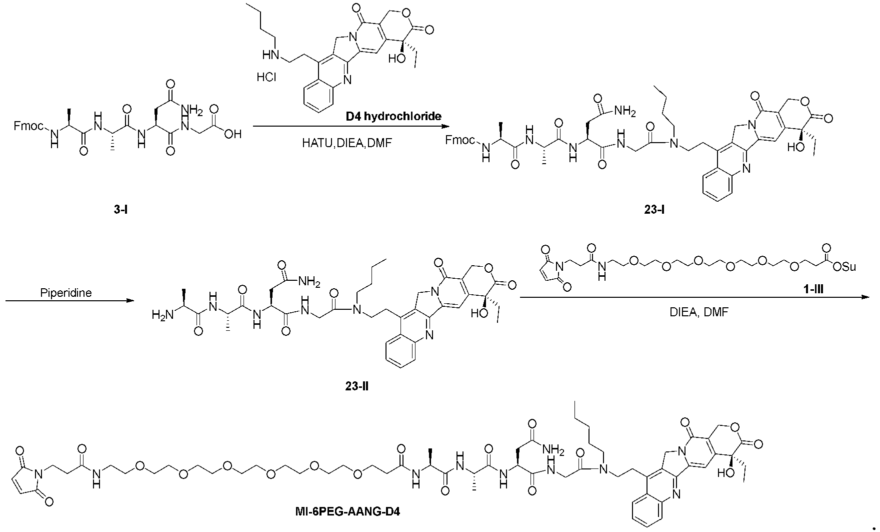

Specific synthesis steps are as follows:

### 1. Synthesis of a compound 23-I

A compound D4 hydrochloride (725 mg, 1.50 mmol) and a compound 3-I (800 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 23-I (820 mg, a yield of 55.6%).

### 2. Synthesis of a compound 23-II

The compound 23-I (820 mg, 0.83 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM: MeOH = 100:1 to 5:1) to obtain a yellow solid compound 23-II (595 mg, a yield of 94.2%).

### 3. Synthesis of a compound MI-6PEG-AANG-D4

The compound 23-II (595 mg, 0.78 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AANG-D4 (135 mg, a yield of 13.7%).

### [Example 21] Synthesis of MI-6PEG-GGFG-D4

A synthesis method for a polypeptide conjugate MI-6PEG-GGFG-D4 is shown as follows:

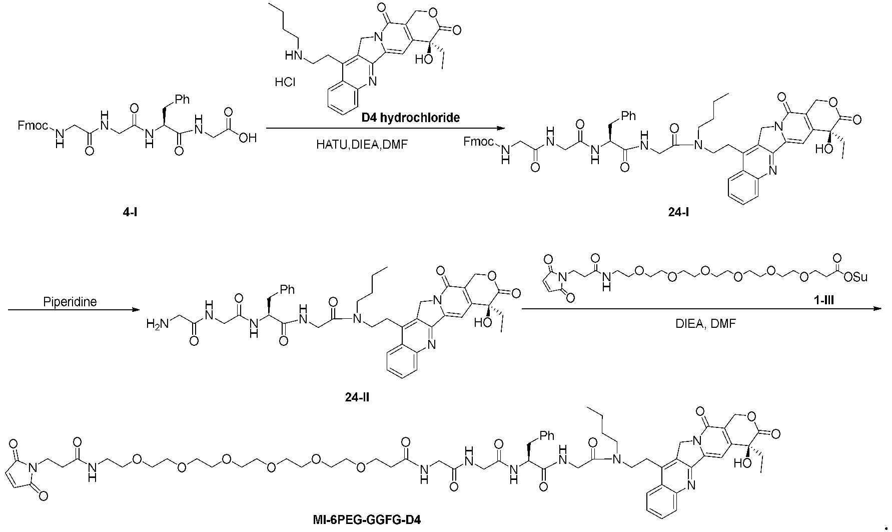

Specific synthesis steps are as follows:

### 1. Synthesis of a compound 24-I

A compound D4 hydrochloride (725 mg, 1.50 mmol) and a compound 4-I (850 mg, 1.52 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM: MeOH = 100:1 to 8:1) to obtain a yellow solid compound 24-I (610 mg, a yield of 41.2%).

### 2. Synthesis of a compound 24-II

The compound 24-I (610 mg, 0.62 mmol) was dissolved in N, N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 24-II (403 mg, a yield of 84.9%).

### 3. Synthesis of a compound MI-6PEG-GGFG-D4

The compound 24-II (403 mg, 0.53 mmol) was dissolved in N, N-dimethylformamide (15 mL), and a compound 1-III (580 mg, 0.96 mmol) and diisopropylethylamine (400 mg, 3.10 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFG-D4 (122 mg, a yield of 18.4%).

### [Example 22] Synthesis of MI-6PEG-AANG-D5

A synthesis method for a polypeptide conjugate MI-6PEG-AANG-D5 is shown as follows:

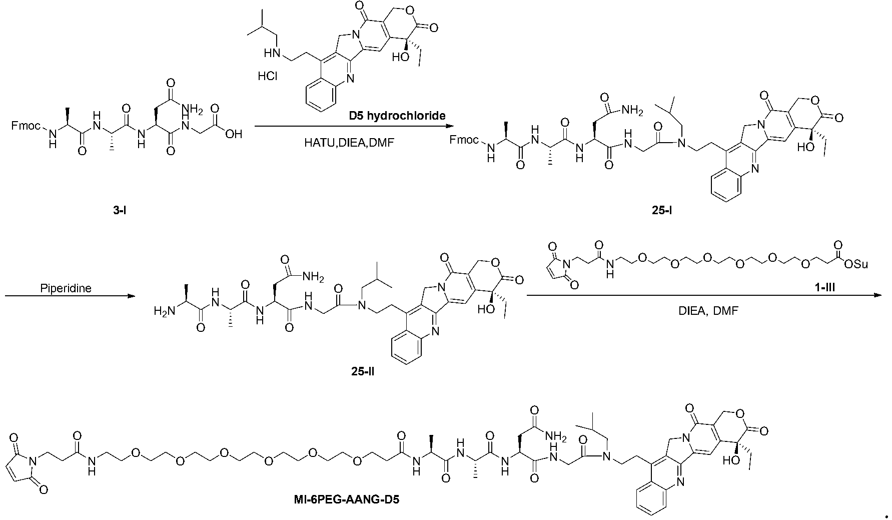

Specific synthesis steps are as follows:

### 1. Synthesis of a compound 25-I

A compound D5 hydrochloride (725 mg, 1.50 mmol) and a compound 3-I (800 mg, 1.50 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 25-I (489 mg, a yield of 33.2%).

### 2. Synthesis of a compound 25-II

The compound 25-I (489 mg, 0.50 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 25-II (350 mg, a yield of 84.1%).

### 3. Synthesis of a compound MI-6PEG-AANG-D5

The compound 25-II (350 mg, 0.42 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AANG-D5 (42 mg, a yield of 8.0%).

### [Example 23] Synthesis of MI-6PEG-GGFG-D5

A synthesis method for a polypeptide conjugate MI-6PEG-GGFG-D5 is shown as follows:

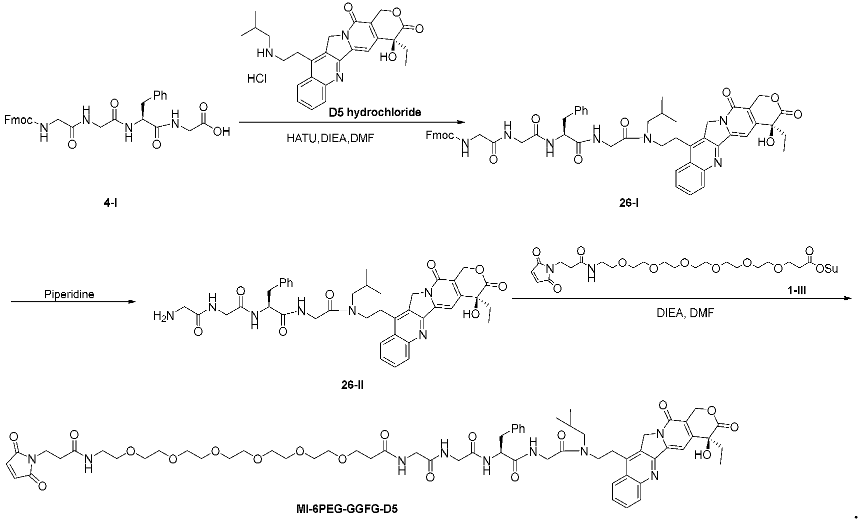

Specific synthesis steps are as follows:

### 1. Synthesis of a compound 26-I

A compound D5 hydrochloride (725 mg, 1.50 mmol) and a compound 4-I (850 mg, 1.52 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 24-I (410 mg, a yield of 27.7%).

### 2. Synthesis of a compound 26-II

The compound 26-I (410 mg, 0.41 mmol) was dissolved in N, N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM: MeOH = 100:1 to 5:1) to obtain a yellow solid compound 26-II (298 mg, a yield of 94.9%).

### 3. Synthesis of a compound MI-6PEG-GGFG-D5

The compound 26-II (298 mg, 0.39 mmol) was dissolved in N, N-dimethylformamide (15 mL), and a compound 1-III (480 mg, 0.79 mmol) and diisopropylethylamine (400 mg, 3.10 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFG-D5 (33 mg, a yield of 6.8%).

### [Example 24] Comparison of toxicity experiments of Belotecan compounds in sarcoma and ovarian cancer cell lines (CCK8)

Different types of cells were cultured with complete media (RPMI1640 (DMEM high sugar medium) + 10% fetal bovine serum + 1XP/S + sodium pyruvate 1 mM solution) in an incubator at 37°C and 5% CO₂ until the cells grew to a sufficient number, and the cells were collected, centrifuged at 1,000 g for 5 min, resuspended with an appropriate volume of a 10% RMPI1640 (DMEM high sugar medium) medium and counted. In a 96-well culture plate, 100 µL of cell culture fluid containing different concentrations of drugs was added, control wells (0.1% DMSO) with only corresponding drug solvents without drugs, as well as blank wells (Blank) with only the medium but without cells were set, and three parallel wells were set in each group. After that, the cells after cell counting were inoculated onto 96-well culture plates, with 100 µL of cell suspension per well, and an inoculation concentration of the cells was 5,000 cells (100 µL)/well. The plates were then cultured in a CO₂ (5%) incubator at 37°C for 48 h. After 48 h, 10 µL of a cell proliferation staining reagent (CCK8) was added to each well, the plates were incubated in a cell incubator for around 2 h, and an absorbance at 450 nm was detected.

Cell survival rates and median inhibitory concentrations of the drugs on the cells were calculated.

Specific experimental results of the median inhibitory concentrations (IC50) of the drugs on the cells are shown in the following table (in ug/mL):

**Table 1 Toxicity of polypeptide conjugate, Irinotecan and Belotecan in different cell lines**

| Compound name | A2780 (ovarian cancer) | SJSA-1 (sarcoma tissue) | SKBR3 (breast cancer tissue) | A549 (non-small cell lung cancer) |
|---|---|---|---|---|
| Belotecan | 0.0044 | 0.0021 | 0.0098 | 0.0066 |
| D1 | 0.018 | 0.028 | 0.050 | 0.027 |
| D2 | 0.018 | 0.023 | 0.036 | 0.030 |
| D3 | 0.021 | 0.029 | 0.049 | 0.026 |
| D4 | 0.029 | 0.054 | 0.076 | 0.043 |
| D5 | 0.014 | 0.012 | 0.013 | 0.019 |
| D6 | 0.0029 | 0.0018 | 0.0038 | 0.0025 |
| D7 | 0.0043 | 0.0084 | 0.016 | 0.0074 |
| D8 | 0.015 | 0.035 | 0.047 | 0.016 |
| Irinotecan | 0.16 | 0.11 | 0.25 | 0.11 |
| MI-6PEG-AAN-PAB-Belotec an | 0.34 | 0.26 | 0.40 | 0.39 |
| MI-6PEG-AANG-Belotecan | 1.27 | 1.18 | 2.88 | 2.32 |
| MI-6PEG-GGFG-Belotecan | 2.60 | 1.50 | 6.15 | 3.61 |
| MI-6PEG-AANG-D1 | 2.42 | 2.96 | 3.32 | 4.36 |
| MI-6PEG-GGFG-D1 | 6.84 | 11.79 | 16.49 | 10.78 |
| MI-6PEG-AANG-D2 | 2.52 | 2.25 | 3.28 | 4.36 |
| MI-6PEG-GGFG-D2 | 5.20 | 10.91 | 14.48 | 13.38 |
| MI-6PEG-GANG-D2 | 2.20 | 1.70 | 3.88 | 2.39 |
| MI-6PEG-TANG-D2 | 3.37 | 2.38 | 6.56 | 8.71 |
| MI-6PEG-SANG-D2 | 3.21 | 1.95 | 4.90 | 5.40 |
| MI-6PEG-FANG-D2 | 2.33 | 2.66 | 5.36 | 3.84 |
| MI-6PEG-AAAG-D2 | 2.50 | 3.80 | 4.19 | 2.71 |
| MI-6PEG-AAKG-D2 | 0.80 | 0.49 | 2.62 | 1.32 |
| MI-6PEG-AACG-D2 | 7.64 | 16.20 | 36.92 | 28.01 |
| MI-6PEG-GGGP-D2 | 1.25 | 2.96 | 8.95 | 6.92 |
| MI-6PEG-AAGP-D2 | 0.71 | 0.85 | 2.25 | 1.99 |
| MI-6PEG-AANG-D3 | 1.80 | 1.63 | 2.42 | 3.41 |
| MI-6PEG-GGFG-D3 | 3.61 | 6.99 | 18.24 | 7.69 |
| MI-6PEG-AANG-D4 | 4.88 | 2.52 | 4.54 | 6.37 |
| MI-6PEG-GGFG-D4 | 8.79 | 19.60 | 62.45 | 38.86 |
| MI-6PEG-AANG-D5 | 1.28 | 1.55 | 2.31 | 2.51 |
| MI-6PEG-GGFG-D5 | 2.38 | 2.97 | 7.54 | 5.16 |
| MI-6PEG-AANG-D6 | 0.77 | 0.49 | 1.81 | 1.55 |
| MI-6PEG-GGFG-D6 | 1.19 | 0.73 | 2.30 | 1.31 |
| MI-6PEG-AANG-D7 | 1.21 | 0.53 | 2.64 | 1.89 |
| MI-6PEG-GGFG-D7 | 1.68 | 1.28 | 2.40 | 1.32 |
| MI-6PEG-AANG-D8 | 1.21 | 0.87 | 2.55 | 2.51 |
| MI-6PEG-GGFG-D8 | 3.77 | 2.35 | 11.26 | 4.93 |

As shown in Table 1, the IC50s of all the polypeptide conjugates in the above cells were generally more than 100-fold higher than the IC50s of Belotecan and derivative drugs thereof, and the IC50s of some of the polypeptide conjugates were even more than 1,000-fold orders of magnitude higher than the IC50s of the Belotecan drugs. It can be seen that the toxicity of the polypeptide conjugates is significantly lower than that of Belotecan, and thus the polypeptide conjugates can significantly reduce the side effect reactions from the use of the drugs for cancer treatment in patients.

### [Example 25] Efficacy study of polypeptide conjugate in treatment of an animal model of sarcoma SJSA-1

Test objective: To investigate the antitumor efficacy of the above compounds in an animal tumor model of sarcoma SJSA-1.

Test drug: Polypeptide conjugate, Irinotecan, Belotecan and normal saline control group.

Test animals: 6-8 weeks old BALB/c mice, all female.

### Tumor model preparation:

Experimental objective: To investigate the antitumor efficacy of the above polypeptide conjugates, Irinotecan and Belotecan in a tumor model at doses of 15 mg/kg and 45 mg/kg (a MTD of a polypeptide conjugate is around 200-400 mg/kg, 45 mg/kg is about one-fifth to one-eighth of the MTD, the MTD of Irinotecan is around 70 mg/kg, and 15 mg/kg is one-fifth of the MTD). SJSA-1 cells were purchased from ATCC and cultured in a DMEM medium containing 10% fetal bovine serum at 37°C in 5% CO₂. Passaging was performed every three days, and cells within 15 generations were used. 2 × 10⁷ corresponding cells were injected into right chests of nude mice subcutaneously. After a volume of the tumors reached at least 100 mm³, the mice were grouped randomly, with three mice in each group. Then treatment was started, and the day when the treatment was started was the first day. An experimental group of Belotecan positive control was administered with Belotecan at a dose of 5 mg/kg, and Irinotecan positive control was administered with Irinotecan at a dose of 15 mg/kg. The control group was administered with normal saline. Administration was performed once a week for three weeks.

Control and dosage instructions: with 15 mg/kg polypeptide conjugate and Belotecan 5 mg/kg control, a concentration of Belotecan contained in 15 mg/kg polypeptide conjugate was 5 mg/kg in equal amounts of substances.

45 mg/kg: a MTD of a polypeptide conjugate was around 200-400 mg/kg, and 45 mg/kg was about one-fifth to one-eighth of the MTD; a MTD of Irinotecan was around 70 mg/kg, and 15 mg/kg was one-fifth of the MTD.

**Table 2**

| Compound name | SJSA-1 (sarcoma tissue) mean tumor volume (mm³) | SJSA-1 (sarcoma) | Mouse weight (%) |
|---|---|---|---|
| | | Growth rate (%) | |
| Normal saline negative control group | 1925.1 | 1665.76 | +35 |
| Belotecan (5 mg/kg) | 1253.2 | 1084.38 | -9 |
| D1 (5 mg/kg) | 1432 | 1235.57 | -6 |
| D2 (5 mg/kg) | 1176.5 | 1018.01 | -8 |
| D3 (5 mg/kg) | 1378.4 | 1192.71 | -5 |
| D4 (5 mg/kg) | 1269.4 | 1098.39 | -7 |
| D5 (5 mg/kg) | 1443.2 | 1248.78 | -5 |
| D6 (5 mg/kg) | 961.3 | 828.71 | +4 |
| D7 (5 mg/kg) | 1369.5 | 1185.01 | -7 |
| D8 (5 mg/kg) | 1497.5 | 1295.77 | +12 |
| Irinotecan (15 mg/kg) | 922.3 | 798.05 | +17 |
| MI-6PEG-AAN-PAB-Belotecan (15 mg/kg) | 1357 | 1174.19 | +6 |
| MI-6PEG-AANG-Belotecan (15 mg/kg) | 967 | 833.61 | +19 |
| MI-6PEG-GGFG-Belotecan (15 mg/kg) | 1453.1 | 1257.35 | +29 |
| MI-6PEG-AAKG-D2 (15 mg/kg) | 1215.6 | 980.32 | +27 |
| MI-6PEG-AACG-D2 (15 mg/kg) | 1034.2 | 854.71 | +11 |
| MI-6PEG-GGGP-D2 (15 mg/kg) | 1521.8 | 1227.26 | +37 |
| MI-6PEG-AAGP-D2 (15 mg/kg) | 1475.6 | 1209.51 | +34 |
| MI-6PEG-AANG-D3 (15 mg/kg) | 1325.4 | 1095.4 | +29 |
| MI-6PEG-GGFG-D3 (15 mg/kg) | 1418.7 | 1153.41 | +34 |
| MI-6PEG-AANG-D4 (15 mg/kg) | 1305.6 | 1081.87 | +27 |
| MI-6PEG-GGFG-D4 (15 mg/kg) | 1402.6 | 1149.67 | +37 |
| MI-6PEG-AANG-D5 (15 mg/kg) | 1195.4 | 982.82 | +27 |
| MI-6PEG-GGFG-D5 (15 mg/kg) | 1265.9 | 1014.26 | +26 |
| MI-6PEG-AANG-D6 (15 mg/kg) | 781.1 | 673.28 | +24 |
| MI-6PEG-GGFG-D6 (15 mg/kg) | 946.5 | 815.95 | +28 |
| MI-6PEG-AANG-D7 (15 mg/kg) | 915.3 | 751.85 | +31 |
| MI-6PEG-GGFG-D7 (15 mg/kg) | 1110.3 | 956.90 | +24 |
| MI-6PEG-AANG-D8 (15 mg/kg) | 1210.4 | 1043.10 | +35 |
| MI-6PEG-GGFG-D8 (15 mg/kg) | 1387.1 | 1195.78 | +19 |
| MI-6PEG-AAN-PAB-Belotecan (45 mg/kg) | 873.5 | 753.02 | -5 |
| MI-6PEG-AANG-Belotecan (45 mg/kg) | 652.1 | 562.07 | +17 |
| MI-6PEG-GGFG-Belotecan (45 mg/kg) | 979.5 | 844.40 | +31 |
| MI-6PEG-AANG-D2 (45 mg/kg) | 927.6 | 799.66 | +18 |
| MI-6PEG-GGFG-D2 (45 mg/kg) | 1161.5 | 1001.29 | +18 |
| MI-6PEG-GANG-D2 (45 mg/kg) | 1065.2 | 918.28 | +18 |
| MI-6PEG-TANG-D2 (45 mg/kg) | 1278.9 | 1102.50 | +25 |
| MI-6PEG-SANG-D2 (45 mg/kg) | 1331.5 | 1147.84 | +27 |
| MI-6PEG-AAKG-D2 (45 mg/kg) | 945.6 | 791.30 | +31 |
| MI-6PEG-AACG-D2 (45 mg/kg) | 1020.8 | 856.14 | +17 |
| MI-6PEG-GGGP-D2 (45 mg/kg) | 1219.6 | 1024.87 | +18 |
| MI-6PEG-AAGP-D2 (45 mg/kg) | 1108.5 | 931.51 | +24 |
| MI-6PEG-AANG-D3 (45 mg/kg) | 1098.2 | 930.68 | +26 |
| MI-6PEG-GGFG-D3 (45 mg/kg) | 1171.6 | 976.44 | +28 |
| MI-6PEG-AANG-D4 (45 mg/kg) | 994.5 | 842.80 | +26 |
| MI-6PEG-GGFG-D4 (45 mg/kg) | 1025.3 | 868.89 | +26 |
| MI-6PEG-AANG-D5 (45 mg/kg) | 893.6 | 770.34 | +27 |
| MI-6PEG-GGFG-D5 (45 mg/kg) | 1018.4 | 855.80 | +35 |
| MI-6PEG-AANG-D6 (45 mg/kg) | 535 | 461.21 | +3 |
| MI-6PEG-GGFG-D6 (45 mg/kg) | 615.2 | 530.34 | +2 |
| MI-6PEG-AANG-D7 (45 mg/kg) | 878.2 | 757.07 | +26 |
| MI-6PEG-GGFG-D7 (45 mg/kg) | 999.6 | 861.72 | +27 |

It can be seen from the data that the therapeutic effect of the polypeptide conjugate of the present disclosure far outweighs that of Irinotecan, and the polypeptide conjugate of the present disclosure has higher inhibitory efficiency and safety than those of Belotecan.

### [Example 26] Efficacy study of polypeptide conjugate in treatment of an animal model of colo320 colorectal cancer

Test objective: To investigate the antitumor efficacy of the above compounds in an animal tumor model of colo320 colorectal cancer.

Test drug: Polypeptide conjugate, Irinotecan, Belotecan and normal saline control group.

Test animals: 6-8 weeks old BALB/c mice, all female.

### Tumor model preparation:

Experimental objective: To investigate the antitumor efficacy of the above polypeptide conjugates, Irinotecan and Belotecan in tumor models at doses of 15 mg/kg and 45 mg/kg. The colo320 cells were purchased from AoRuiCell Biotechnology (Shanghai) Co., Ltd. and cultured in a 1640 medium containing 10% fetal bovine serum at 37°C in 5% CO₂. Passaging was performed every three days, and cells within 15 generations were used. 7.5 × 10⁶ corresponding cells were injected into right chests of nude mice subcutaneously. After a volume of the tumors reached at least 120 mm³, the mice were grouped randomly, with three mice in each group. Then treatment was started, and the day when the treatment was started was the first day. An experimental group of Belotecan positive control was administered with Belotecan at a dose of 5 mg/kg, and Irinotecan positive control was administered with Irinotecan at a dose of 15 mg/kg. The control group was administered with normal saline. Administration was performed once a week for four weeks.

Control and dosage instructions: with 15 mg/kg polypeptide conjugate and Belotecan 5 mg/kg control, a concentration of Belotecan contained in 15 mg/kg polypeptide conjugate was 5 mg/kg in equal amounts of substances.

45 mg/kg: a MTD of a polypeptide conjugate was around 200-400 mg/kg, and 45 mg/kg was about one-fifth to one-eighth of the MTD; a MTD of Irinotecan was around 70 mg/kg, and 15 mg/kg was one-fifth of the MTD.

**Table 3**

| Compound name | colo320 (colorectal cancer cell) mean tumor volume (mm³) | colo320 (colorectal cancer cell) growth rate (%) | Mouse weight (%) |
|---|---|---|---|
| Normal saline negative control group | 1648.7 | 1488.58 | +41 |
| Belotecan (5 mg/kg) | 963.5 | 869.93 | -9 |
| D1 (5 mg/kg) | 1121.1 | 1012.22 | -7 |
| D2 (5 mg/kg) | 1047.9 | 946.13 | -7 |
| D3 (5 mg/kg) | 966 | 872.19 | +7 |
| D4 (5 mg/kg) | 1047.5 | 945.77 | +5 |
| D5 (5 mg/kg) | 1098.6 | 991.91 | +8 |
| D6 (5 mg/kg) | 787.3 | 715.73 | -4 |
| D7 (5 mg/kg) | 1258.9 | 1136.64 | -8 |
| D8 (5 mg/kg) | 1199.1 | 1082.65 | -6 |
| Irinotecan (15 mg/kg) | 903.1 | 815.39 | +35 |
| MI-6PEG-AAN-PAB-Bel otecan (15 mg/kg) | 1224.3 | 1105.40 | +29 |
| MI-6PEG-AANG-Belotec an (15 mg/kg) | 971.1 | 882.73 | +35 |
| MI-6PEG-GGFG-Belotec an (15 mg/kg) | 1171.5 | 1057.73 | +27 |
| MI-6PEG-TANG-D2 (15 mg/kg) | 1102.8 | 995.70 | +37 |
| MI-6PEG-SANG-D2 (15 mg/kg) | 1024.6 | 925.09 | +27 |
| MI-6PEG-FANG-D2 (15 mg/kg) | 956.5 | 863.61 | +31 |
| MI-6PEG-AAAG-D2 (15 mg/kg) | 1198.3 | 1081.93 | +30 |
| MI-6PEG-AAKG-D2 (15 mg/kg) | 1044.8 | 943.33 | +27 |
| MI-6PEG-GGGP-D2 (15 mg/kg) | 1481.2 | 1337.35 | +27 |
| MI-6PEG-AAGP-D2 (15 mg/kg) | 1077.5 | 972.86 | +24 |
| MI-6PEG-AANG-D3 (15 mg/kg) | 1091.1 | 985.14 | +31 |
| MI-6PEG-AANG-D4 (15 mg/kg) | 963.7 | 870.11 | +40 |
| MI-6PEG-GGFG-D4 (15 mg/kg) | 1078.5 | 973.76 | +37 |
| MI-6PEG-AANG-D5 (15 mg/kg) | 1152.1 | 984.70 | +31 |
| MI-6PEG-AANG-D6 (15 mg/kg) | 826.9 | 700.76 | +27 |
| MI-6PEG-GGFG-D6 (15 mg/kg) | 973.1 | 817.37 | +29 |
| MI-6PEG-AANG-D7 (15 mg/kg) | 957.9 | 818.72 | +26 |
| MI-6PEG-GGFG-D7 (15 mg/kg) | 1061.7 | 958.59 | +28 |
| MI-6PEG-AAN-PAB-Bel otecan (45 mg/kg) | 635.7 | 573.97 | +6 |
| MI-6PEG-AANG-Belotec an (45 mg/kg) | 366 | 330.46 | +29 |
| MI-6PEG-GGFG-Belotec an (45 mg/kg) | 581.5 | 525.03 | +34 |
| MI-6PEG-AANG-D1 (45 mg/kg) | 676.6 | 610.90 | +18 |
| MI-6PEG-GGFG-D1 (45 mg/kg) | 671.3 | 605.35 | +19 |
| MI-6PEG-AANG-D2 (45 mg/kg) | 588.5 | 531.35 | +11 |
| MI-6PEG-GGFG-D2 (45 mg/kg) | 568.5 | 513.29 | +41 |
| MI-6PEG-GANG-D2 (45 mg/kg) | 499.2 | 450.72 | +39 |
| MI-6PEG-TANG-D2 (45 mg/kg) | 712.4 | 598.66 | +34 |
| MI-6PEG-SANG-D2 (45 mg/kg) | 702.8 | 595.59 | +28 |
| MI-6PEG-FANG-D2 (45 mg/kg) | 561.1 | 506.61 | +24 |
| MI-6PEG-AAAG-D2 (45 mg/kg) | 491 | 443.32 | +25 |
| MI-6PEG-AAKG-D2 (45 mg/kg) | 521.2 | 470.58 | +24 |
| MI-6PEG-AACG-D2 (45 mg/kg) | 471 | 425.26 | +26 |
| MI-6PEG-GGGP-D2 (45 mg/kg) | 467 | 421.65 | +28 |
| MI-6PEG-AAGP-D2 (45 mg/kg) | 563.2 | 508.50 | +35 |
| MI-6PEG-AANG-D3 (45 mg/kg) | 532.8 | 481.06 | +20 |
| MI-6PEG-GGFG-D3 (45 mg/kg) | 577.5 | 521.42 | +14 |
| MI-6PEG-AANG-D4 (45 mg/kg) | 603.4 | 544.80 | +18 |
| MI-6PEG-GGFG-D4 (45 mg/kg) | 594.1 | 536.41 | +9 |
| MI-6PEG-AANG-D5 (45 mg/kg) | 755.5 | 682.13 | +31 |
| MI-6PEG-GGFG-D5 (45 mg/kg) | 829.4 | 748.86 | +19 |
| MI-6PEG-AANG-D6 (45 mg/kg) | 283.4 | 255.88 | +17 |
| MI-6PEG-GGFG-D6 (45 mg/kg) | 472.1 | 426.25 | +19 |
| MI-6PEG-AANG-D7 (45 mg/kg) | 463.7 | 418.66 | +31 |
| MI-6PEG-GGFG-D7 (45 mg/kg) | 539.5 | 487.11 | +20 |
| MI-6PEG-AANG-D8 (45 mg/kg) | 640.9 | 578.65 | +21 |
| MI-6PEG-GGFG-D8 (45 mg/kg) | 650 | 586.88 | +17 |

It can be seen from the data in the table above that the polypeptide conjugate of the present disclosure has higher inhibitory efficiency and safety than those of Belotecan.

### [Example 27] MTD determination of polypeptide conjugate, Belotecan, Irinotecan and other compounds

Test animals: 6-8 weeks old mice, all female; mice were grouped randomly, with six mice in each group, administered with drugs at different concentration gradients separately and monitored for 14 d. A mouse was euthanized at a loss of 20% of its initial weight and deemed to have died of poisoning. A MTD was defined as the highest dose level, in which none of the six mice died of the drugs, no individual mouse lost more than 20% of its weight, or a mean weight loss in a group did not exceed 15%.

**Table 4**

| Compound name | MTD |
|---|---|
| Belotecan | 15 mg/kg |
| D1 | 25 mg/kg |
| D2 | 25 mg/kg |
| D3 | 25 mg/kg |
| D4 | 30 mg/kg |
| D5 | 30 mg/kg |
| D6 | 10 mg/kg |
| D7 | 15 mg/kg |
| D8 | 25 mg/kg |
| Irinotecan | 75 mg/kg |
| MI-6PEG-AAN-PAB-Belotecan | 100 mg/kg |
| MI-6PEG-AANG-Belotecan | 300 mg/kg |
| MI-6PEG-GGFG-Belotecan | 180 mg/kg |
| MI-6PEG-AANG-D1 | 420 mg/kg |
| MI-6PEG-GGFG-D1 | 250 mg/kg |
| MI-6PEG-AANG-D2 | 400 mg/kg |
| MI-6PEG-GGFG-D2 | 250 mg/kg |
| MI-6PEG-GANG-D2 | 200 mg/kg |
| MI-6PEG-SANG-D2 | 200 mg/kg |
| MI-6PEG-FANG-D2 | 250 mg/kg |
| MI-6PEG-AAAG-D2 | 300 mg/kg |
| MI-6PEG-AAKG-D2 | 350 mg/kg |
| MI-6PEG-AACG-D2 | 400 mg/kg |
| MI-6PEG-GGGP-D2 | 250 mg/kg |
| MI-6PEG-AAGP-D2 | 200 mg/kg |
| MI-6PEG-AANG-D3 | 350 mg/kg |
| MI-6PEG-GGFG-D3 | 250 mg/kg |
| MI-6PEG-AANG-D4 | 350 mg/kg |
| MI-6PEG-GGFG-D4 | 250 mg/kg |
| MI-6PEG-AANG-D5 | 350 mg/kg |
| MI-6PEG-GGFG-D5 | 250 mg/kg |
| MI-6PEG-AANG-D6 | 200 mg/kg |
| MI-6PEG-AANG-D7 | 250 mg/kg |
| MI-6PEG-AANG-D8 | 400 mg/kg |
| MI-6PEG-GGFG-D8 | 250 mg/kg |

It can be seen from the data in the table above that the MTD of the polypeptide conjugate of the present disclosure rises by tens of times compared with Belotecan alone, and is significantly higher than that of Belotecan alone and safer.

### [Example 28] Polypeptide conjugate tumor homogenate activation efficiency experiment

Buffer preparation: 50 mM PH buffer (MES), 250 mM NaCl, pH adjusted to 5.0 with 0.5 M of sodium hydroxide.

The polypeptide conjugate was prepared into a 1 mg/ml solution with pH 5.0 buffer, 100 µg of tumor tissue homogenate was added, (the tumor tissue homogenate was the homogenate of tumor tissues prepared with a Jingxin F6/10 handheld homogenizer), and the solution was placed at 37°C for 2 h. The tumor tissue homogenate could effectively activate the polypeptide conjugate and release Belotecan, and the activation efficiencies of the drugs in tumor tissues were compared by high-performance liquid chromatography (HPLC) allowing determination of a decrease in the compounds and an increase in Belotecan: 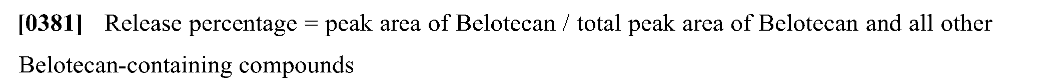
Release percentage = peak area of Belotecan / total peak area of Belotecan and all other Belotecan-containing compounds

**Table 5 Percentage of release efficiency (%) of polypeptide conjugate in different tissues**

| Tissue name | Ovarian cancer tissue | Sarcoma tissue | Breast cancer tissue | Non-small cell lung cancer tissue | Small cell lung cancer tissue | Heart tissue |
|---|---|---|---|---|---|---|
| Tumor-producing cell | A2780 | SJSA 1 | SKBR3 | A549 | NCI-H841 | |
| MI-6PEG-AAN-PA B-Belotecan | 75.3 | 78.5 | 69.4 | 79.9 | 61.5 | 2.7 |
| MI-6PEG-AANG-B elotecan | 69.3 | 72.1 | 80.5 | 73.7 | 62.7 | 3.5 |
| MI-6PEG-GGFG-B elotecan | 81.5 | 83.4 | 67.7 | 72.7 | 77.3 | 1.9 |
| MI-6PEG-AANG-D 1 | 72.4 | 82.1 | 77.8 | 69.2 | 73 | 2 |
| MI-6PEG-GGFG-D 1 | 67.2 | 81.5 | 74.8 | 71.7 | 66.3 | 1.7 |
| MI-6PEG-AANG-D 2 | 85.6 | 85.9 | 80.9 | 64.3 | 86.3 | 2.6 |
| MI-6PEG-GGFG-D 2 | 89.3 | 72.9 | 70.3 | 65.6 | 70.1 | 1 |
| MI-6PEG-GANG-D 2 | 69.9 | 88.4 | 68.8 | 65.9 | 63.1 | 1.7 |
| MI-6PEG-TANG-D 2 | 74.8 | 81.1 | 62.4 | 79.7 | 72.3 | 0.9 |
| MI-6PEG-SANG-D 2 | 85.6 | 76.6 | 69.5 | 69.8 | 68 | 1 |
| MI-6PEG-FANG-D 2 | 75.9 | 81.8 | 68 | 80.5 | 80 | 0.8 |
| MI-6PEG-AAAG-D 2 | 68.3 | 68.8 | 87.6 | 61.6 | 62.8 | 1.8 |
| MI-6PEG-AAKG-D 2 | 85.8 | 76.6 | 69.5 | 69.8 | 68 | 1.5 |
| MI-6PEG-AACG-D 2 | 75.9 | 63.9 | 81.8 | 68 | 80.5 | 1.5 |
| MI-6PEG-GGGP-D 2 | 68.3 | 68.8 | 87.6 | 61.6 | 62.8 | 1.3 |
| MI-6PEG-AAGP-D 2 | 76.2 | 88.9 | 84.6 | 61.6 | 62.8 | 2.4 |
| MI-6PEG-AANG-D 3 | 76.2 | 88.9 | 84.6 | 82.6 | 71.2 | 3.6 |
| MI-6PEG-GGFG-D 3 | 69.9 | 83.7 | 61.1 | 67.6 | 60.6 | 3.2 |
| MI-6PEG-AANG-D 4 | 86.7 | 72.4 | 80.7 | 78.7 | 67.2 | 2.4 |
| MI-6PEG-GGFG-D 4 | 73 | 66.8 | 62.7 | 63.9 | 74.5 | 1.4 |
| MI-6PEG-AANG-D 5 | 79.4 | 73.1 | 80.7 | 66.7 | 75.5 | 1.6 |
| MI-6PEG-GGFG-D 5 | 72.3 | 66.6 | 75.4 | 69 | 66.7 | 1.3 |
| MI-6PEG-AANG-D 6 | 75.9 | 79.9 | 82.1 | 73.1 | 76 | 2.5 |
| MI-6PEG-GGFG-D 6 | 66.3 | 80.7 | 67.1 | 83.7 | 72.1 | 2.4 |
| MI-6PEG-AANG-D 7 | 84.5 | 64.3 | 78.8 | 77 | 66.1 | 2 |
| MI-6PEG-GGFG-D 7 | 73.6 | 69.7 | 72.7 | 75.2 | 76.4 | 2.9 |
| MI-6PEG-AANG-D 8 | 70.1 | 71.1 | 70.9 | 85.1 | 80.8 | 3 |
| MI-6PEG-GGFG-D 8 | 76 | 72.7 | 81.2 | 77.5 | 66.7 | 3.1 |

It can be seen from the contents of the above examples in conjunction with results of Example 28 that: the MTD of the polypeptide conjugate has risen by tens of times compared with that of Belotecan alone, and the polypeptide conjugate is released well in the tumor tissues but not released in normal tissues, so that targeted release is achieved. Compared with the current commonly used first-line chemotherapy drug Irinotecan, the tumor-suppressing effectiveness of the polypeptide conjugate at one-tenth of the MTD is much better than that of Irinotecan at one-fifth of the MTD, and the polypeptide conjugate demonstrates superior therapeutic effects in many cancers such as colorectal, sarcoma, lung and ovarian cancers, improves the safety window effectively, and servers as a better choice for patients with various types of solid tumors in the thoracic and abdominal regions.

### [Example 29] Synthesis of MI-6PEG-AANL-Belotecan

A synthesis method for a polypeptide conjugate MI-6PEG-AANL-Belotecan is shown as follows:

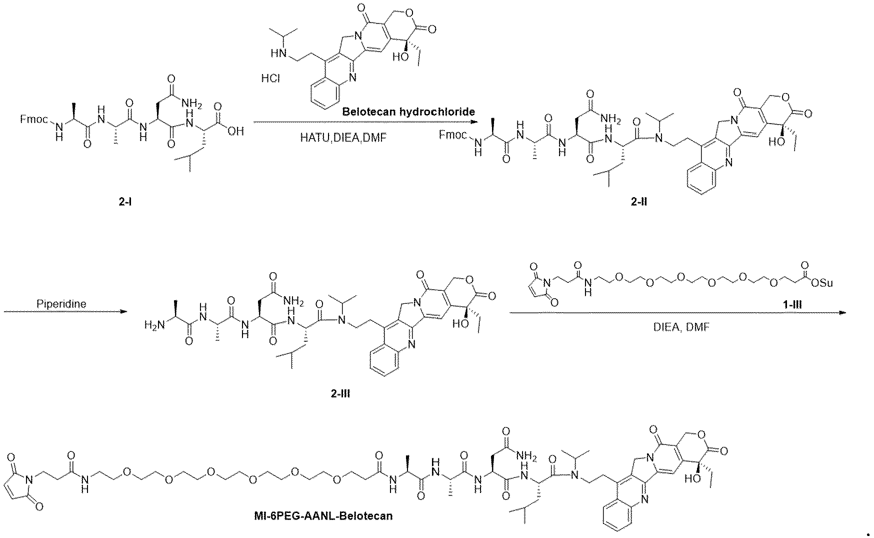

Specific synthesis steps are as follows:

### 1. Synthesis of a compound 2-II

A compound Belotecan hydrochloride (705 mg, 1.50 mmol) and a compound 2-I (920 mg, 1.51 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 2-II (890 mg, a yield of 57.9%).

### 2. Synthesis of a compound 2-III

The compound 2-II (890 mg, 0.87 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM: MeOH = 100:1 to 5:1) to obtain a yellow solid compound 2-III (630 mg, a yield of 90.2%).

### 3. Synthesis of a compound MI-6PEG-AANL-Belotecan

The compound 2-III (630 mg, 0.78 mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain a yellow solid compound MI-6PEG-AANL-Belotecan (185 mg, a yield of 18.4%).

### [Example 30] Synthesis of MI-6PEG-GGFL-Belotecan

A synthesis method for a polypeptide conjugate MI-6PEG-GGFL-Belotecan is shown as follows:

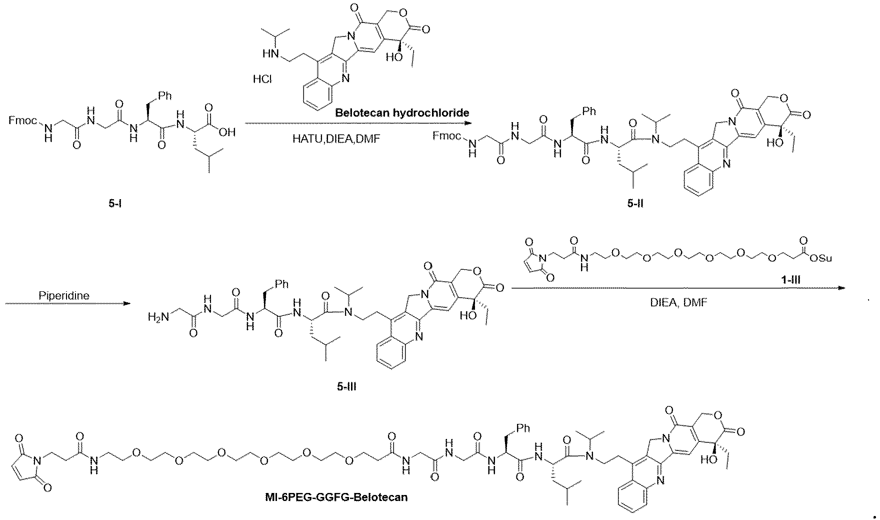

Specific synthesis steps are as follows:

### 1. Synthesis of a compound 5-II

A compound Belotecan hydrochloride (705 mg, 1.50 mmol) and a compound 5-I (935 mg, 1.52 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 5-II (720 mg, a yield of 46.6%).

### 2. Synthesis of a compound 5-III

The compound 5-II (720 mg, 0.70 mmol) was dissolved in N, N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 5-III (465 mg, a yield of 82.2%).

### 3. Synthesis of a compound MI-6PEG-GGFL-Belotecan

The compound 5-III (465 mg, 0.58 mmol) was dissolved in N, N-dimethylformamide (15 mL), and a compound 1-III (520 mg, 0.86 mmol) and diisopropylethylamine (350 mg, 2.71 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFL-Belotecan (265 mg, a yield of 35.3%).

### [Example 31] Synthesis of MI-6PEG-AANL-D1

A synthesis method for a polypeptide conjugate MI-6PEG-AANL-D1 is shown as follows:

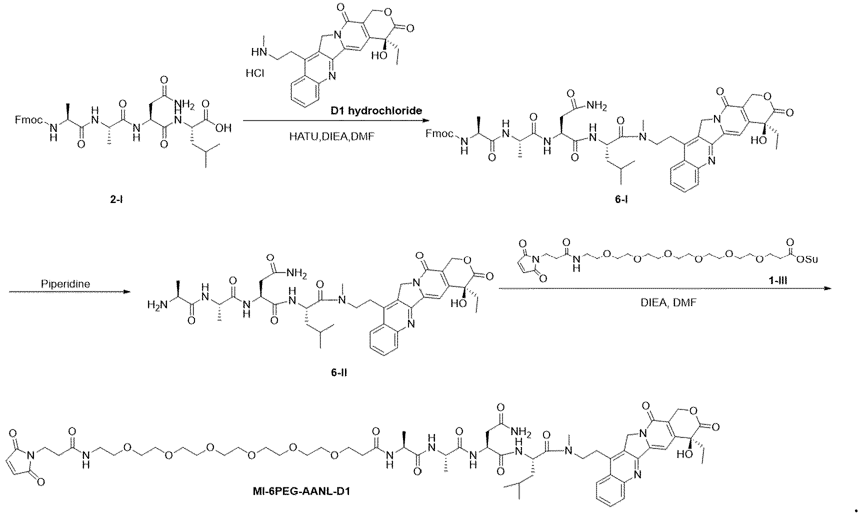

Specific synthesis steps are as follows:

### 1. Synthesis of a compound 6-I

A compound D1 hydrochloride (663 mg, 1.50 mmol) and a compound 2-I (920 mg, 1.51 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 6-I (950 mg, a yield of 63.5%).

### 2. Synthesis of a compound 6-II

The compound 6-I (950 mg, 0.95 mmol) was dissolved in N, N-dimethylformamide (20 mL), and piperidine (1.5 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM: MeOH = 100:1 to 5:1) to obtain a yellow solid compound 6-II (580 mg, a yield of 78.8%).

### 3. Synthesis of a compound MI-6PEG-AANL-D1

The compound 6-II (580 mg, 0.75mmol) was dissolved in N, N-dimethylformamide (15 mL), and a compound 1-III (500 mg, 0.83 mmol) and diisopropylethylamine (250 mg, 1.94 mmol) were added thereto. A reaction solution reacted at room temperature of 25°C for 3 h. The reaction solution was filtered, and the filtered solution was purified through a reversed-phase column to obtain an off-white solid compound MI-6PEG-AANL-D1 (153 mg, a yield of 16.2%).

### [Example 32] Synthesis of MI-6PEG-GGFL-D1

A synthesis method for a polypeptide conjugate MI-6PEG-GGFL-D1 is shown as follows:

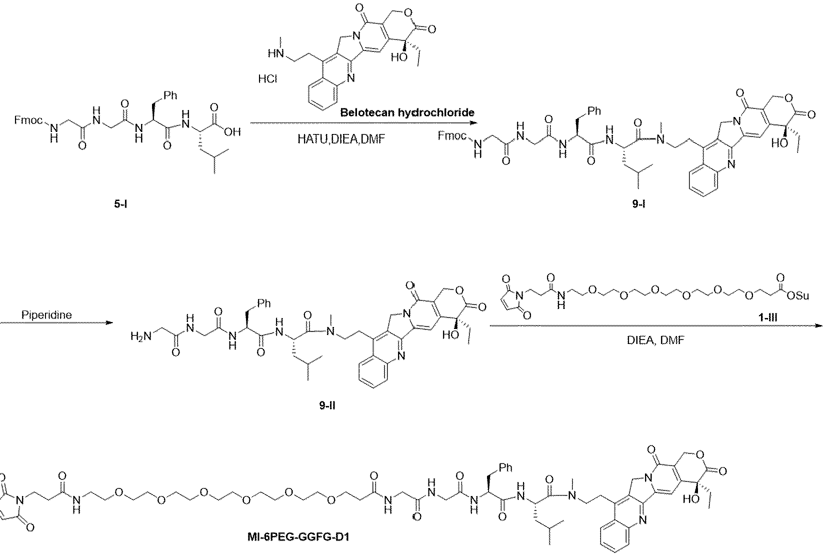

Specific synthesis steps are as follows:

### 1. Synthesis of a compound 9-I

A compound D1 hydrochloride (663 mg, 1.50 mmol) and a compound 5-I (935 mg, 1.52 mmol) were dissolved in dichloromethane (30 mL), and HATU (800 mg, 2.11 mmol) and diisopropylethylamine (550 mg, 4.26 mmol) were added thereto under cooling in an ice bath. A reaction solution reacted at room temperature of 25°C for 2 h. A TLC was adopted to detect and ensure that the reaction was complete. Water (80 mL) was added to the reaction solution, liquid was separated, an aqueous phase was extracted with dichloromethane (50 mL*2), and organic phases were merged, dried with anhydrous sodium sulfate, and evaporated to dryness under a reduced pressure. Residues therein were purified through a silica gel column (DCM:MeOH = 100:1 to 8:1) to obtain a yellow solid compound 9-I (742 mg, a yield of 49.4%).

### 2. Synthesis of a compound 9-II

The compound 9-I (742 mg, 0.74 mmol) was dissolved in N, N-dimethylformamide (20 mL), and piperidine (1 mL) was added thereto. A reaction solution reacted at room temperature of 25°C for 1 h. A TLC was adopted to detect and ensure that the reaction was complete. The reaction solution was then evaporated to dryness under a reduced pressure. Residues therein were purified through a silica gel column (DCM:MeOH = 100:1 to 5:1) to obtain a yellow solid compound 9-II (454 mg, a yield of 78.7%).

### 3. Synthesis of a compound MI-6PEG-GGFL-D1

The compound 9-II (454 mg, 0.58 mmol) was dissolved in N, N-dimethylformamide (15 mL), and a compound 1-III (520 mg, 0.86 mmol) and diisopropylethylamine (350 mg, 2.71 mmol) were added thereto. A reaction solution reacted at room temperature of 25°C for 3 h. A TLC was adopted to detect and ensure that the reaction was complete. The reaction solution was then evaporated to dryness under a reduced pressure. Residues therein were purified through a reversed-phase column to obtain an off-white solid compound MI-6PEG-GGFL-D1 (159 mg, a yield of 21.6%).

### [Example 33] Comparison of toxicity experiments of polypeptide conjugate in sarcoma and ovarian cancer cell lines (CCK8)

Different types of cells were cultured with complete media (RPMI1640 (DMEM high sugar medium) + 10% fetal bovine serum + 1XP/S + sodium pyruvate 1 mM solution) in an incubator at 37°C and 5% CO₂ until the cells grew to a sufficient number, and the cells were collected, centrifuged at 1,000 g for 5 min, resuspended with an appropriate volume of a 10% RMPI1640 (DMEM high sugar medium) medium and adjusted to an appropriate density. In a 96-well culture plate, 100 µL of cell culture fluid containing different concentrations of drugs was added, control wells (0.1% DMSO) with only corresponding drug solvents without drugs, as well as blank wells (Blank) with only the medium but without cells were set, and three parallel wells were set in each group. After that, the cells after cell counting were inoculated onto 96-well culture plates, with 100 µL of cell suspension per well, and an inoculation concentration of the cells was 5,000 cells (100 µL)/well. The plates were then cultured in a CO₂ (5%) incubator at 37°C for 48 h. After 48 h, 10 µL of a cell proliferation staining reagent (CCK8) was added to each well, the plates were incubated in a cell incubator for around 2 h, and an absorbance at 450 nm was detected.

Cell survival rates and median inhibitory concentrations (IC50) of the drugs on the cells were calculated.

Specific experimental results of the median inhibitory concentrations (IC50) of the drugs on the cells are shown in the following table (in ug/mL):

**Table 6 Toxicity of polypeptide conjugate, Belotecan, Irinotecan and the like in different cell lines**

| Tissue name | Ovarian cancer tissue (A2780) | Sarcoma tissue (SJSA-1) | Breast cancer tissue (SKBR3) | Non-small cell lung cancer tissue (A549) | Colorectal cancer tissue (colo320) |
|---|---|---|---|---|---|
| Belotecan | 0.0044 | 0.0021 | 0.0098 | 0.0066 | 0.014 |
| D1 | 0.018 | 0.028 | 0.050 | 0.027 | 0.040 |
| D2 | 0.018 | 0.023 | 0.036 | 0.030 | 0.051 |
| D3 | 0.021 | 0.029 | 0.049 | 0.026 | 0.040 |
| D4 | 0.029 | 0.054 | 0.076 | 0.043 | 0.078 |
| D5 | 0.014 | 0.012 | 0.013 | 0.019 | 0.029 |
| D6 | 0.0029 | 0.0018 | 0.0038 | 0.0025 | 0.0048 |
| D7 | 0.0043 | 0.0084 | 0.016 | 0.0074 | 0.017 |
| D8 | 0.015 | 0.035 | 0.047 | 0.016 | 0.042 |
| Irinotecan | 0.16 | 0.11 | 0.25 | 0.11 | 0.11 |
| MI-6PEG-AANL-Belotecan | 0.91 | 0.90 | 2.48 | 1.55 | 2.74 |
| MI-6PEG-GGFL-B elotecan | 1.91 | 1.24 | 3.69 | 2.21 | 2.86 |
| MI-6PEG-AANL-D1 | 2.09 | 2.33 | 3.25 | 3.05 | 3.82 |
| MI-6PEG-AANL-D2 | 1.31 | 1.58 | 3.28 | 2.57 | 4.65 |
| MI-6PEG-AANL-D3 | 1.15 | 1.52 | 2.56 | 1.87 | 3.28 |
| MI-6PEG-AANL-D4 | 2.47 | 2.94 | 3.94 | 3.72 | 5.40 |
| MI-6PEG-AANL-D5 | 1.13 | 1.58 | 2.20 | 1.34 | 2.42 |
| MI-6PEG-AANL-D6 | 0.70 | 0.51 | 2.02 | 1.38 | 1.87 |
| MI-6PEG-AANL-D7 | 0.91 | 1.33 | 2.01 | 1.81 | 2.48 |
| MI-6PEG-AANL-D8 | 1.27 | 1.54 | 1.98 | 2.27 | 2.97 |

As shown in Table 6, the IC50s of the polypeptide conjugates of the present disclosure in the above cells were generally more than 100-fold higher than the IC50s of Irinotecan and Belotecan, and the IC50s of some of the polypeptide conjugates were even more than 1,000-fold orders of magnitude higher than the IC50s of the Belotecan drugs. It can be seen that the toxicity of the polypeptide conjugates is significantly lower than that of the Belotecan compounds, and thus the polypeptide conjugates can significantly reduce the side effect reactions from the use of the drugs for cancer treatment in patients.

### [Example 34] Efficacy study of polypeptide conjugate in treatment of an animal model of sarcoma SJSA-1

Test objective: To investigate the antitumor efficacy of the above compounds in an animal tumor model of sarcoma SJSA-1.

Test drug: Polypeptide conjugate, Irinotecan, Belotecan and normal saline control group.

Test animals: 6-8 weeks old BALB/c mice, all female.

### Tumor model preparation:

Experimental objective: To investigate the antitumor efficacy of the above polypeptide conjugates, Irinotecan and Belotecan in tumor models at doses of 15 mg/kg and 45 mg/kg. SJSA-1 cells were purchased from ATCC and cultured in a DMEM medium containing 10% fetal bovine serum at 37°C in 5% CO₂. Passaging was performed every three days, and cells within 15 generations were used. 2 × 10⁷ corresponding cells were injected into right chests of nude mice subcutaneously. After a volume of the tumors reached at least 100 mm³, the mice were grouped randomly, with three mice in each group. Then treatment was started, and the day when the treatment was started was the first day. An experimental group of Belotecan positive control was administered with Belotecan at a dose of 5 mg/kg, and Irinotecan positive control was administered with Irinotecan at a dose of 15 mg/kg. The control group was administered with normal saline. Administration was performed once a week for three weeks.

Control and dosage instructions: with 15 mg/kg polypeptide conjugate and Belotecan 5 mg/kg control, a concentration of Belotecan contained in 15 mg/kg polypeptide conjugate was 5 mg/kg in equal amounts of substances.

45 mg/kg: a MTD of a polypeptide conjugate was around 150-200 mg/kg, and 45 mg/kg was about one-third to one-fifth of the MTD; a MTD of Irinotecan was around 70 mg/kg, and 15 mg/kg was one-fifth of the MTD.

**Table 7**

| Compound name | SJSA-1 (sarcoma tissue) mean tumor volume (mm³) | SJSA-1 (sarcoma tissue) growth rate (%) | Mouse weight (%) |
|---|---|---|---|
| Normal saline negative control group | 1925.1 | 1665.76 | +35 |
| Belotecan (5 mg/kg) | 1253.2 | 1084.38 | -9 |
| D1 (5 mg/kg) | 1432 | 1235.57 | -6 |
| D2 (5 mg/kg) | 1176.5 | 1018.01 | -8 |
| D3 (5 mg/kg) | 1378.4 | 1192.71 | -5 |
| D4 (5 mg/kg) | 1269.4 | 1098.39 | -7 |
| D5 (5 mg/kg) | 1443.2 | 1248.78 | -5 |
| D6 (5 mg/kg) | 961.3 | 831.80 | +4 |
| D7 (5 mg/kg) | 1369.5 | 1185.01 | -7 |
| D8 (5 mg/kg) | 1497.5 | 1295.77 | +12 |
| Irinotecan (15 mg/kg) | 922.3 | 798.05 | +17 |
| MI-6PEG-AANL-Belotec an (15 mg/kg) | 1354.9 | 1202.68 | +24 |
| MI-6PEG-GGFL-Belotec an (15 mg/kg) | 1278.6 | 1134.95 | +27 |
| MI-6PEG-AANL-D2 (15 mg/kg) | 1645.7 | 1316.56 | +41 |
| MI-6PEG-AANL-D3 (15 mg/kg) | 1521.5 | 1207.54 | +21 |
| MI-6PEG-AANL-D4 (15 mg/kg) | 1289.8 | 1031.84 | +19 |
| MI-6PEG-AANL-D5 (15 mg/kg) | 1304.2 | 1069.02 | +18 |
| MI-6PEG-AANL-D6 (15 mg/kg) | 1321.5 | 978.89 | +19 |
| MI-6PEG-AANL-D7 (15 mg/kg) | 1295.2 | 1036.16 | +12 |
| MI-6PEG-AANL-Belotec an (45 mg/kg) | 1023.9 | 839.26 | +21 |
| MI-6PEG-GGFL-Belotec an (45 mg/kg) | 1254.1 | 995.32 | +22 |
| MI-6PEG-AANL-D1 (45 mg/kg) | 1504.3 | 1203.44 | +29 |
| MI-6PEG-GGFL-D1 (45 mg/kg) | 1485.2 | 1181.64 | +11 |
| MI-6PEG-AANL-D2 (45 mg/kg) | 1213.3 | 964.08 | +24 |
| MI-6PEG-AANL-D3 (45 mg/kg) | 1205.8 | 941.15 | +16 |
| MI-6PEG-AANL-D4 (45 mg/kg) | 1149.5 | 924.41 | +19 |
| MI-6PEG-AANL-D5 (45 mg/kg) | 1165.2 | 933.13 | +17 |
| MI-6PEG-AANL-D6 (45 mg/kg) | 849.4 | 675.84 | +17 |
| MI-6PEG-AANL-D7 (45 mg/kg) | 989.1 | 788.44 | +19 |
| MI-6PEG-AANL-D8 (45 mg/kg) | 1235.3 | 975.05 | +17 |

It can be seen from the data that the therapeutic effect of the polypeptide conjugates of the present disclosure is comparable to that of Irinotecan and Belotecan, and the efficacy of some of the conjugates is superior to that of Irinotecan and Belotecan; also, compared with Irinotecan and Belotecan, the targeting property of the polypeptide conjugates of the present disclosure is significantly improved, the toxicity is significantly reduced, and therefore, the polypeptide conjugates of the present disclosure show favorable effects on the treatment of cancers from a comprehensive point of view.

### [Example 35] Efficacy study of polypeptide conjugate in treatment of an animal model of colo320 colorectal cancer

Test objective: To investigate the antitumor efficacy of the above compounds in an animal tumor model of colo320 colorectal cancer.

Test drug: Polypeptide conjugate, Irinotecan, Belotecan and normal saline control group.

Test animals: 6-8 weeks old BALB/c mice, all female.

### Tumor model preparation:

Experimental objective: To investigate the antitumor efficacy of the above polypeptide conjugates, Irinotecan and Belotecan in tumor models at doses of 15 mg/kg and 45 mg/kg. The colo320 cells were purchased from AoRuiCell Biotechnology (Shanghai) Co., Ltd. and cultured in a 1640 medium containing 10% fetal bovine serum at 37°C in 5% CO₂. Passaging was performed every three days, and cells within 15 generations were used. 7.5 × 10⁶ corresponding cells were injected into right chests of nude mice subcutaneously. After a volume of the tumors reached at least 120 mm³, the mice were grouped randomly, with three mice in each group. Then treatment was started, and the day when the treatment was started was the first day. An experimental group of Belotecan positive control was administered with Belotecan at a dose of 5 mg/kg, and Irinotecan positive control was administered with Irinotecan at a dose of 15 mg/kg. The control group was administered with normal saline. Administration was performed once a week for four weeks.

Control and dosage instructions: with 15 mg/kg polypeptide conjugate and Belotecan 5 mg/kg control, a concentration of Belotecan contained in 15 mg/kg polypeptide conjugate was 5 mg/kg in equal amounts of substances.

45 mg/kg: a MTD of a polypeptide conjugate was around 150-200 mg/kg, and 45 mg/kg was about one-third to one-fifth of the MTD; a MTD of Irinotecan was around 70 mg/kg, and 15 mg/kg was one-fifth of the MTD.

**Table 8**

| Compound name | colo320 (colorectal cancer cell) mean tumor volume (mm³) | colo320 (colorectal cancer cell) growth rate (%) | Mouse weight (%) |
|---|---|---|---|
| Normal saline negative control group | 1648.7 | 1488.58 | +41 |
| Belotecan (5 mg/kg) | 963.5 | 869.93 | -9 |
| D1 (5 mg/kg) | 1121.1 | 1012.22 | -7 |
| D2 (5 mg/kg) | 1047.9 | 946.13 | -7 |
| D3 (5 mg/kg) | 966 | 872.19 | +7 |
| D4 (5 mg/kg) | 1047.5 | 945.77 | +5 |
| D5 (5 mg/kg) | 1098.6 | 991.91 | +8 |
| D6 (5 mg/kg) | 787.3 | 710.84 | -4 |
| D7 (5 mg/kg) | 1258.9 | 1136.64 | -8 |
| D8 (5 mg/kg) | 1199.1 | 1082.65 | -6 |
| Irinotecan (15 mg/kg) | 903.1 | 815.39 | +35 |
| MI-6PEG-AANL-Belote can (15 mg/kg) | 1179.5 | 969.27 | +22 |
| MI-6PEG-GGFL-Belote can (15 mg/kg) | 1254.2 | 994.05 | +27 |
| MI-6PEG-AANL-D3 (15 mg/kg) | 1279.8 | 1088.75 | +18 |
| MI-6PEG-AANL-D4 (15 mg/kg) | 1271.1 | 1081.25 | +17 |
| MI-6PEG-AANL-D5 (15 mg/kg) | 1257.2 | 1027.54 | +19 |
| MI-6PEG-AANL-D6 (15 mg/kg) | 919.8 | 859.22 | +25 |
| MI-6PEG-AANL-D7 (15 mg/kg) | 1008.1 | 857.61 | +29 |
| MI-6PEG-AANL-D8 (15 mg/kg) | 1117.3 | 950.50 | +33 |
| MI-6PEG-AANL-Belote can (45 mg/kg) | 833.5 | 690.61 | +2 |
| MI-6PEG-GGFL-Belote can (45 mg/kg) | 789.4 | 671.56 | +5 |
| MI-6PEG-AANL-D1 (45 mg/kg) | 953.2 | 810.91 | +16 |
| MI-6PEG-GGFL-D1 (45 mg/kg) | 900.6 | 766.16 | +19 |
| MI-6PEG-AANL-D2 (45 mg/kg) | 894.4 | 760.87 | +19 |
| MI-6PEG-AANL-D3 (45 mg/kg) | 753.9 | 641.35 | +18 |
| MI-6PEG-AANL-D4 (45 mg/kg) | 978.4 | 832.33 | +18 |
| MI-6PEG-AANL-D5 (45 mg/kg) | 755.9 | 643.05 | +19 |
| MI-6PEG-AANL-D6 (45 mg/kg) | 468.4 | 398.47 | +5 |
| MI-6PEG-AANL-D7 (45 mg/kg) | 604.8 | 514.51 | +9 |
| MI-6PEG-AANL-D8 (45 mg/kg) | 800.9 | 681.33 | +15 |

It can be seen from the data that the therapeutic effect of the polypeptide conjugates of the present disclosure is comparable to that of Irinotecan and Belotecan, and the efficacy of some of the conjugates is superior to that of Irinotecan and Belotecan; also, compared with Irinotecan and Belotecan, the targeting property of the polypeptide conjugates of the present disclosure is significantly improved, the toxicity is significantly reduced, and therefore, they show favorable effects on the treatment of cancers from a comprehensive point of view.

### [Example 36] MTD determination of polypeptide conjugates

Test animals: 6-8 weeks old mice, all female; mice were grouped randomly, with six mice in each group, administered with drugs at different concentration gradients separately and monitored for 14 d. A mouse was euthanized at a loss of 20% of its initial weight and deemed to have died of poisoning. A MTD was defined as the highest dose level, in which none of the six mice died of the drugs, no individual mouse lost more than 20% of its weight, or a mean weight loss in a group did not exceed 15%.

Final statistical results of MTD of the polypeptide conjugates are shown in the table below:

**Table 9**

| Compound name | MTD |
|---|---|
| Belotecan | 15 mg/kg |
| D1 | 25 mg/kg |
| D2 | 25 mg/kg |
| D3 | 25 mg/kg |
| D4 | 30 mg/kg |
| D5 | 30 mg/kg |
| D6 | 10 mg/kg |
| D7 | 15 mg/kg |
| D8 | 25 mg/kg |
| Irinotecan | 75 mg/kg |
| MI-6PEG-AANL-Belotecan | 150 mg/kg |
| MI-6PEG-GGFL-Belotecan | 100 mg/kg |
| MI-6PEG-AANL-D1 | 200 mg/kg |
| MI-6PEG-GGFL-D1 | 120 mg/kg |
| MI-6PEG-AANL-D2 | 200 mg/kg |
| MI-6PEG-AANL-D3 | 200 mg/kg |
| MI-6PEG-AANL-D6 | 120 mg/kg |
| MI-6PEG-AANL-D7 | 150 mg/kg |
| MI-6PEG-AANL-D8 | 200 mg/kg |

It can be seen from the data in the table above that the MTD of the polypeptide conjugate of the present disclosure rises by tens of times compared with Belotecan alone, and is significantly higher than that of Belotecan alone and safer.

### [Example 37] Polypeptide conjugate tumor homogenate activation efficiency experiment

Buffer preparation: 50 mM PH buffer (MES), 250 mM NaCl, pH adjusted to 5.0 with 0.5 M of sodium hydroxide.

The polypeptide conjugate was prepared into a 1 mg/ml solution with pH 5.0 buffer, 100 µg of tumor tissue homogenate was added, (the tumor tissue homogenate was the homogenate of tumor tissues prepared with a Jingxin F6/10 handheld homogenizer), and the solution was placed at 37°C for 2 h. The tumor tissue homogenate could effectively activate the polypeptide conjugate and release Belotecan, and the activation efficiencies of the drugs in tumor tissues were compared by HPLC allowing determination of a decrease in the compounds and an increase in Belotecan: 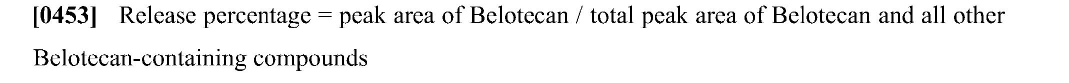
Release percentage = peak area of Belotecan / total peak area of Belotecan and all other Belotecan-containing compounds

Results are shown in the table below:

**Table 10 Percentage of release efficiency (%) of polypeptide conjugate in different tissues**

| Tissue name | Ovarian cancer tissue | Sarcoma tissue | Breast cancer tissue | Non-small cell lung cancer tissue | Colorectal cancer tissue | Heart tissue |
|---|---|---|---|---|---|---|
| Tumor-producing cell | A2780 | SJSA1 | SKBR3 | A549 | colo320 | |
| MI-6PEG-AANL-Belotecan | 81.2 | 75.5 | 69.5 | 76.5 | 75.6 | 2.5 |
| MI-6PEG-GGFL-Belotecan | 73.6 | 68 | 73.2 | 77.3 | 83.1 | 1.8 |
| MI-6PEG-AANL-D1 | 66.4 | 81.2 | 80 | 69.5 | 72.4 | 3 |
| MI-6PEG-GGFL-D1 | 80 | 73.5 | 69.5 | 82.5 | 86.7 | 1.4 |
| MI-6PEG-AANL-D2 | 71.3 | 67.1 | 73.5 | 73.6 | 65 | 0.9 |
| MI-6PEG-AANL-D3 | 69.5 | 90 | 67.9 | 66.8 | 71.6 | 3.9 |
| MI-6PEG-AANL-D4 | 66.7 | 81.2 | 75.3 | 77.7 | 76.5 | 4 |
| MI-6PEG-AANL-D5 | 74.3 | 77.5 | 70.2 | 85.5 | 69.8 | 5.5 |
| MI-6PEG-AANL-D6 | 76.2 | 69.5 | 66.9 | 66.1 | 73.5 | 3 |
| MI-6PEG-AANL-D7 | 66.7 | 71.7 | 72.3 | 74.6 | 76.4 | 2.2 |
| MI-6PEG-AANL-D8 | 71 | 81.1 | 70.2 | 68.4 | 78.9 | 2.2 |

It can be seen from the data in the table above that the polypeptide conjugate of the present disclosure is well released in homogenates of different cancer tissues, but only a very small amount of polypeptide conjugate can be activated in the heart.

### [Example 38] Enzyme cleaving activation experiment of Belotecan polypeptide conjugate

Buffer preparation: 50 mM MES, 250 mM NaCl, pH adjusted to 5.0 with 0.5 M sodium hydroxide. A concentration of 1 mg/mL was selected for Legumain and cathepsin B. The compounds were prepared to a concentration of 0.5 µmol/mL using buffer. 50 µL of 0.5 µmol/mL compound and 50 µL of buffer were accurately pipetted into a centrifuge tube, and 100 µL of Legumain or cathepsin B was added and reacted at 37°C for 2 h. A reaction solution was detected by LC-MS.

| Structure name | Enzymatically cleaved structure |
|---|---|
| MI-6PEG-AAN-PAB-Belotecan | Belotecan |
| MI-6PEG-AANG-Belotecan | G-Belotecan |
| MI-6PEG-GGFG-Belotecan | Belotecan |
| MI-6PEG-AANG-D1 | G-D1 |
| MI-6PEG-GGFG-D1 | D1 |
| MI-6PEG-AANG-D2 | G-D2 |
| MI-6PEG-GGFG-D2 | D2 |
| MI-6PEG-GANG-D2 | G-D2 |
| MI-6PEG-TANG-D2 | G-D2 |
| MI-6PEG-SANG-D2 | G-D2 |
| MI-6PEG-FANG-D2 | G-D2 |
| MI-6PEG-AANG-D3 | G-D3 |
| MI-6PEG-GGFG-D3 | D3 |
| MI-6PEG-AANG-D4 | G-D4 |
| MI-6PEG-GGFG-D4 | D4 |
| MI-6PEG-AANG-D5 | G-D5 |
| MI-6PEG-GGFG-D5 | D5 |
| MI-6PEG-AANG-D6 | G-D6 |
| MI-6PEG-GGFG-D6 | D6 |
| MI-6PEG-AANG-D7 | G-D7 |
| MI-6PEG-GGFG-D7 | D7 |
| MI-6PEG-AANG-D8 | G-D8 |
| MI-6PEG-GGFG-D8 | D8 |

Legumain is highly expressed in a tumor microenvironment. The Belotecan polypeptide conjugate can be cleaved by Legumain that can be highly expressed by tumor cells and tumor-associated macrophages to release Belotecan.

### [Example 39] Enzyme cleaving activation experiment of Belotecan polypeptide conjugate

Buffer preparation: 50 mM MES, 250 mM NaCl, pH adjusted to 5.0 with 0.5 M sodium hydroxide. A concentration of 1 mg/mL was selected for Legumain and cathepsin B. The compounds were prepared to a concentration of 0.5 µmol/mL using buffer. 50 µL of 0.5 µmol/mL compound and 50 µL of buffer were accurately pipetted into a centrifuge tube, and 100 µL of Legumain or cathepsin B was added and reacted at 37°C for 2 h. A reaction solution was detected by LC-MS.

| Structure name | Enzymatically cleaved structure |
|---|---|
| MI-6PEG-AANL-Belotecan | L-Belotecan |
| MI-6PEG-GGFL-Belotecan | Belotecan |
| MI-6PEG-AANL-D1 | L-D1 |
| MI-6PEG-GGFL-D1 | D1 |
| MI-6PEG-AANL-D2 | L-D2 |
| MI-6PEG-AANL-D3 | L-D3 |
| MI-6PEG-AANL-D4 | L-D4 |
| MI-6PEG-AANL-D5 | L-D5 |
| MI-6PEG-AANL-D6 | L-D6 |
| MI-6PEG-AANL-D7 | L-D7 |
| MI-6PEG-AANL-D8 | L-D8 |

Legumain is highly expressed in a tumor microenvironment. The Belotecan polypeptide conjugate can be cleaved by Legumain that can be highly expressed by tumor cells and tumor-associated macrophages to release Belotecan.

### [Example 40] Efficacy study of polypeptide conjugate in treatment of an animal model of sarcoma SJSA-1

Test objective: To further compare the antitumor efficacy of different polypeptide Linkers in an animal tumor model of sarcoma SJSA-1.

Test drug: Polypeptide conjugate and normal saline control group.

Test animals: 6-8 weeks old BALB/c mice, all female.

### Tumor model preparation:

Experimental objective: To investigate the antitumor efficacy of the above polypeptide conjugates in a tumor model at a dose of 30 mg/kg.

Experimental operation: SJSA-1 cells were purchased from ATCC and cultured in a DMEM medium containing 10% fetal bovine serum at 37°C in 5% CO₂. Passaging was performed every three days, and cells within 15 generations were used. 4 × 10⁶ corresponding cells were injected into right chests of nude mice subcutaneously. After a volume of the tumors reached at least 100 mm³, the mice were grouped randomly, with three mice in each group. Then treatment was started, and the day when the treatment was started was the first day. The control group was administered normal saline. Administration was performed once a week for two weeks.

Experimental results are as follows:

**Table 11**

| Compound name | SJSA-1 (sarcoma tissue) mean tumor volume (mm³) | | | | | SJSA-1 (sarcoma tissue) growth rate (%) on Day 14 | Mouse weight (%) on Day 14 |
|---|---|---|---|---|---|---|---|
| | Day 1 | Day 4 | Day 7 | Day 10 | Day 14 | | |
| Normal saline negative control group | 183.07 | 724.17 | 1147.2 0 | 1994.7 3 | 3066.2 | 1674.88 | +7.8 |
| MI-6PEG-AAN-P AB-Belotecan 30 mg/kg | 131.25 | 208.13 | 554.02 | 706.21 | 1319.7 | 1005.49 | -6.9 |
| MI-6PEG-AANG-Belotecan 30 mg/kg | 165.90 | 366.32 | 673.37 | 734.54 | 896.00 | 540.08 | +3.5 |
| MI-6PEG-AANL-Belotecan 30 mg/kg | 177.30 | 371.57 | 705.02 | 790.68 | 1295.67 | 730.78 | -1.3 |
| MI-6PEG-AANG-D2 30 mg/kg | 131.07 | 366.64 | 647.08 | 963.52 | 1574.31 | 1201.12 | +2.8 |
| MI-6PEG-AANL-D2 30 mg/kg | 136.59 | 465.17 | 891.36 | 1320.5 5 | 2218.14 | 1623.94 | +1.9 |
| MI-6PEG-AAGP-D2 30 mg/kg | 178.47 | 398.93 | 750.65 | 920.01 | 1873.27 | 1049.63 | -0.3 |
| MI-6PEG-AANG-D3 30 mg/kg | 132.12 | 360.28 | 610.04 | 833.76 | 1324.00 | 1002.12 | +3.6 |
| MI-6PEG-AANL-D3 30 mg/kg | 137.17 | 436.65 | 735.18 | 1111.34 | 1805.67 | 1316.37 | +4.2 |
| MI-6PEG-AANG-D4 30 mg/kg | 131.57 | 385.46 | 607.62 | 847.33 | 1239.14 | 941.81 | +4.1 |
| MI-6PEG-AANL-D4 30 mg/kg | 142.01 | 486.37 | 739.25 | 1068.2 1 | 1615.26 | 1137.43 | +2.3 |
| MI-6PEG-AANG-D5 30 mg/kg | 136.42 | 359.37 | 603.23 | 715.65 | 913.35 | 669.51 | +2.9 |
| MI-6PEG-AANL-D5 30 mg/kg | 139.89 | 429.56 | 658.17 | 876.45 | 1370.14 | 979.44 | -1.1 |
| MI-6PEG-AANG-D6 30 mg/kg | 179.32 | 245.10 | 415.17 | 487.62 | 626.67 | 349.47 | -0.6 |
| MI-6PEG-AANL-D6 30 mg/kg | 145.91 | 309.25 | 606.14 | 715.43 | 1142.25 | 782.85 | -2.5 |
| MI-6PEG-AANG-D7 30 mg/kg | 176.15 | 268.54 | 455.37 | 498.05 | 694.67 | 394.36 | +1.6 |
| MI-6PEG-AANL-D7 30 mg/kg | 147.33 | 313.65 | 644.39 | 795.08 | 1193.62 | 810.17 | -3.3 |

It can be seen from the data that neither MI-6PEG-AAGP-D2 nor MI-6PEG-AAN-PAB-Belotecan had a satisfactory tumor suppression effect, it is clear in the comparison of the tetrapeptide Linker AANG with AANL that AANG had a better tumor suppression effect than AANL, it can be seen from Examples 27 and 36 that the MTD in mice was increased by approximately 2-fold with AANG compared with AANL, and it is clear that AANG can provide a more significant molecular toxicity reduction effect than that provided by AANL. AANG is a polypeptide Linker having a better effect than those of AANL and AAN-PAB.

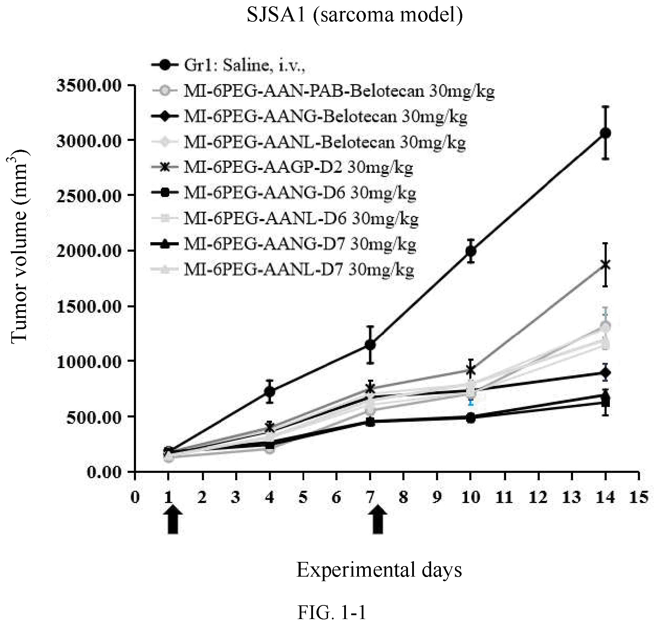

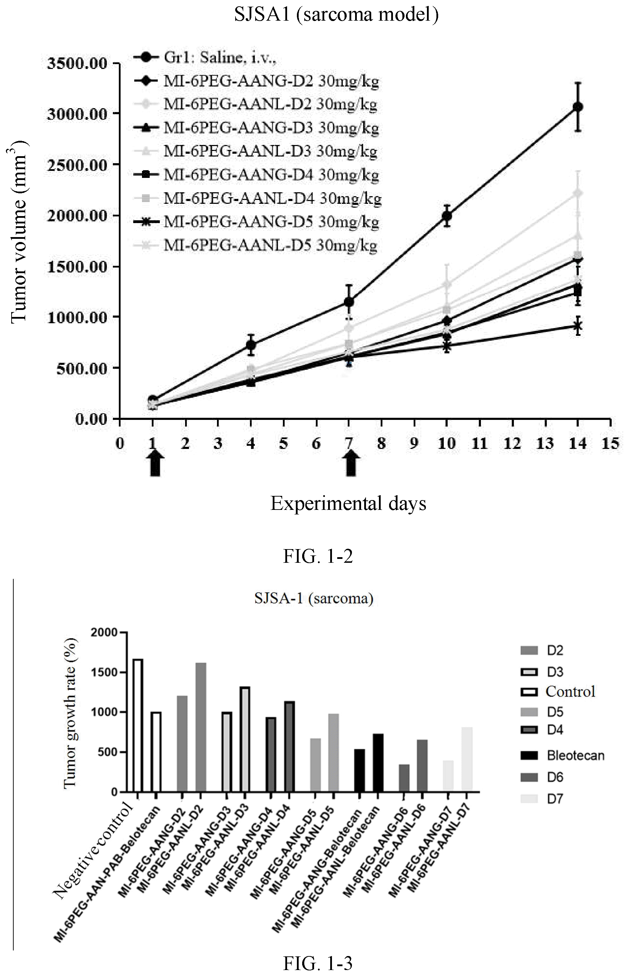

FIGs. 1-1, 1-2 and 1-3 of the specification show a schematic diagram produced based on the data in Table 11.

It can also be seen from FIGs. 1-1, 1-2 and 1-3 that a comparison of tumor volume growth curves with AANG, AANL and AAN-PAB as tetrapeptide Linkers shows that the tumor volume growth curve of AANG was significantly slower and the tumor volume growth curves of AANL and AAN-PAB were significantly steeper, and this indicates that the polypeptide conjugate with AANG as the Linker was released in tumor cells at a higher rate than that of the polypeptide conjugate with AANL and AAN-PAB as the Linkers and can produce a better efficacy.

### [Example 41] Efficacy study of polypeptide conjugate in treatment of an animal model of A2780 ovarian cancer

Test objective: To further compare the antitumor efficacy of different polypeptide Linkers in an animal tumor model of A2780 ovarian cancer.

Test drug: Polypeptide conjugate and normal saline control group.

Test animals: 6-8 weeks old BALB/c mice, all female.

Experimental objective: To investigate the antitumor efficacy of the above polypeptide conjugates in a tumor model at a dose of 30 mg/kg.

Experimental operation: A2780 cells were purchased from ATCC and cultured in a DMEM (1640) medium containing 10% fetal bovine serum at 37°C in 5% CO₂. Passaging was performed every three days, and cells within 15 generations were used. 8 × 10⁶ corresponding cells were injected into right axillas of nude mice subcutaneously. After a volume of the tumors reached at least 100 mm³, the mice were grouped randomly, with three mice in each group. Then treatment was started, and the day when the treatment was started was the first day. The negative control group was administered normal saline. Administration was performed once a week for two weeks.

Experimental results are as follows:

**Table 12**

| Compound name | A2780 (ovarian cancer) mean tumor volume (mm³) | | | | | Growth rate (%) of A2780 (ovarian cancer) on Day 14 | Mouse weight (%) on Day 14 |
|---|---|---|---|---|---|---|---|
| | Day 1 | Day 4 | Day 7 | Day 10 | Day 14 | | |
| Normal saline negative control group | 131.14 | 354.08 | 784.26 | 1045.1 5 | 2099.5 0 | 1600.96 | +16.8 |
| MI-6PEG-AAN-P AB-Belotecan 30 mg/kg | 151.03 | 282.87 | 579.35 | 837.75 | 1135.0 0 | 751.51 | +1.2 |
| MI-6PEG-AANG-D3 30 mg/kg | 138.55 | 302.74 | 742.51 | 898.06 | 1141.6 5 | 824.00 | +11.4 |
| MI-6PEG-AANL-D3 30 mg/kg | 134.07 | 387.76 | 768.59 | 998.38 | 1508.2 3 | 1124.96 | +12.2 |
| MI-6PEG-AANG-D4 30 mg/kg | 138.58 | 278.49 | 624.53 | 791.18 | 977.54 | 705.40 | +9.6 |
| MI-6PEG-AANL-D4 30 mg/kg | 133.89 | 309.51 | 703.24 | 894.45 | 1345.0 3 | 1004.58 | +14.5 |
| MI-6PEG-AANG-D5 30 mg/kg | 138.17 | 245.39 | 425.66 | 514.73 | 752.03 | 544.28 | +15.2 |
| MI-6PEG-AANL-D5 30 mg/kg | 134.01 | 254.52 | 612.55 | 730.28 | 1116.7 1 | 833.30 | +7.9 |
| MI-6PEG-AANG-D6 30 mg/kg | 133.65 | 179.74 | 231.50 | 356.48 | 521.34 | 390.08 | +8.5 |
| MI-6PEG-AANL-D6 30 mg/kg | 128.26 | 247.43 | 396.52 | 561.20 | 726.75 | 566.62 | +5.2 |
| MI-6PEG-AANG-D7 30 mg/kg | 138.52 | 228.26 | 362.93 | 566.23 | 678.75 | 490.00 | +15.6 |
| MI-6PEG-AANL-D7 30 mg/kg | 134.03 | 241.52 | 494.28 | 748.06 | 1327.2 5 | 990.26 | +4.8 |

This mouse tumor model experiment further confirms that the polypeptide Linker AANG is very advantageous as it allows compounds to obtain better antitumor effects and lower toxicity compared with AANL and AAN-PAB.

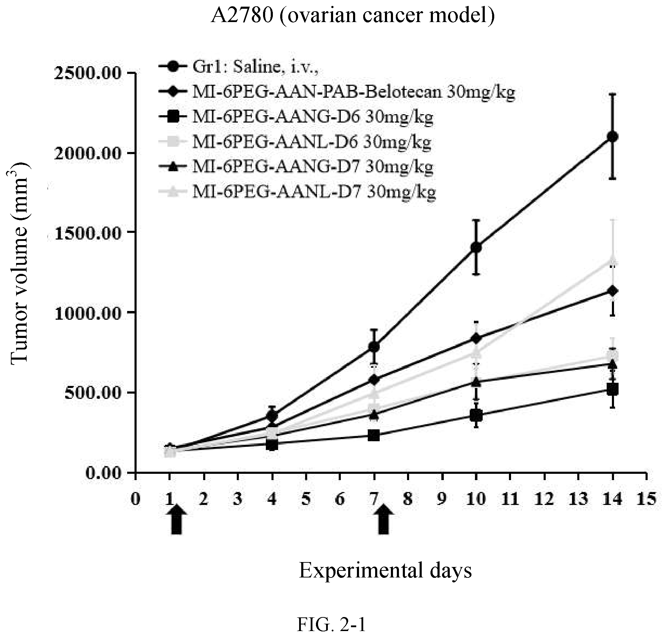

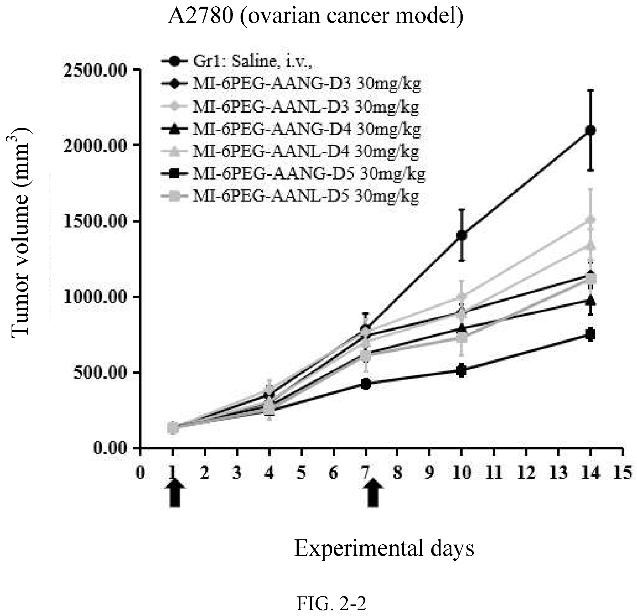

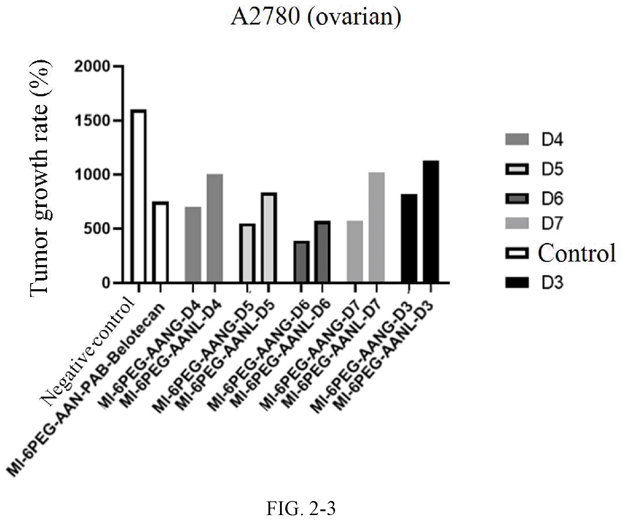

FIGs. 2-1, 2-2 and 2-3 of the specification show a schematic diagram produced based on the data in Table 12.

It can also be seen from FIGs. 2-1, 2-2 and 2-3 that a comparison of tumor volume growth curves with AANG, AANL and AAN-PAB as tetrapeptide Linkers shows that the tumor volume growth curve of AANG was significantly slower and the tumor volume growth curves of AANL and AAN-PAB were significantly steeper, and this indicates that the polypeptide conjugate with AANG as the Linker was released in tumor cells at a higher rate than that of the polypeptide conjugate with AANL and AAN-PAB as the Linkers and can produce a better efficacy.

### [Example 42] Efficacy study of polypeptide conjugate in treatment of an animal model of colo320 colorectal cancer

Test objective: To investigate the antitumor efficacy of the above compounds in an animal tumor model of colo320 colorectal cancer.

Test drug: Polypeptide conjugate, Irinotecan, Belotecan and normal saline control group.

Test animals: 6-8 weeks old BALB/c mice, all female.

### Tumor model preparation:

Experimental objective: To investigate the antitumor efficacy of the above polypeptide conjugates, Irinotecan and Belotecan in tumor models at doses of 15 mg/kg and 45 mg/kg. The colo320 cells were purchased from AoRuiCell Biotechnology (Shanghai) Co., Ltd. and cultured in a 1640 medium containing 10% fetal bovine serum at 37°C in 5% CO₂. Passaging was performed every three days, and cells within 15 generations were used. 7.5 × 10⁶ corresponding cells were injected into right chests of nude mice subcutaneously. After a volume of the tumors reached at least 120 mm³, the mice were grouped randomly, with three mice in each group. Then treatment was started, and the day when the treatment was started was the first day. An experimental group of Belotecan positive control was administered with Belotecan at a dose of 5 mg/kg, and Irinotecan positive control was administered with Irinotecan at a dose of 15 mg/kg. The control group was administered with normal saline. Administration was performed once a week for four weeks.

**Table 13**

| Compound name | colo320 (colorectal cancer cell) mean tumor volume (mm³) | colo320 (colorectal cancer cell) growth rate (%) | Mouse weight (%) |
|---|---|---|---|
| Normal saline negative control group | 1648.7 | 1488.58 | +41 |
| Belotecan (5 mg/kg) | 963.5 | 869.93 | -9 |
| D1 (5 mg/kg) | 1121.1 | 1012.22 | -7 |
| Irinotecan (15 mg/kg) | 903.1 | 815.39 | +35 |
| MI-6PEG-GGFG-Belotecan (45 mg/kg) | 581.5 | 525.03 | +34 |
| MI-6PEG-GGFL-Belotecan (45 mg/kg) | 789.4 | 671.56 | +5 |
| MI-6PEG-GGFG-D1 (45 mg/kg) | 671.3 | 605.35 | +19 |
| MI-6PEG-GGFL-D1 (45 mg/kg) | 900.6 | 766.16 | +19 |

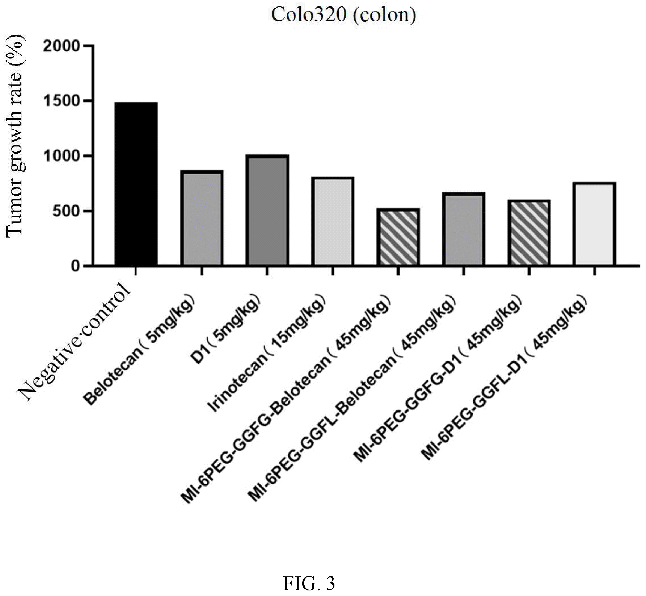

FIG. 3 of the specification shows a schematic diagram produced based on the data in Table 13.

It is clear in the comparison of the tetrapeptide Linker GGFG with GGFL that GGFG had a better tumor suppression effect than GGFL, it can be seen from Examples 27 and 36 that the MTD in mice was increased by approximately 2-fold with GGFG compared with GGFL, and it is clear that GGFG can provide a more significant molecular toxicity reduction effect than that provided by GGFL. GGFG is a polypeptide Linker having a better effect than that of GGFL.

### [Example 43] Efficacy study of polypeptide conjugate in treatment of an animal model of colo320 colorectal cancer

Test objective: To investigate the antitumor efficacy of the above compounds in an animal tumor model of colo320 colorectal cancer.

Test drug: Polypeptide conjugate, Irinotecan, Belotecan and normal saline control group.

Test animals: 6-8 weeks old BALB/c mice, all female.

### Tumor model preparation:

Experimental objective: To investigate the antitumor efficacy of the above polypeptide conjugates, Irinotecan and Belotecan in tumor models at doses of 15 mg/kg and 45 mg/kg. The colo320 cells were purchased from AoRuiCell Biotechnology (Shanghai) Co., Ltd. and cultured in a 1640 medium containing 10% fetal bovine serum at 37°C in 5% CO₂. Passaging was performed every three days, and cells within 15 generations were used. 7.5 × 10⁶ corresponding cells were injected into right chests of nude mice subcutaneously. After a volume of the tumors reached at least 120 mm³, the mice were grouped randomly, with three mice in each group. Then treatment was started, and the day when the treatment was started was the first day. An experimental group of Belotecan positive control was administered with Belotecan at a dose of 5 mg/kg, and Irinotecan positive control was administered with Irinotecan at a dose of 15 mg/kg. The control group was administered with normal saline. Administration was performed once a week for four weeks.

**Table 14**

| Compound name | colo320 (colorectal cancer cell) mean tumor volume (mm³) | colo320 (colorectal cancer cell) growth rate (%) | Mouse weight (%) |
|---|---|---|---|
| Normal saline negative control group | 1648.7 | 1488.58 | +41 |
| Belotecan (5 mg/kg) | 963.5 | 869.93 | -9 |
| Irinotecan (15 mg/kg) | 903.1 | 815.39 | +35 |
| MI-6PEG-AAN-PAB-Belotecan (45 mg/kg) | 635.7 | 573.97 | +6 |
| MI-6PEG-AANL-Belotecan (45 mg/kg) | 833.5 | 690.61 | +2 |
| MI-6PEG-GGFL-Belotecan (45 mg/kg) | 789.4 | 671.56 | +5 |
| MI-6PEG-AANL-D1 (45 mg/kg) | 953.2 | 810.91 | +16 |
| MI-6PEG-GGFL-D1 (45 mg/kg) | 900.6 | 766.16 | +19 |
| MI-6PEG-AANL-D2 (45 mg/kg) | 894.4 | 760.87 | +19 |
| MI-6PEG-AANL-D4 (45 mg/kg) | 978.4 | 832.33 | +18 |
| MI-6PEG-AANG-Belotecan (45 mg/kg) | 366 | 330.46 | +29 |
| MI-6PEG-GGFG-Belotecan (45 mg/kg) | 581.5 | 525.03 | +34 |
| MI-6PEG-AANG-D2 (45 mg/kg) | 588.5 | 531.35 | +11 |
| MI-6PEG-GGFG-D2 (45 mg/kg) | 568.5 | 513.29 | +41 |
| MI-6PEG-GANG-D2 (45 mg/kg) | 499.2 | 450.72 | +39 |
| MI-6PEG-FANG-D2 (45 mg/kg) | 561.1 | 506.61 | +24 |
| MI-6PEG-AAAG-D2 (45 mg/kg) | 491 | 443.32 | +25 |
| MI-6PEG-AAKG-D2 (45 mg/kg) | 521.2 | 470.58 | +24 |
| MI-6PEG-AACG-D2 (45 mg/kg) | 471 | 425.26 | +26 |
| MI-6PEG-AANG-D6 (45 mg/kg) | 283.4 | 255.88 | +17 |
| MI-6PEG-AANG-D7 (45 mg/kg) | 463.7 | 418.66 | +31 |

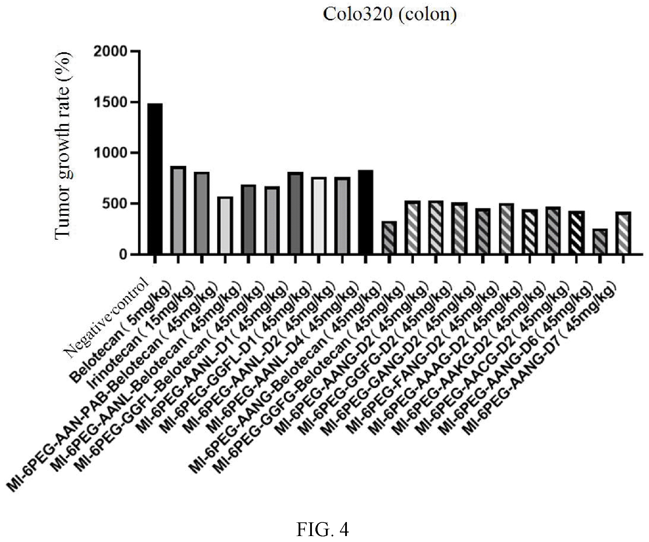

FIG. 4 of the specification shows a schematic diagram produced based on the data in Table 14.

It can be seen from Table 14 and FIG. 4 in conjunction with Examples 40, 41 and 42 that with respect to the polypeptide conjugate of the present disclosure having a general formula of E-nPEG-L1-L2-L3-L4-D, the polypeptide conjugate exhibits a better efficacy when the L4 group is G (glycine) compared with the case where the L4 group is L (leucine) or PAB (aminobenzyl alcohol). Taking specific compounds as examples, MI-6PEG-AANG-Belotecan and MI-6PEG-AANL-Belotecan were activated in the tumor microenvironment in a first step to generate G-Belotecan and L-Belotecan, respectively, then entered the tumor cells, activated in a second step to generate Belotecan, thereby killing the tumor cells, while G-Belotecan was released in the tumor cells at a higher rate than that of L-Belotecan and could produce a better efficacy, so MI-6PEG-AANG-Belotecan has a better efficacy than that of MI-6PEG-AANL-Belotecan.

### [Example 44] MTD determination of polypeptide conjugates

Test animals: 6-8 weeks old mice, all female; mice were grouped randomly, with six mice in each group, administered with drugs at different concentration gradients separately and monitored for 14 d. A mouse was euthanized at a loss of 20% of its initial weight and deemed to have died of poisoning. An MTD was defined as the highest dose level, in which none of the six mice died of the drugs, no individual mouse lost more than 20% of its weight, or a mean weight loss in a group did not exceed 15%.

Final statistical results of MTD of the polypeptide conjugates are shown in the table below:

**Table 15**

| Compound name | MTD |
|---|---|
| Belotecan | 15 mg/kg |
| Irinotecan | 75 mg/kg |
| MI-6PEG-AAN-PAB-Belotecan | 100 mg/kg |
| MI-6PEG-AANL-Belotecan | 150 mg/kg |
| MI-6PEG-GGFL-Belotecan | 100 mg/kg |
| MI-6PEG-GGFL-D1 | 120 mg/kg |
| MI-6PEG-AANL-D6 | 120 mg/kg |
| MI-6PEG-AANL-D7 | 150 mg/kg |
| MI-6PEG-AANG-Belotecan | 300 mg/kg |
| MI-6PEG-AAKG-D2 | 350 mg/kg |
| MI-6PEG-AACG-D2 | 400 mg/kg |
| MI-6PEG-AANG-D1 | 420 mg/kg |
| MI-6PEG-AANG-D8 | 400 mg/kg |

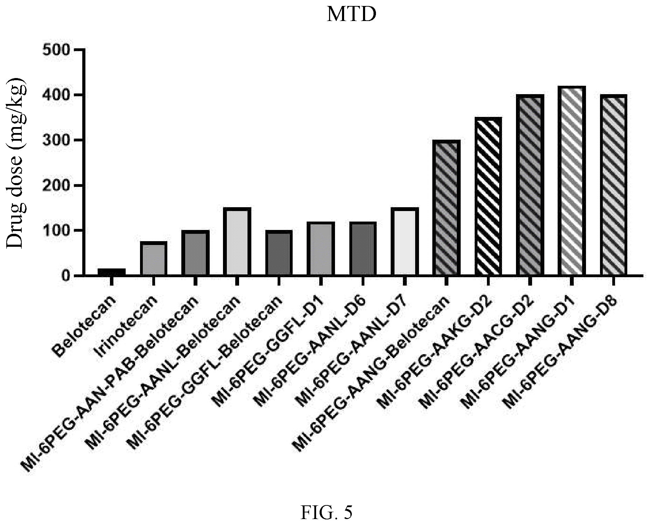

FIG. 5 of the specification shows a schematic diagram produced based on the data in Table 15.

It can be seen from Table 15 and FIG. 5 that with respect to the polypeptide conjugate of the present disclosure having a general formula of E-nPEG-L1-L2-L3-L4-D, the polypeptide conjugate exhibits lower toxicity when the L4 group is G (glycine) compared with the case where the L4 group is L (leucine) or PAB (aminobenzyl alcohol). Taking a specific compound as an example, MI-6PEG-AANG-Belotecan is more stable than MI-6PEG-AANL-Belotecan because AANG is more stable in blood and normal tissues compared with AANL, AANL is easily hydrolytically disconnected between N and L by proteolytic enzymes in normal tissues and releases more L-Belotecan in normal tissues and blood, resulting in additional toxicity, while AANG is more stable and released in a very small amount in normal tissues and blood to generate lower toxicity.

In summary, the above examples are only preferred examples of the present disclosure, and are not intended to limit the protection scope of the present disclosure; any modifications, equivalent replacements, improvements and the like made within the spirit and principle of the present disclosure should be included in the protection scope of the present disclosure.

## Claims

1. A polypeptide conjugate, wherein the polypeptide conjugate has a structural formula as follows:
E-nPEG-L1-L2-L3-L4-D
wherein,
E is a group with maleimide;
nPEG is n-polyethylene glycol, and n is an integer greater than or equal to 2 and less than or equal to 40;
L 1 and L2 are glycine, alanine, phenylalanine, threonine or serine;
L3 is glycine, alanine, lysine, citrulline or asparagine;
L4 is glycine, proline or p-aminobenzyl alcohol; and
D is Belotecan or a derivative thereof.

2. A polypeptide conjugate, wherein the polypeptide conjugate has a structural formula as follows:
E-nPEG-L1-L2-L3-L4-D
wherein,
E is a group with maleimide;
nPEG is n-polyethylene glycol, and n is an integer greater than or equal to 2 and less than or equal to 40;
L1 and L2 are glycine, alanine, phenylalanine, threonine or serine;
L3 is glycine, alanine, lysine, citrulline or asparagine;
L4 is leucine or isoleucine; and
D is Belotecan or a derivative thereof.

3. The polypeptide conjugate according to claim 1, wherein the polypeptide conjugate has a structural formula as follows:
E-nPEG-L1-L2-L3-L4-D
wherein,
E is a group with maleimide;
nPEG is n-polyethylene glycol, and n is an integer greater than or equal to 2 and less than or equal to 40;
L1 and L2 are glycine, alanine, phenylalanine, threonine or serine;
L3 is glycine, alanine, lysine, citrulline or asparagine;
L4 is glycine; and
D is Belotecan or a derivative thereof.

4. The polypeptide conjugate according to any one of claims 1 to 3, wherein the D group has a structure as follows: 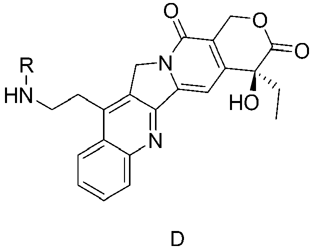 wherein, R is methyl, ethyl, n-propyl, isopropyl, n-butyl, isobutyl, cyclopropyl, cyclobutyl or cyclopentyl.

5. The polypeptide conjugate according to any one of claims 1 to 3, wherein the E group has a structure as follows: 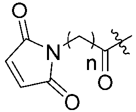 wherein, n=1-18.

6. The polypeptide conjugate according to claim 1, wherein the polypeptide conjugate has any of the following structures:

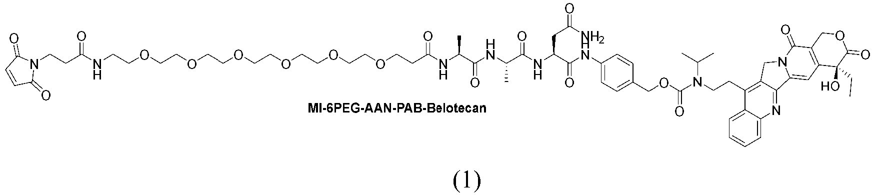

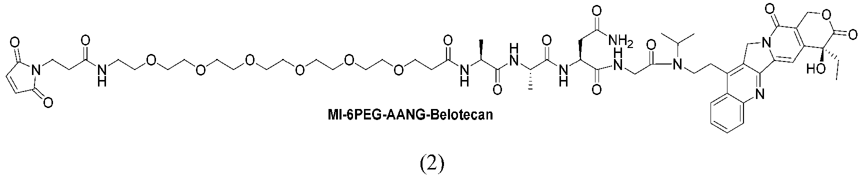

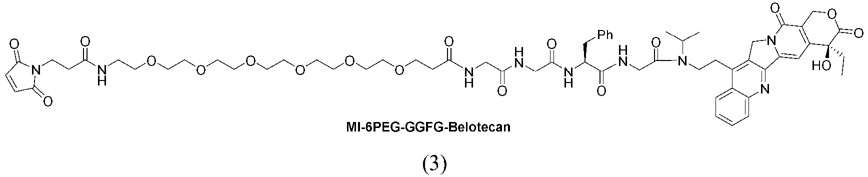

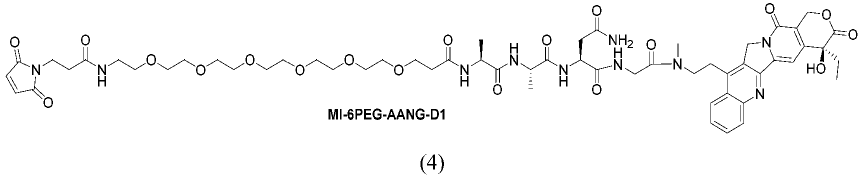

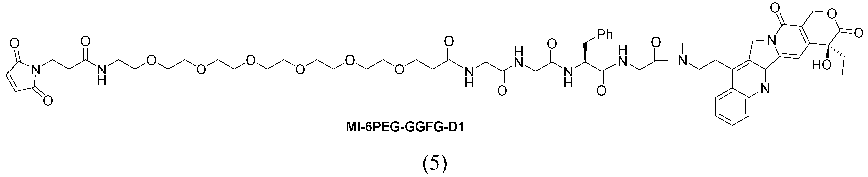

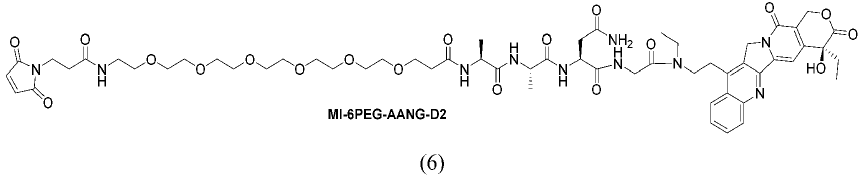

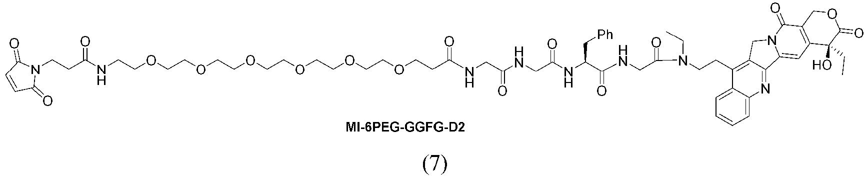

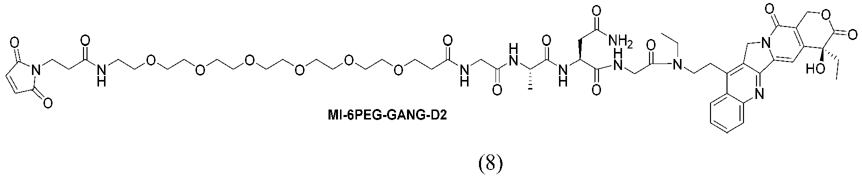

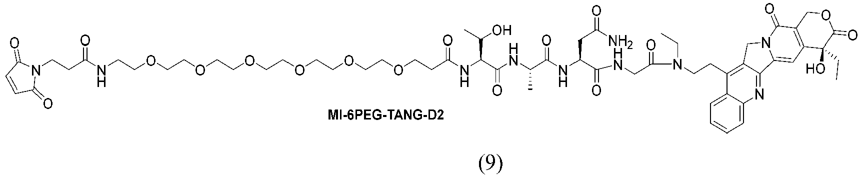

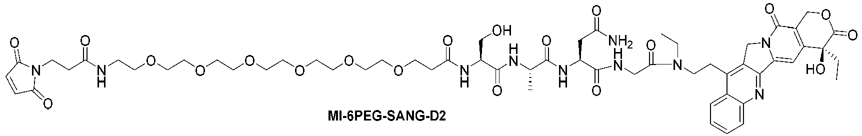

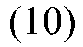

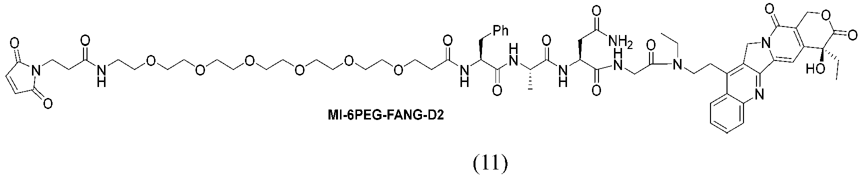

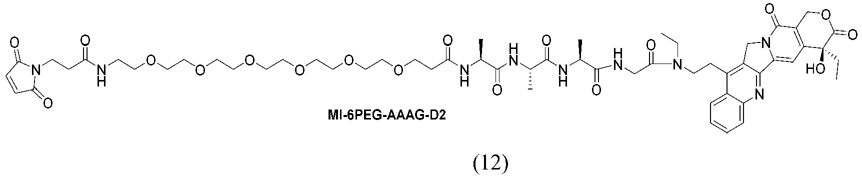

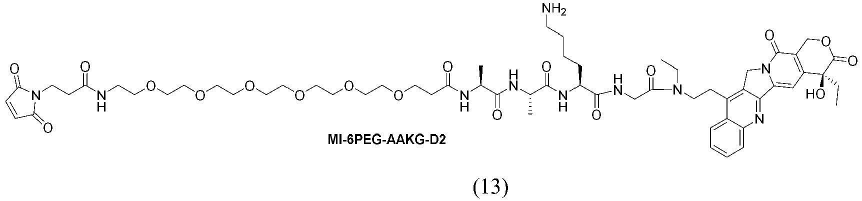

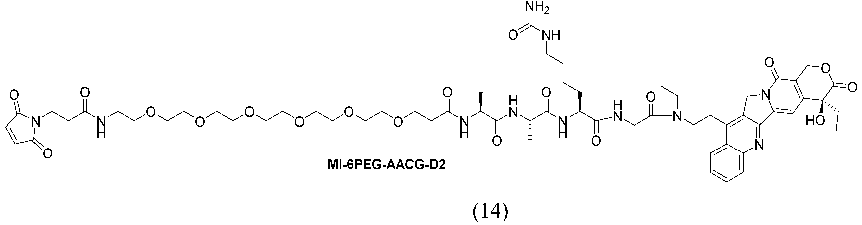

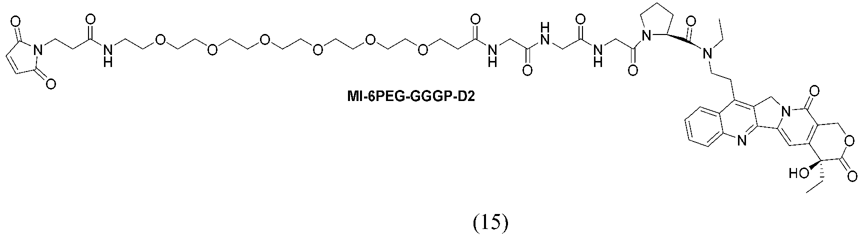

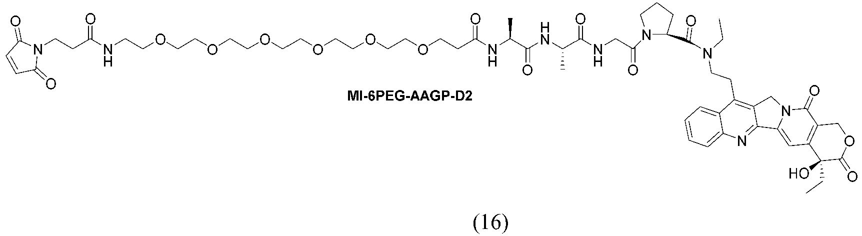

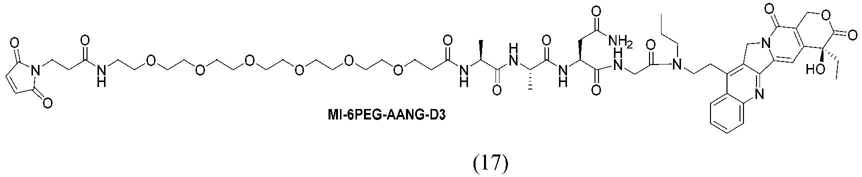

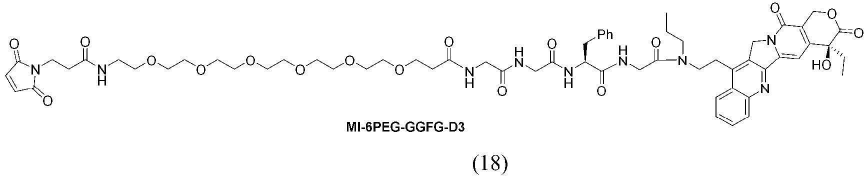

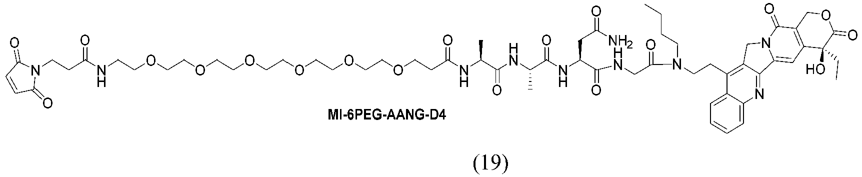

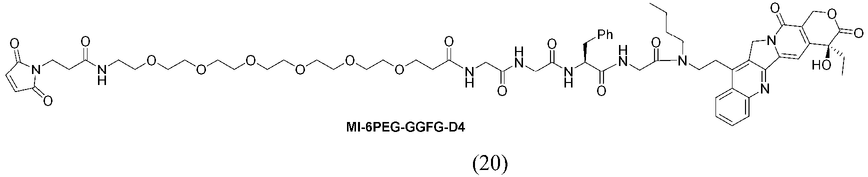

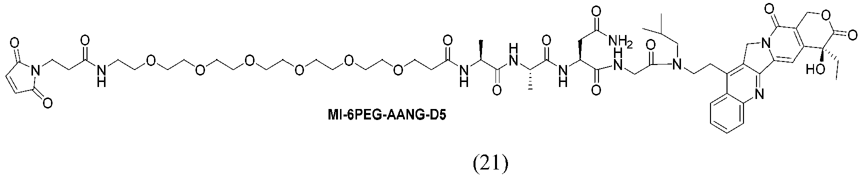

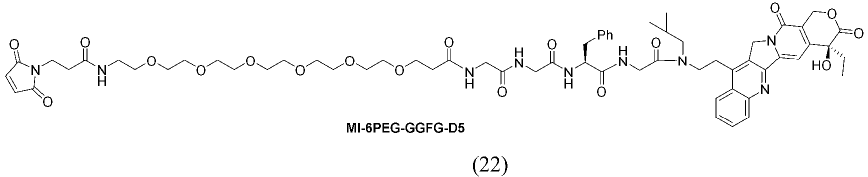

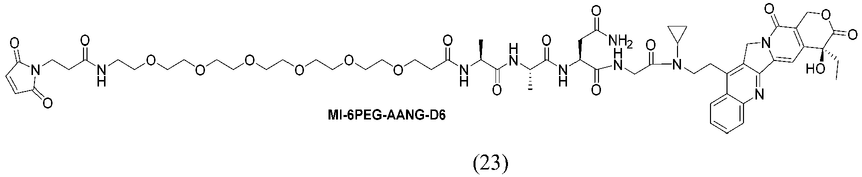

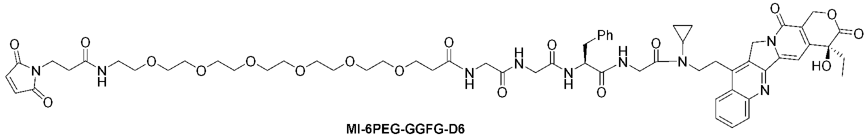

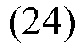

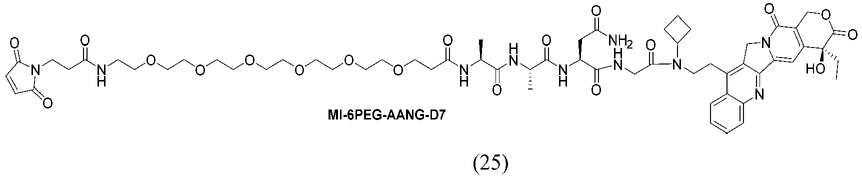

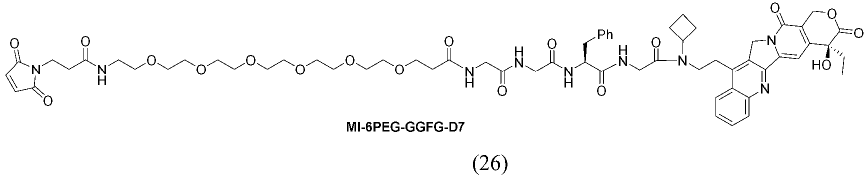

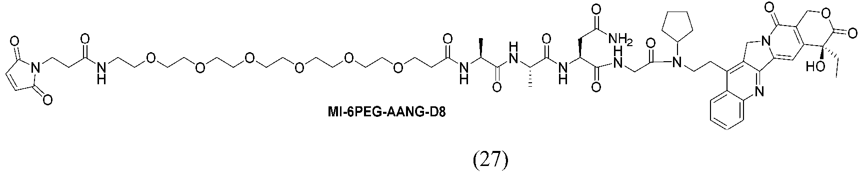

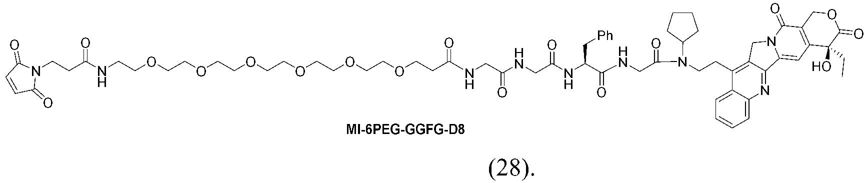

7. The polypeptide conjugate according to claim 3, wherein the polypeptide conjugate has any of the following structures:

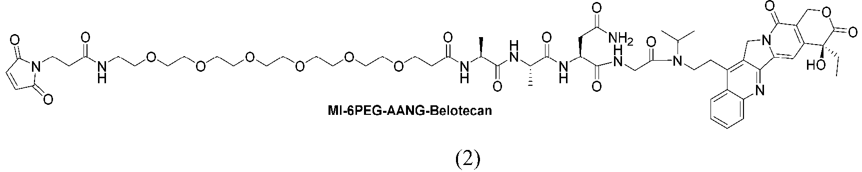

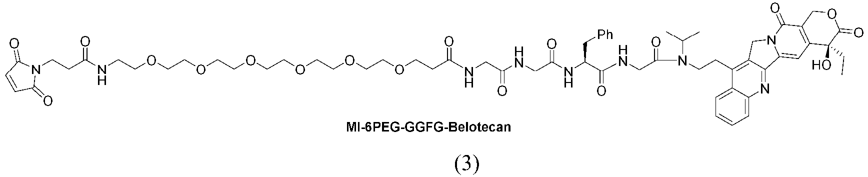

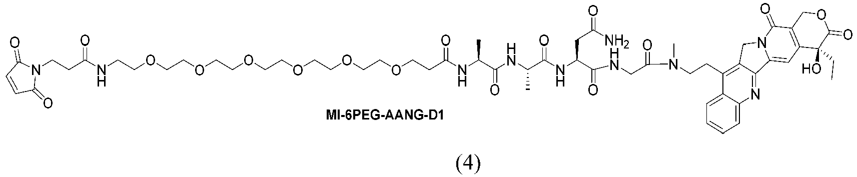

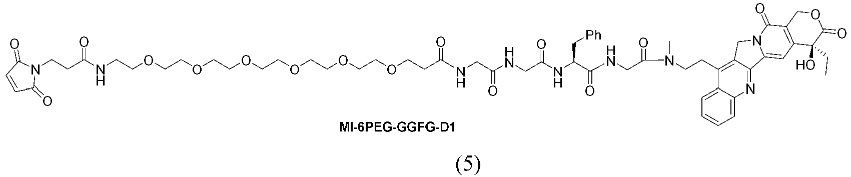

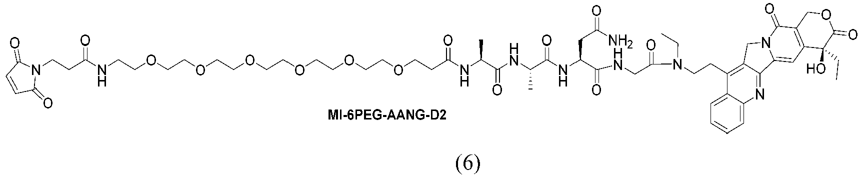

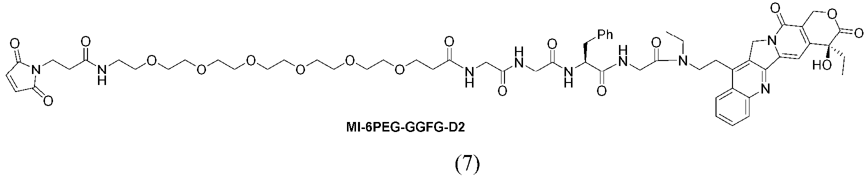

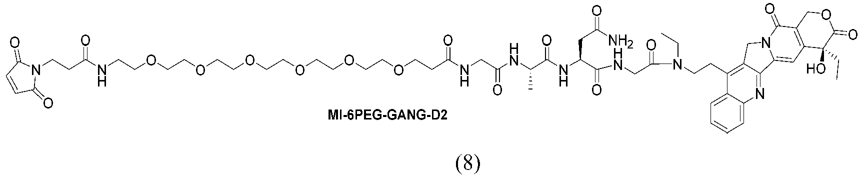

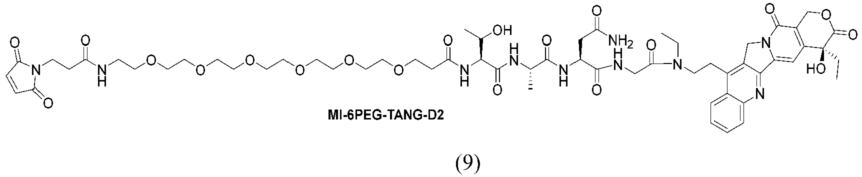

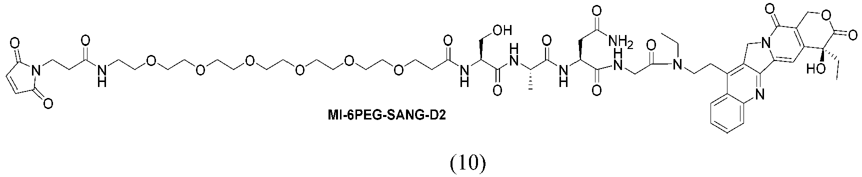

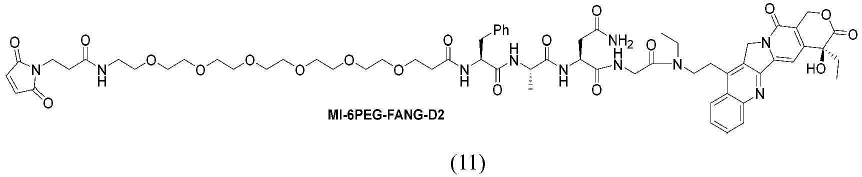

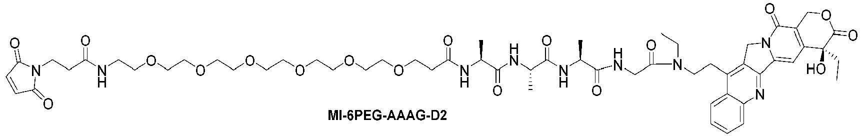

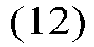

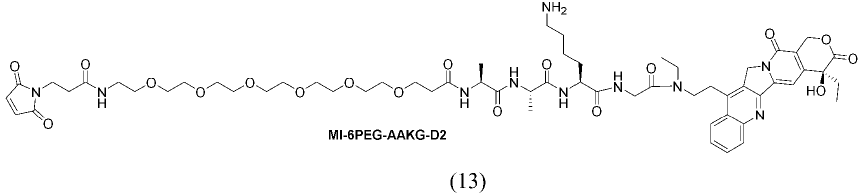

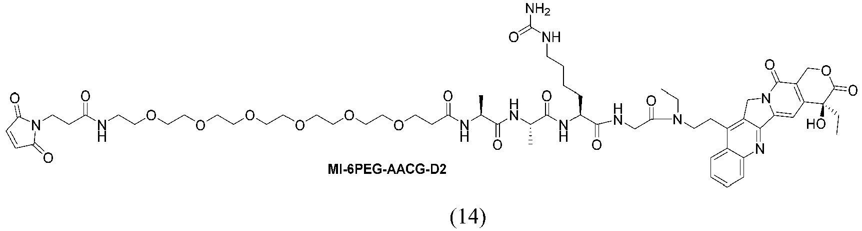

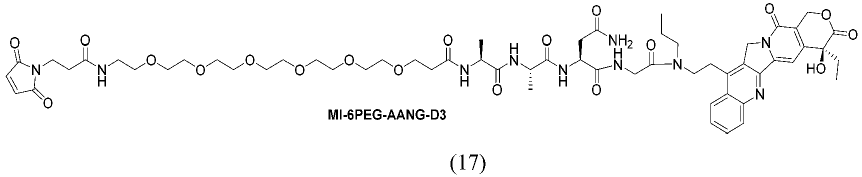

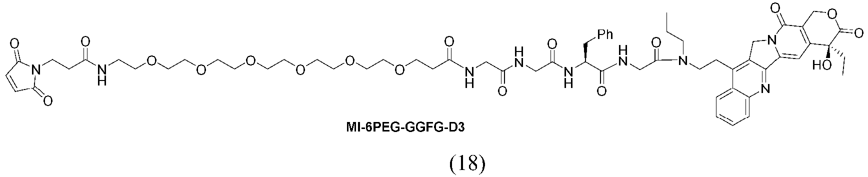

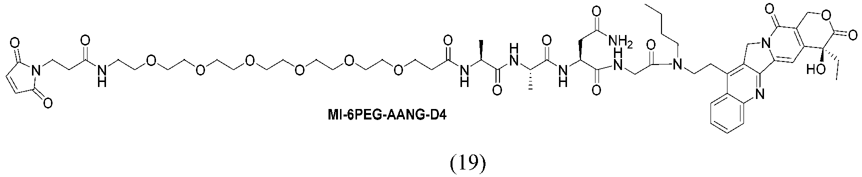

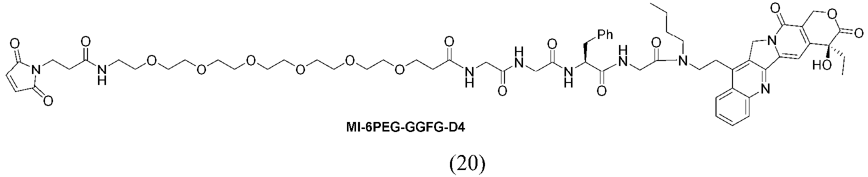

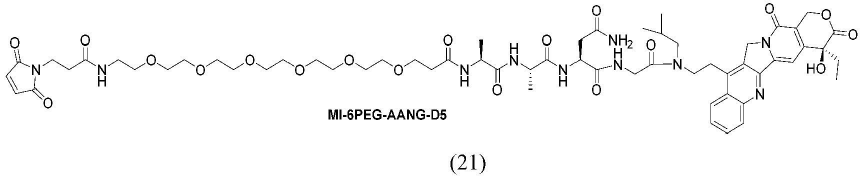

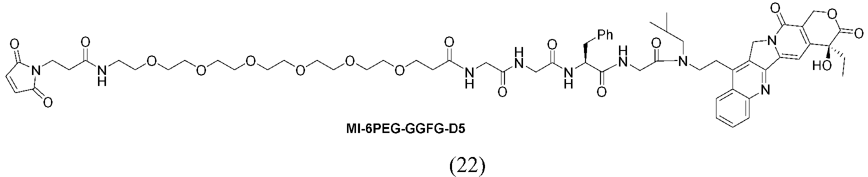

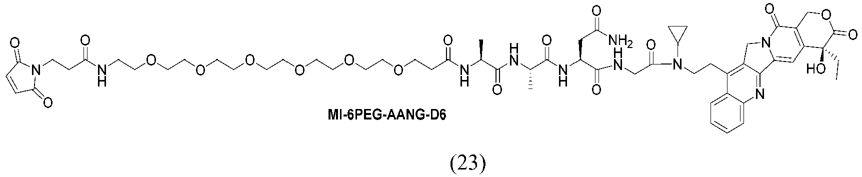

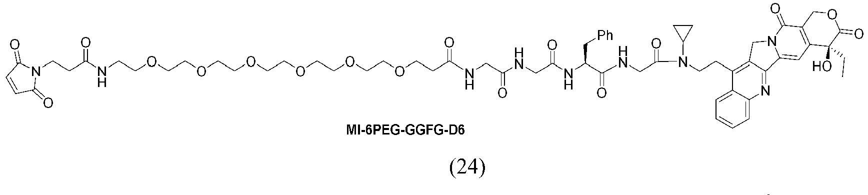

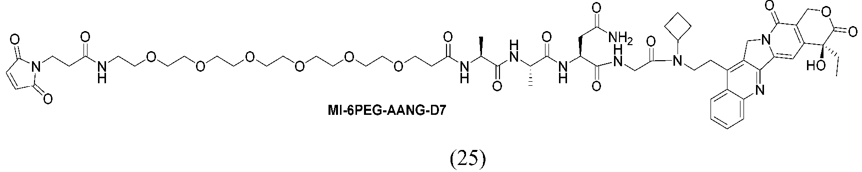

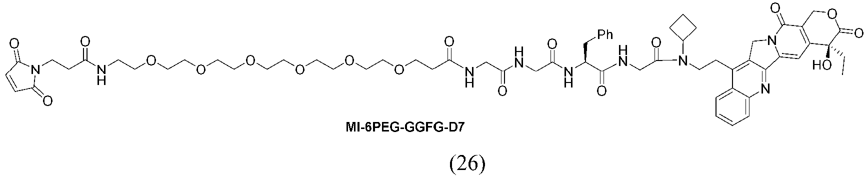

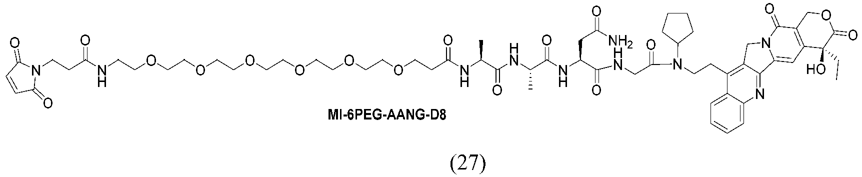

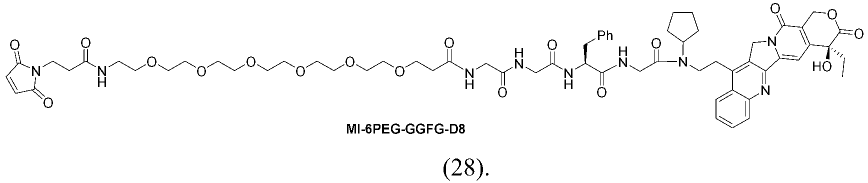

8. The polypeptide conjugate according to claim 2, wherein the polypeptide conjugate has any of the following structures:

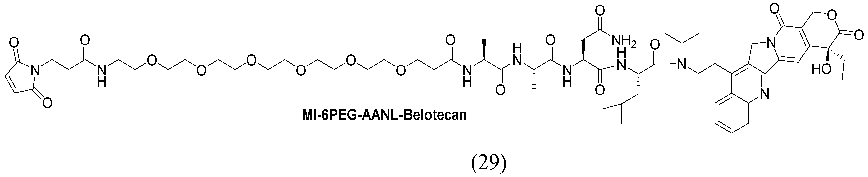

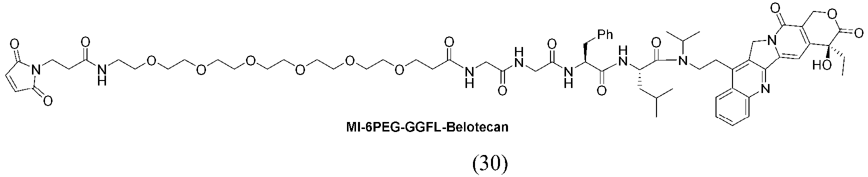

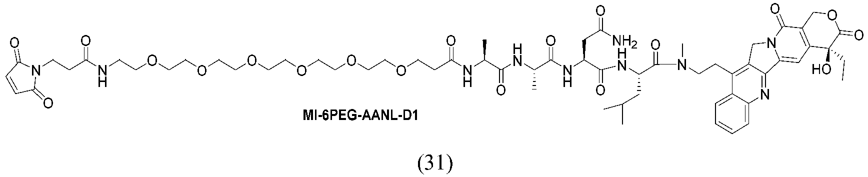

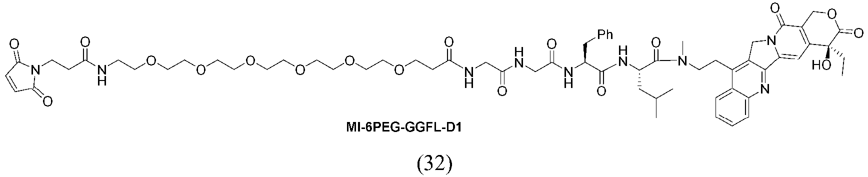

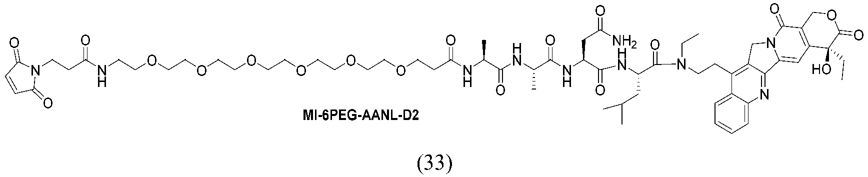

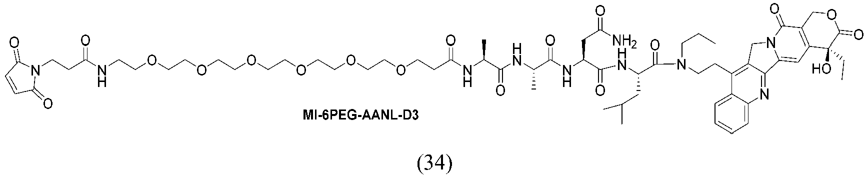

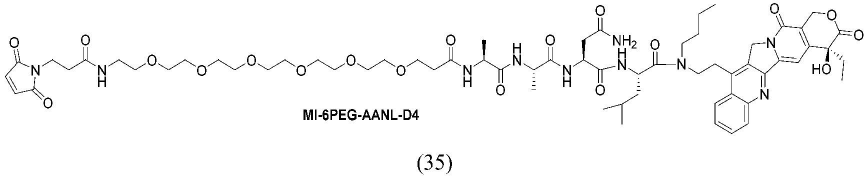

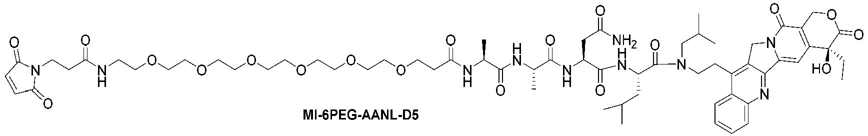

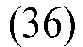

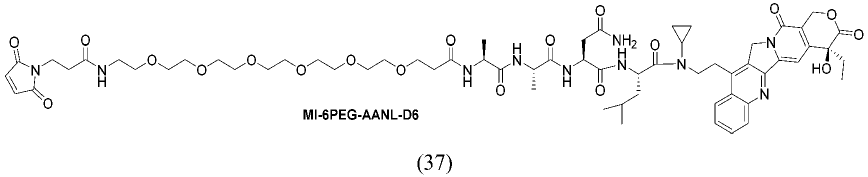

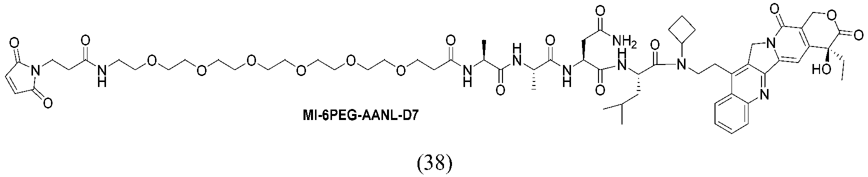

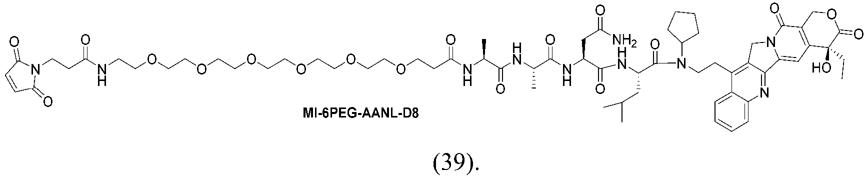

9. A pharmaceutical composition, wherein the pharmaceutical composition comprises the polypeptide conjugate according to any one of claims 1 to 8 and a pharmaceutically acceptable carrier.

10. The polypeptide conjugate according to any of claims 1-8, and a use of the pharmaceutical composition according to claim 9 in preparing an anti-tumor drug.
